# EUROPEAN PATENT SPECIFICATION

(11) **EP 2 885 427 B1**
(45) Date of publication and mention of the grant of the patent: **10.01.2018**
(21) Application number: 13759978.3
(22) Date of filing: 14.08.2013
(51) Int. Cl.: C12Q 1/68

(54) **COLORECTAL CANCER METHYLATION MARKER**
KOLOREKTALKREBSMETHYLIERUNGSMARKER
MARQUEUR DE METHYLATION POUR LE CANCER COLORECTAL

(30) Priority: 14.08.2012 EP 12180459
(43) Date of publication of application: 24.06.2015
(73) Proprietor: Max-Planck-Gesellschaft zur Förderung der Wissenschaften, 80539 Munich (DE)
(72) Inventor: SCHWEIGER, Michal-Ruth, 14167 Berlin (DE); GRIMM, Christina, 50354 Hürth (DE); HERWIG, Ralf, 14476 Potsdam (DE); LEHRACH, Hans, 14129 Berlin (DE)
(74) Representative: CH Kilger Anwaltspartnerschaft mbB
(86) International application number: PCT/EP2013/002462
(87) International publication number: WO 2014/026768

(56) References cited:
- EP-A1- 1 295 941
- WO-A1-2012/034170
- RAFAEL A IRIZARRY ET AL: "The human colon cancer methylome shows similar hypo- and hypermethylation at conserved tissue-specific CpG island shores", NATURE GENETICS, vol. 41, no. 2, 1 February 2009 (2009-02-01), pages 178-186, XP055029485, ISSN: 1061-4036, DOI: 10.1038/ng.298
- M. D. ROBINSON ET AL: "Evaluation of affinity-based genome-wide DNA methylation data: Effects of CpG density, amplification bias, and copy number variation", GENOME RESEARCH, vol. 20, no. 12, 1 December 2010 (2010-12-01), pages 1719-1729, XP055041625, ISSN: 1088-9051, DOI: 10.1101/gr.110601.110
- E. A. HOUSEMAN ET AL: "Copy number variation has little impact on bead-array-based measures of DNA methylation", BIOINFORMATICS, vol. 25, no. 16, 15 August 2009 (2009-08-15), pages 1999-2005, XP055041627, ISSN: 1367-4803, DOI: 10.1093/bioinformatics/btp364
- Douglas Roberts ET AL: "Authors Genome-wide Monitoring of CpG Island Methylation With Agilent's Tiling Microarray Technology", , 13 June 2007 (2007-06-13), XP055094900, Retrieved from the Internet: URL:http://www.chem.agilent.com/library/ap plications/5989-6838EN_FINAL_low.pdf [retrieved on 2014-01-06]
- ANONYMOUS: 'NimbleGen CGH Microarrays and Services High-resolution array Comparative Genomic Hybridization (CGH) platform' INTERNET CITATION, [Online] 01 January 2008, pages 1 - 12, XP055276316 Retrieved from the Internet: <URL:https://roche-biochem.jp/pdf/products/ microarray/user_guide/CGH/CGH_brochure.pdf> [retrieved on 2016-05-30]

### SEQUENCE LISTING

<110> Max-Planck-Gesellschaft zur Förderung der Wissenschaften e.V.
<120> Colorectal Cancer Markers
<130> B117-0005WO1
<150> EP12180459.5
   <151> 2012-08-14
<160> 64
<170> PatentIn version 3.5
<210> 1
   <211> 1999
   <212> DNA
   <213> Homo sapiens
<400> 1
   cttatttcca tgcaaatttc acaatccccg ttacttgccc agatacaaca attaaagctt      60
   aaaaggtggc gggagtgggg gacttgagga ctggtctgag gagaaagtga atctcccaag     120
   ggttcctaaa tggttttgct tccagtataa aaactgcgag ctaccagtag aatttaacaa     180
   cagctcaacc ttgcatttgg aacagttact atatagttca ctttcttttt tcatgggggc     240
   ggggtatggt gtcttaccta ctcttaaatt tgaacgtatt aacaggttcc cctccgcgca     300
   cactgacata tttcttatcc cccataatga attcagccat atggcattct ttcccatcga     360
   aggccatcgg gaatggcttt aggaagctga ttttcaagct ttaagcggca gcaggtgccg     420
   gcagcgcggg gaccgatcga tggagagaag gcgggcaaga cgccgggaag cgcattcctc     480
   ctcaaccgag tgccacaacc gccctcccga agtgccccgg ggcttcgagc atcacctcgc     540
   ggtaatccgg gagggtggag ggatgcggct ggacccgggc gttgcgtgct ccacacagcg     600
   cccagcccgt gccagccccg cgcccacctc tccacgacgc tcgtgccggg atcagcgcga     660
   agccccttcc agtccccgaa gccctcgccc gcgcccgttc tcccccagct cgccccctcc     720
   agcccgctgc gccttgccgc agcatctccg ggcactctga ggctgccgcc gggacagggt     780
   cggagcgccg cagaacccac cgaaacttcc caggggggca attcaaaatt cgccggacgc     840
   gtcgccgccg cgcgcccctc ggctcattcc cttccgcgcg cccgcagccc caggctctcc     900
   ctctctcagg accccccagc gccctgcgcg gcgagaatag gcccccaggt gcctcccggc     960
   cccgggggct gccgtcgcac gtccgctccc gcaggggtcc tcactccgcc aatcgccgcg   1020
   gccgcgcgcc ctcgcgcaca ctcaccagcc cgagccgggg cggccatctt agcgctcacc    1080
   ccggcccccc gccccccggt tcggcggccg cgacgacccg gtgcggcggc tacgacagcc    1140
   gtgacgcgca gcaggccccg ccccctccca cagccccacc cctgcgccgg ctcttcgcgg    1200
   gcaccgagaa cctgccggtg gccgccttcc gcgcctcgtg ggggggtcgg ggccacggac     1260
   ggtccccggc gccgcaagtg ggtctgcgcg aacaacaagc actgcctccc cgggcgggct 1320
   tcgcacctgt agtgccgtcg ggacacggga gggtaaaccc agcgtgtcct gtgtgcctgt 1380
   gagccgcaga atcatccacg gacgtcgtta gtccttcctg gaatttctgc gatttacaca 1440
   acgtcgaatt gtttggcaga aacgcgtggc aaactccgtt atctttaaaa ccttccccaa 1500
   ttcactggca tagaaattct taaagaaaac gtttccttct tgaagcgacc cctgggtgta 1560
   acttcagtgg cgatgacggc tgtgaattgg gttttttcgc accgcagaag ggcgagagag 1620
   gttccagaac gggcacagga agggaaccgc tatctagaac tgcctaaccc gaaattgccc 1680
   atttaaataa tgaagtacat accgaaaagg aaaaggaggg gaaatctgga aaacaggaaa 1740
   gtcaaggcta aggtacctga aaattaaccc attaatattt attggattct ttgtgttcaa 1800
   ctctgagcca gattgttgtt tttaactgaa cctatactca atgacaaagc agttctactt 1860
   tggccaccct gtggagtgta ctgaaaattt aaaaactctc caaggagagc ttaaaaagaa 1920
   gacaaacatg caaagttaac aatacatcaa tgcagtgcaa aatcttgcaa tatgtaagac 1980
   aaggtataaa attgttcct 1999
<210> 2
   <211> 1499
   <212> DNA
   <213> Homo sapiens
<400> 2
   ctccacggac tctgcgggaa gttagagcct ctgcgtgcgc tccggggccc ggcgagagga      60
   tgcgcaaggt ggagagccgc ggggaagggg gcagagaggt aaaggctgaa ggtgccccgg     120
   ggaaccccgg cgggcggccc accgagggag ggagaggcgg ccgggaccaa ggaatggggc     180
   ctcttggttc cccattaacg cacgctgaag aaatctgctg cgctcctgac ggccgctcac     240
   cgggttcgag ccccgtcctc ctatagccgg ggcgctcgct ggccaaagcg acccgagcag     300
   gcgaatgacc tttaggcgga cggggttttc cctctgcttt cttgtttctt ttgaggagac     360
   gggtgtgtgt ttgtgaggtg gggatggggg aagagtgtcc cagacatccg tagtctgctg     420
   agcggaacgg agcttgggga gcggcgaggc attaacgatt aagtggagcc gggaaggcgc     480
   tggctttggt gatgtgttgg gtttggatgt gtcgcgtctg cacagatgag gtgccctgcg     540
   tgggctgagg gttattcctg tctctttccc gtccgtctac acccgccaac ccctttttgt    600
   tttggtcttt agaaatctgt agcataaccg taccgtcgtg gatccccatc tcgtctctgt     660
   ccctgatctg gggtgattgg gacttcggtg tcgctctttt tccaaagttg gagggtcggg      720
   agcgccgaga caccctggcg aggaggagga ggaggaggag ggaggctgcg ctgagccggg     780
   tgcaggtgcg ctcacgtttg catcaattag gaactccggg cagagagagc tgcacttagg     840
   tcagggatta actgtggacc cgcgggaccc aagcgctggg gtaggaggac tggggatctt     900
   tgttcggagt gcgctgcgaa ggctgctgga ggcggacacc ctcccagctt attgctagcg     960
   tgggatagag ggagcgcacg cggctaggct ccagcagcga ctcggctttt cgcgtattct    1020
   aagcactgaa gagcctctta aggggagctg tccaaatcgc ccaggagtgg tggcgagaca    1080
   caggaggcca tgccagcgat gctgttatta atattgcaga cttggtcatc tctcctggct    1140
   tgcggtttct tttctcctct tccctcccct tctcttttct ctcacatgtg tttcacacag    1200
   gtggtgggga ttactcaatg acttacagct cccttctcgt ttattagtgg gagggggttg    1260
   aatgttggca gttcttacaa agcatttgtt ttcttaaacg atcctgtttg atccatactc    1320
   tgagataagt atgaaaatat taaaacatca tacgttcctt ccttttatac cccttcctcc    1380
   taatccccag cacacatcag aatgtaaaca ttggttagca gatatagaaa aataatttca    1440
   gaacgggaac atggattgaa catcctcttt caggctgaca gcccttaaat ttcattaac     1499
<210> 3
   <211> 1999
   <212> DNA
   <213> Homo sapiens
<400> 3
   cataaaagaa tcacacttat ttgatattag tttggtgggg tttttttcat tcaattttta    60
   atggcctttc tcaatatctc agttcattca aaggttctga ttttctttct tttcctccgt    120
   gtagtctttc ccagggcacc cggttgaagc ccaaggctaa ctgggaccct cctacttcag    180
   caccaaggac agaaatcgct aaatctccag gggaaacgta cccctaacca ccgccagatg    240
   tctacttttc agacaaagca agaaaaagaa aatatacctg ccttgccagc catctgttta    300
   aaagtcccct ctcctgtgga acgcacgagc aacttttcgg agacactgaa caactccaag    360
   tcgcgcgccg ccctcgcaaa tcgcagagag ggccgcgaga aggtgcgaac gcaggtcacg    420
   gccagcgccg cttggagaga gacccgcagg tttcagccca ggcgcgcccg gcgaaagcca    480
   acgcgctctc cctacaaagc gtcgatgact tcagggattt aaaagaaaaa atacccacag    540
   acagaaccag cggaggggcc ctgacctcgc cccagtcggg aaacgccttc cctccgccac    600
   aggcagcgct gaatgaagca gaggagggcg gcggagaggg ccccggaaga agggaagggg    660
   gcattctgca gtgtttgggg gctggggaaa gaacattttc tcaccacttg ggctgtcgct    720
   ggacctcagg ctccttccac agagacactg cagcatatgc actcctttct tcagagaaag    780
   ctcaagaatc ttcatggaga agcgcgtgtg tggggttggt caactccccg cccacctgcg    840
   ctagtagtcc aaccaacagg cggcctgtct tcggaagccg ggtcccgagt ccatcgcgcg    900
   cgcccaggtg gaggggagtt tgcacatgga gccggaggga gcccgggcgc cggcaggggg    960
   cgggccggga cgcggaagtg ccggtccgcc gggggcagcc ctccgagagc ccgaggcgct    1020
   gccacccctc ggtgggctcg agcacggccc cttgagacct tccggaggcg gtggctggtc    1080
   tgaggacgac gcggaggacg tcactgcggg tcggtgcttc cttacaggtg ccttctggac    1140
   cggggtcctt ggcacctccc ctgctcctgc cctcggtgcc ggaccctgtg ccctgggagc 1200
   ccgactacct cggtgtccca gccgtcccgg gcttgaggcg ctgagagggc tgcgcggctt 1260
   ccagcccgga aggcagcggt cccgcgggct gcgcgcggcc aagggcgact ccggtgtggg 1320
   aatccggcgg aagggaagca cccgcaggga gggctggacc ccggaggctg cagagcgtca 1380
   gaagcgactc tagggaacta gggggtgggg tagggaggcg gggacgtgga ataaagaaag 1440
   ctcctgggtg ccggctatga gaagtcaggt gtgcgtaggc gtggacagag tgccgatgtg 1500
   ggagtctgga cacctggatt ttctggtcgg ggctctgtgt ccttgggtaa gtcacttacc 1560
   accctgggcg tctccccgtc aatctgggtg gggaagaggg tgtgagatag aggattggca 1620
   gcggcgtgct tgtttgtccc cgtgcctttc aggctcctag aaaagcttag cataggtgca 1680
   gtgggaagtg gagctagaag ggacagaggg agaggaggca ggtgaggcga gaaatctgaa 1740
   gacaaaagag cgcttcgctt tggcgccagt attctggcag gctttgcctc tgccagcccg 1800
   ccccgatgac caaacagctt ctccatgagt ttaaagatct cgattttttt ttcccagcag 1860
   cccccttgac tctttttttt tttcttttcc tgatgccaac aatgcccttt tggaagtgca 1920
   atgagtaagc atgggaagaa tgctgtcgaa gtgacaggac gtaaccctat gtggaatctc 1980
   agggcaagag gggacttta 1999
<210> 4
   <211> 1749
   <212> DNA
   <213> Homo sapiens
<400> 4
   cgaaatagaa atacgtgccc cgactcggga agtgggagtc cctttcacac cccagcaatt      60
   gatcccctct ctcctcgccg gcccgcccgc cgctgctctt cttccaggca caatcgaaga     120
   ggaggcagtg agcgagtcaa ggccacagag tggatggaat caaggttcac ccccaaagct     180
   cacctccttt gcaacccgga tccccactcc tcaccaccta cggcccctct tcccttccat     240
   ccccgcccag tcacccaacg ctgaagccac cgcggggtgt gggggggtga cgtgtgggaa     300
   gagctggggg cttccttcgc acccaccctc acgcgcccta gaatgtcctc tggggaaggg     360
   gctgcccata acttggagga acttagaagg caaaacctac tgcgccccaa cccttagagg     420
   ggcctcaacc ccgaaggcga ggggcgagat cagggactcg gcgacgaggg cgagcgcccc     480
   cgggcttacc acgagcacgg ggaccccggc ggccagcgag tagaggagca cggggggcac     540
   ggcgctgctg tcctccgggc ccgggtaggg tttgcggtag gcgctgtcgt ggcagaagaa     600
   gccctgcacg ttcacggtga acgtgtccgt atactcgaag tagtacgcca gcatcaccgt     660
   ccctgccatg atcaccatct ggaaatagag catgctgctg gtgagcgccg cgggcagcag     720
   gggcatgcac gcctcccggg ccgggccgag ccgagccgag cgggcggtcg acgcggtggg     780
   ccccctcccc ggtccgccga ggcagccacc gggggcgcgg cggcggaggc ggcgggagga     840
   cgaggcacgg gaggcgggat ggagccgctg gaggaagagg cggaggcagg tccgggcttc     900
   gaggcgccgg caggctgcag aggaggcggc tacccccgga cgagccccct ctcccctgcc     960
   cgccccctgc ccgccgcaag cgccgcccgc cccggcgcgg ggtcgcgagg gagggcgggg    1020
   agtcccgggc gacgggcagc ggccgctgcg cccctgcacg agaccattcg agaagcagcg    1080
   gcgctgggtc aatcccccag gctagcccgg aggaggcgct gcgtgggcgg acggggcggc    1140
   agccggcggg acagcggcac ctgtacccct cacagggcgg acgctgtggg gctggagaag    1200
   ctcctggcgg gggtaaaatc aaaagggggg gaggggaggc agtagagatg gagcttccag    1260
   aaactcttcc gaggcaccag ctgagaggtt taagaaaccc gcacaacgcc tgggaaaatg    1320
   gtgcgtggac gcgtcttccg agcgcaaagc ccaccaaggc gcaaagtgcc gatgcggcgc    1380
   ccagagtttc aaccggtgcg ttcagcctgc atccctcgaa ttccttgacc cagcccgggg    1440
   ctggagcctg gcggtggttt ctaggcgctg ttagaaaaat ctcagcgagg tttctttgcc    1500
   tcctctgcag cttcctaggg ctttgtgtat atatatatgt atatacaaat aataatagaa    1560
   atcatagccc agtagctccc gaagcatcat ctcttgtaca gcggcccctt cctggatcca    1620
   tgcattctct tgctcatctt ttcagtctgt ctttattagc tgcttgtgag aggaggcatt    1680
   gcagattcca ggcactgagc ggtcccagcc accagggtag gaaaaaggac tatttgcctc    1740
   atctcgttc                                                            1749
<210> 5
   <211> 1999
   <212> DNA
   <213> Homo sapiens
<400> 5
   ggccccgccc gccccacccc atcctgtgct taaatagagc ctttcttgaa gctgcgaaca      60
   tttccaggcc ccttgggcag ggctggaggg gccgaggaga gctattcgag ggaaaggtgc     120
   cccgaggggc aggaaattaa gttggggctg cccgggcgag ctgccaggta gccgtgctcg     180
   ccacggcgtc tcatggggca cctagctagt ggcgggcctc atagggcggg aaaagaatcg     240
   tcgctcacac cccagcaaaa cgtggccctc gacggtccgt tggagagccc cggcggccgt     300
   gagccccggg cagggctgga cgtctgcgga gccctcgggc actttgtccc gggcgcctgg     360
   ggaggaacgc ggagctccca gggccttagg tgcaacggct gcgcagagcc caaacgaaat     420
   gtccccagtg cggaaaagcc ggtgacgccc tggtagcaag accaagagct tccgaagaac     480
   gctgcgccct taactagggg gcctcgcaga gatgcctgtg tgggcctgca ttgtatattt     540
   ctgcgaaata gcgaatggac acgtttgctc agggttttta tggttgccaa agggggtaaa     600
   attacccagt cccccaaatc tgtgtcccat gaatccctct catagtaccc ctctccaggg     660
   ggccaagagg tcctccaggt ccccgtgggt tcgcagctcc acccgccctt cctcgccctg     720
   catccctaag gagaggtgtc cgctctgaag ggctaggggc cagccatgga gtgaggggac 780
   cggggctgac cacgcgcggc acagacagag gtcctcaggc gggccctctc ctggacggtg 840
   gggccggagc tgatctagaa gaaatacgga gggacgtgcc gagaagccgc tctccttcgc 900
   cgcgaccctg gagagcgcct ctccacccaa aggatctgcc gagctgagag atccagggcg 960
   ggcgtccgca gccgtgaggc cccctgcgcc gccagtatgg gaagatcctg cctccttaca 1020
   ccttggagaa cgctgggcga cgactaaagc gccttccgcc ggcctgtcac tccatgtgac 1080
   acaggagcca cgtgagaccc agaagagtcc agcgactcgc cgcgcggcgc actttaaact 1140
   ctagcctgag tctgcgaccc ctccagctct ccagtcccca gctgttgggg acatcaagcc 1200
   ggagccctgg gctctctgcc ctgtgggtcg ctgaaagcag agactcctca aaccaaccga 1260
   accgggcgca ttaaccctct cgcctgcacc ccgctgcctc ccggttgagc cccgaggcgg 1320
   ctccaggtag aacctgctgg actgactgcg gcgtccagaa atctggagtg tgggctccag 1380
   acactctcca cggtttggcc ccgggtctca acacccaagt cgcctcttct ggctccttca 1440
   ccacacagcg gggcctgtgg aaagggaggg gccgagagac ccgtcggcgc accactgtcc 1500
   tcgaggggtc cccaccctgt gcactgctga agcgcagggc gcgccgcggc aggaatggcc 1560
   ccgagtgcgg atcccctgcc ctgagcctcc cactcttggc ccgcgctgcg cctacccagt 1620
   ggccctggcc ccgcagggcg acagcggctg ctccctccca tttgcgtccc agaccgcgcg 1680
   gcctcgctta gctcccggga gccgacaggc gcttgccctg gtgccagcgc agggcttccc 1740
   gggggcttgg ggtaggggta ggggtgcggg ggggaagggg agaacgtaat ttccttctgc 1800
   aggagtcgtg gagacgtgag ctgcaaccag ccaccgcgct ctctccaggc ttgtttacca 1860
   gttttaggtc atcattgtgc acgaaacatt ctttcatcca aataaaagca aatgcagaag 1920
   aacacctgat cccaaacagt gtatgactgc gttcattatc ttacctggtt actccgaagg 1980
   agttgaattt ttttaatgt 1999
<210> 6
   <211> 1499
   <212> DNA
   <213> Homo sapiens
<400> 6
   gtgcgccgtg cgggttgtga tccgttaccc catcggtcat cctggggtct ccccaagcct 60
   ctaggtaggg ctgtgagagt cccctagagc tgaagccccg gaggctgacc tgtgggtctg 120
   gctgctatgg gaacccggtt ggtccaaaga agcctttctt ccgggcacct ggaattccag 180
   tttagtgtgg ggcatcgggg aagtggcgct ggggggctgg gttgggggac ctcagccggc 240
   agctccggag agggcctacc cttggggtcg ctgggtgagg ccggcacgat tcttggctcc 300
   aaaaggaaag tttctgcttc ttgttctggc gcgagaagcc aaagacttat tttgagagcg 360
   gagagagaaa tgttattggt aacgttttct ttggaaagtt cgagaggggt cttctggaca 420
   cactacctag tgcccccaaa ccagagaagt agtttttctt tggtgcctgg gctcagaagt 480
   cgccactcac tcagcccatg gttcgaaatc agcatgggaa gcgccggggc aaggcttcgt 540
   cggagactag aggcctgcct gtcgggagga gcccctgggg gatggggacc ccattctcct 600
   gcttgctctg gttcccacct gggacgcctc cgtaggagcc cagaaagacg atccactaca 660
   tggtcccggg acagagcagc gcgcccaact ttgagggaac tttgtgcgcc tctctgaggc 720
   cctagctttc caaggcaccg ccgtccgttc ttctttccct agaccgaaac tggggaagag 780
   tgtgggcgct tctttgcccc gatgagttcg cctccccaaa cgcctacttc ggctgcacca 840
   gagcatctgg gaaactctga aaggtgccca ggcctcacac agcagcgtct ccctactcag 900
   cctctgtctt tgggtttttt caagagagtc tctacctcat gcctcggtct ttcttcgatg 960
   tcgggtcccc gaggtaggca cggagtccct ctgaaagcag ttgcctatct gtgccccttt 1020
   ggtgtaaagt tagagtttac tttgttgggg gaaggggagg tagaaaagat cacagttggg 1080
   aaagtgcgct tttcgccttg ttcctaaaac atgcctcaag actgtcatcg cgattgttag 1140
   gagagctatc aacgtctagg ggctataaag gaatttctga accctcggcc cttcccaaac 1200
   ccccaggttc ctaaaaccct agtgggggtc tcttggggct gggattcagg ctggcaccgc 1260
   tgggaggacc tcgcctagca tccctttatt aatatttcac gaaggcaggc tcctgccttc 1320
   tctggagcct cttttctcgg aatgttccca aactctggct aactcactcc cctgtgagcc 1380
   atcctagggc tctgtggccc gggaagagac gcgtcaactc cgcgggtctg cgcgcagtcc 1440
   ttagccgcaa agtgctgcaa gtgacccccc tgacggccct ttccgaccga agagctcgg 1499
<210> 7
   <211> 999
   <212> DNA
   <213> Homo sapiens
<400> 7
   ggctctatta atagctgggt gtctggtggg gctgccgcac atttcacata tggttaccca      60
   tatgcagcgg ggggcgggga tgggggtgtg gcgcggggat tgtccctctg tcttgccgga     120
   atgcaaaaag gtagagagac ccttcctggt cttcttccct cgagttctta actctgcgct     180
   aaaaccccta ccccacggcg taggcagcaa agctttataa atcccccttc tctgagagac     240
   tagaagcagc atgcatctga caattgtcaa tttcaaaaca aacacgctcc gggacttgaa     300
   cgcagcgggg cattcagtag cgaatgctgt ctccttgagt tagggcaaag cctgcgtgcc     360
   cgccgtcccc tcaccacttc ctcttcccca gcccccacct gagagcagac attcggaatg     420
   atgtgtagtg cgaggcggct agcctcccag cagaaagcca tccttaccat tcccctcacc     480
   ctccgccctc tgatcgccca cccgccgaaa gggtttctaa aaatagccca gggcttcaag     540
   gccgcgcttc tgtgaagtgt ggagcgagcg ggcacgtagc ggtctctgcc aggtggctgg     600
   agccctggaa gcgagaaggc gcttcctccc tgcatttcca cctcacccca cccccggctc     660
   atttttctaa gaaaaagttt ttgcggttcc ctttgcctcc tacccccgct gccgcgcggg     720
   gtctgggtgc agacccctgc caggttccgc agtgtgcagc ggcggctgct gcgctctccc     780
   agcctcggcg agggttaaag gcgtccggag caggcagagc gccgcgcgcc agtctatttt     840
   tacttgcttc ccccgccgct ccgcgctccc ccttctcagc agttgcacat gccagctctg     900
   ctgaaggcat caatgaaaac agcagtaggg gcggccgggc tcctgcgaac aacaacaaaa     960
   caaacaaaca aaaaaccacg tcgcgtgcgg ggcaccaag                            999
<210> 8
   <211> 1499
   <212> DNA
   <213> Homo sapiens
<400> 8
   ccctcaggcc ccagcagctc caccatcatg ggcacgtagt cacggttggg cgaggaggtg      60
   gctgtgtgct gatagcgcac cccagcacgg aggaaagcct cacagtctac gcccttgccc     120
   ctggggagag gggcccccac cgcgtccacc aagcgcccgt acttgggcag ggggccgtcc     180
   tcgtgaggaa gtggggtaag ccggcacctg cgggtggccg tggctccaga cttcagggag     240
   gcgaagtcca gcactctcct gtctatggcg cggctccagc ttcgcagctt ctccactacc     300
   aaaggcctgt tacgcgtcac cagctccagc tgggagaaga ccaagtccac cgccagcgtg     360
   aagggcagca ccagagtgtg agtcggggcg tcgtagcgca gctgcagcag cacccgggcg     420
   cgtccggggc tgtgggagcc gaagtgagtg tactggactt ggcggggccc gaaggtgcag     480
   gggaagcggc gcggggagag cgcgcccttg agccgcggca gggcgtccag taccgtgact     540
   tcgcaccggt cccccggctg cactccaatc accagatccc ggagcgggtc gagccaaagg     600
   gaacgaccca ggggcacccg gagtccaggg ttggcaatca gcacgctggg gccgtcgggg     660
   cgagtgccgt caagcgcacc ccgggcgggc aggtaaagcg ccgggtcggg ctcggtccca     720
   agtgaggatg cccgtccctg cagcgcgggg cgactcaaga gcaggcaggc gagcgccaca     780
   aggagctgcc ggggcgtccc agtcgggtgc cgagaagccc ccgccatggc cacggatggc     840
   tcctggcgtt gggattcccg gggtggggtg ccctgtgcaa agagggatct gctgagcggc     900
   aggtgcaggc agtggaagca gtagctgctg tccagtcggt agccgacttg cggatccagc     960
   aagagccagc ggctgcgctt cggctgctgc aggtaacggc agcgggggaa ggggctctgc    1020
   ccacttcctg ctcagccccg gtcgcaagtc tctctctgct ggcttctggg gaccccagat    1080
   acgcgcccag cgcggcgaga cttagcgagg gtgcagcgct gtcccctccg ctcctgggcg    1140
   cttcacccag cctaccttac acaccttctc gccgggagcc gtggccgccg cactgctgcc    1200
   cgcgctgcca gactccgacc agctgtctgg atactctctt ccccaggtgc cacaaaggga    1260
   ttgtccctca gggttgggag agagacggtg actgtactcg ggtcagtcct gcgtctgtga    1320
   gattgagctc ctgttgtcca ttcatccagg gattggtgtt tctgaaaagg gggagagaca    1380
   ccattcctct tccttaccgc tgacaggagt gtatcttcta gccaaaaact gagtctcact    1440
   tcggacataa aagaagctgg tgggagctat tttgcaaata ggattttcta gctgtctgt     1499
<210> 9
   <211> 2999
   <212> DNA
   <213> Homo sapiens
<400> 9
   gcttcacggt ttttgatatt taattcaatg ctgttggaac agcacaaaaa ctaagtgtca      60
   gtttaacaga atcacttgtc cttttagcat taaaataaca tggaacttaa tgctttaatt     120
   tcccaacatg cctttttatt tagaaagatt cagacttata tttcatttag aaataaaatg     180
   ccattttatt tagaaagata caggagcatt cattcacgga actttcagat ctcagtccac     240
   tgcataaaat cttgatcctg taataatagt ttctgtatct tgcatattca ttcaacaggt     300
   ttaacgcgat gagcaaatta atgttcatcg tttttaacat gtttcatctt aatcagaacc     360
   cacattctca acgttaattg aacgtacata ggactataca agggttagta aataagacag     420
   aaactgttgt tcatttaacc accgtcactt tggaccaaaa aagaaaaaat atatattttt     480
   aaaattgagc ttaaaagagt ctctagaagc tggaagcgtg gctctttttc agcaaactgg     540
   gggaataggt ttaccgtgtt ccccctctgg ggaattttga gtcgccacac tcatgtctcg     600
   accgagcctg gctcgctgcg tctgagcgag tacttgagga aggctgatct agaaaaacca     660
   gctgagagaa ggggcagaag cccctgaaac cacgggcggg ggtggggtgg ggagcgcagc     720
   tttgggaccc tctagccgga gacttccggc agctgcctcc gacttgttct aagtacagga     780
   aaaatctgtg cgcccagttg cctcactcca acagcgcgca gttgtgcccg gcgaggatgc     840
   cgcgctagtc gtggagatgc cccaccacaa agaggattca ggtgcttcct actccggcac     900
   ccagtgggtt ggtagtcctg ttggcaggag acaagaatcg tctgggctgc tcctatctct     960
   ggcaggacta gacggggcgt gaaggaaaga aggaaagaag gaaagcaggg atcgggcact    1020
   gcccgagggc agatacttgg gctttggtgt tgtccagcgc gctcggagtg cgctgcctcg    1080
   ctcacgcggt cccaggcccc gcttcttcag gcagtgcctg gggcgggagg gttggggtgt    1140
   gggtggctcc ctaagtcgac actcgtgcgg ctgcggttcc agccccctcc ccccgccact    1200
   caggggcggg aagtggcggg tgggagtcac ccaagcgtga ctgcccgagg cccctcctgc    1260
   cgcggcgagg aagctccata aaagccctgt cgcgacccgc tctctgcacc ccatccgctg    1320
   gctctcaccc ctcggagacg ctcgcccgac agcatagtac ttgccgccca gccacgcccg    1380
   cgcgccagcc accgtgagtg ctacgacccg tctgtctagg ggtgggagcg aacggggcgc    1440
   ccgcgaactt gctagagacg cagcctcccg ctctgtggag ccctggggcc ctgggatgat    1500
   cgcgctccac tccccagcgg actatgccgg ctccgcgccc cgacgcggac cagccctctt 1560
   ggcggctaaa ttccacttgt tcctctgctc ccctctgatt gtccacggcc cttctcccgg 1620
   gcccttcccg ctgggcggtt cttctgagtt accttttagc agatatggag ggagaacccg 1680
   ggaccgctat cccaaggcag ctggcggtct ccctgcgggt cgccgccttg aggcccagga 1740
   agcggtgcgc ggtaggaagg tttccccggc agcgccatcg agtgaggaat ccctggagct 1800
   ctagagcccc gcgccctgcc acctccctgg attcttgggc tccaaatctc tttggagcaa 1860
   ttctggccca gggagcaatt ctctttcccc ttccccaccg cagtcgtcac cccgaggtga 1920
   tctctgctgt cagcgttgat cccctgaagc taggcagacc agaagtaaca gagaagaaac 1980
   ttttcttccc agacaagagt ttgggcaaga agggagaaaa gtgacccagc aggaagaact 2040
   tccaattcgg ttttgaatgc taaactggcg gggcccccac cttgcactct cgccgcgcgc 2100
   ttcttggtcc ctgagacttc gaacgaagtt gcgcgaagtt ttcaggtgga gcagaggggc 2160
   aggtcccgac cggacggcgc ccggagcccg caaggtggtg ctagccactc ctgggttctc 2220
   tctgcgggac tgggacgaga gcggattggg ggtcgcgtgt ggtagcagga ggaggagcgc 2280
   ggggggcaga ggagggaggt gctgcgcgtg ggtgctctga atccccaagc ccgtccgttg 2340
   agccttctgt gcctgcagat gctaggtaac aagcgactgg ggctgtccgg actgaccctc 2400
   gccctgtccc tgctcgtgtg cctgggtgcg ctggccgagg cgtacccctc caagccggac 2460
   aacccgggcg aggacgcacc agcggaggac atggccagat actactcggc gctgcgacac 2520
   tacatcaacc tcatcaccag gcagaggtgg gtgggaccgc gggaccgatt ccgggagcgc 2580
   cagtgcctgc acaccaggag atcctgggga tgttagggaa agggattgtt tcttttcctt 2640
   cgctctatcc cagggcagga cagtatcagg cacttagtca gctctaggta aatgtttgta 2700
   cagggcacac tctacacaaa atgggtacct tccattttgt gcaactacag tcacagagtc 2760
   gtgatcccca gattcaggtt ccccaggctg gtaggctggc aatctcctct cactcacctc 2820
   ttatggtttg ttgtggttct tacggcagtg gggcccggtc cagaaatctc gaaagtaccc 2880
   agtgaaaggg gcaagaatgc gccagagaaa tgctgtaggg ggaaacgcta gcaaggtgtc 2940
   taggagaaac agaacgacca ccaaagaaaa ccaaaccaag gagtaaactg cagggttgc 2999
<210> 10
   <211> 2749
   <212> DNA
   <213> Homo sapiens
<400> 10
   gcaacggtgg tgagaagggt ggtcccaagg ccgcgggagg agccaatcag cggcgactct 60
   gggctcttgc agcctcctta gagactccgc agccctggag gtaccaagct gcctgctgcc 120
   ttttctcgcg ctgcaggcgc ggagatgcag cgcctctggg ggcgcagctc cagccgcact 180
   cgcagggcaa ggcacacgcc cccggctcct gctgccatgc gcctctgcgg gggacccttt 240
   ccaaataaat tgcaagcttt gaaagtggcc ctgtggaggc actaggctgg ggaaaaaggc 300
   tgcgggagga gggacatagg gtgggaggtg agtaggcgac ttgcttctca gattattccc 360
   aattagcacc aagttggcag acaaccccac aaacccacga agccttcggt cccccacaag 420
   tcacattccc tgtatttcag aataatcgga tcgtaagaaa acttcaagtc ccatcgtagg 480
   ttaaagaggg acaggctctt agtaccgccg ccgcccagta aaactacatg gaacaaaccc 540
   agggatcctc atctgcacag ctctgcccaa agtctgcagc tctgcgagtc cagccggcgg 600
   gggaagctgg gtgggccccg cagagagcaa gggccttctt gggggaggag cgggatgggg 660
   cgcagagcag tgcgatcgaa gagggttact gtgggactgc acaaaagcaa acccgtcgga 720
   ggagttttgc cagaaacacc accgcctgca ttgcgtcgga cctgaccatt tccaatgtga 780
   aattcccggg gaaggtcgcg agccgctagg ggccgttcgt gggcggggcg gcgggccaca 840
   ggggaagtag agttagcggt cggcttttct ggtaggagag gaaaaagctg tgctggcaag 900
   ggtgggaact gaatgacaac cccgctctct tccaaaccac cccctcatat tttccatcca 960
   cctcctcgct cctgccctcc cccgccctcc ccaacccacg cccgggtggg ccaatcgctg 1020
   ctcggtattc caggcgcttt ctcaggtttc tgctgatctt gcagcgccca gaaatggacc 1080
   gagcggaccc gccgccgcac gcaccctgct ccactccaag ctcctaaggg ctcctggcgc 1140
   gccgcgtagc cttggcgagg tccgcgctgg ggtgcggaga gcgaagggaa ctggagagcc 1200
   atgtagatcc aggctctcgc ccgcccgcct ccttcgggat cgaatcaagg gctcccatag 1260
   tgttaggagg gggcgagagt gctgtttatc gtcatttgcc tcggagcttc gagagagggt 1320
   ggtattttgc ttttccgccc cgcatcctcc ggaactccct gcaccggaga gaggacggcg 1380
   tctccaggtt gctggcaacc ggtgagaatg gggtagggga aggaacattt tcgccgtagc 1440
   tgctccgtaa agcgattgtc caactgagag gggcgtcgga cgagtggacc agggcggcga 1500
   gtttgcccgg cgcgtctcgg atgctgctgc ggcggccgcc gcggctcccg ccagggcact 1560
   gcaaagacga cctgccgcat tcccactcgg gctctccgct gactcagcac cgcccctgcg 1620
   ccaagccagc cggccaggta gggggttccc cagctcgggg atgcagaagc gggggttggg 1680
   gggaccgggt gggggaggcc gggggtgcgg ggatgctgtc cgggaccctg agcttccccc 1740
   ggcgtctctc ggcgcttttc cgatctctag tttaacgaag ttgtaaacag atcggctgtt 1800
   gggcattggg gaaagtggga tggaagagcc ccaaacttgg atttccgggt gtctgcgtgt 1860
   cgtctgtccg tgtgtgtgtg atagccctag caaacgtcca gtgctttctc aagctagagg 1920
   tctgtgttct tcggtgtctg taggtccgtc ccatctgaat gcttctgatt ttctaccccc 1980
   gtatcacttt ctatttctct gcagcgtgca tcgatcgccc tggtgggagc ttagaaggcg 2040
   gcaggcgaag aggggtagga ggggggagag ccgaggagaa gcagagaggg tggcaggcgt 2100
   ggggatctgc cgagccggca ctgcaccggg tcctaggaag gctctcggag gggaggggag 2160
   gccagggcga cccccgaagc aatggcccag tccgctagaa cggcactgcg ttaaggcacc     2220
   tgggatcagg aagaaatatc taaacaacaa caacagaaaa ccaacaaacc cccaaaccca     2280
   aacccaaccc tctgcaaaaa gctgcacccg gcccgcaggc gagggggatt ccaaactgag     2340
   tgaaaggcag ggtggagggg aaggcagcga gaggcaaagt cgcagatctc ccgacctgct     2400
   cgtgttgaag cacctccccc tgggcgtgag ggagacgcgc gctccggtgg gggggccgct     2460
   tgggtccccc ccacccctgg tccctggctg cttcccaccc cgggctctct cctggcctcc     2520
   cacccccgcg cccggcttcc accatgacgg tgatgtctgg ggagaacgtg gacgaggctt     2580
   cggccgcccc gggccacccc caggatggca gctacccccg gcaggccgac cacgacgacc     2640
   acgagtgctg cgagcgcgtg gtgatcaaca tctccgggct gcgcttcgag acgcagctca     2700
   agaccctggc gcagttcccc aacacgctgc tgggcaaccc taagaaacg                 2749
<210> 11
   <211> 3249
   <212> DNA
   <213> Homo sapiens
<400> 11
   atgaatgaat taatgaatga agtggtcact cccctcaagg actctacagg ctcttttgga      60
   ataagtgcat ctatacatgt aattcttctc ctggtcaaac cccggactga tcaaagtaga     120
   gtgtttttgc tgaatatggg gcaagaagct attaactgac agagtggttg aaagaagtct     180
   ggaaatgaga gaagaggggt cagaatgtaa aagaggaatc ctggttccct tccacggggg     240
   tcccgaggtg ctttgaggag ggagaaagag ggcgtcccct ctggggagcc cactctccgg     300
   gcttctactg acctggtctc cgcctcaccg gcctcttgcg gccgctgcag aagcgcactt     360
   tgctgaacac cccgaggacg tgcctctcgc acagggagcg cccgtctttg ctggggctgg     420
   agcggcgctt ggaggccgac actcggtcgc tgttggactc cctcgcctgc cgcttctgcc     480
   ggatcaagga gctggctatc gccgcagcca tagctgctca gcgagggcct caggccccag     540
   cctctactgc gccctccggc ttgcgctccg ccggggcgag ggcaggacct gggcggccag     600
   ggaaagggca gtcgcgggga ggcagtgcta aaatttgagg aggctgcagt atcgaaaacc     660
   cggcgctcac aaggttagtc aaagtctggg cagtggcgac aaaatgtgtg aaaatccaga     720
   tgtaaacttc cccaacctct ggcggccggg gggcggggcg gggcggtccc aggccctctt     780
   gcgaagtaga cgtttgcacc ccaaacttgc accccaaggc gatcggcgtc caaggggcag     840
   tggggagttt agtcacactg cgttcggggt accaagtgga aggggaagaa cgatgcccaa     900
   aataacaaga cgtgcctctg ttggagaggc gcaagcgttg taaggtgtcc aaagtatacc     960
   tacacataca tacatagaaa acccgtttac aaagcagagt ctggacccag gcgggtagcg    1020
   cgcccccggt agaaaatact aaaaagtgaa taaaacgttc ctttagaaaa caagccacca    1080
   accgcacgag agaaggagag gaaggcagca atttaactcc ctgcggcccg cggttctgaa    1140
   gattaggagg tccgtcccag cagggtgagg tctacagaat gcatcgcgcc ggctgcggct    1200
   ttccaggggc cggccacccg agttctggaa ttccgagagg cgcgaagtgg gagcggttac    1260
   ccggagtctg ggtaggggcg cggggcgggg gcagctgttt ccagctgcgg tgagagcaac    1320
   tcccggccag cagcactgca aagagagcgg gaggcgaggg aggggggagg gcgcgaggga    1380
   gggagggaga tcctcgaggg ccaagcaccc ctcggggaga aaccagcgag aggcgatctg    1440
   cggggtccca agagtgggcg ctctttctct ttccgcttgc tttccggcac gagacgggca    1500
   cagttggtga ttatttaggg aatcctaaat ctggaatgac tcagtagttt aaataagccc    1560
   cctcaaaagg cagcgatgcc gaaggtgtcc tctccagctc ggcgcccaca cgcctttaac    1620
   tggagctccc cgccatggtc cacccggggc cgccgcaccg agctggtctc cgcacaggct    1680
   cagagggagc gagggaaggg agggaaggaa ggggcgccct ggcgggctcg ggatcaggtc    1740
   atcgccgcgc tgctgcccgt gccccctagg ctcgcgcgcc ccggcagtca gcagctcaca    1800
   ggcagcagat cagatgggga ttacccgccg gacgcaaggc cgatcactca gtcccgcgcc    1860
   gcccatcccg gccgaggaag gaagtgaccc gcgcgctgcg aatacccgcg cgtccgctcg    1920
   ggtggggcgg gggctggctg caggcgatgt tggctcgcgg cggctgaggc tcctggccgg    1980
   agctgcccac catggtctgg cgccaggggc gcaggcgggg cccctaggcc tcctggggct    2040
   acctcgcgag gcagccgagg gcgcaacccg ggcgcttggg gccggaggcg gaatcagggg    2100
   ccggggccag gaggcaggtg caggcggctg ccaactcgcc caacttgctg cgcgggtggc    2160
   cgctcagagc cgcgggcttg cggggcgccc cccgccgccg cgccgccgcc tccccaggcc    2220
   cgggaggggg cgctcagggt ggagtcccat tcatgggctg aggctctggg cgcgcggagc    2280
   cgccgccgcc cctccggctg gctcagctgg agtgctagct ccgcaggaaa ctcggggccc    2340
   gggcgagagc caccgagatg gcaggtggga cgcagagccc gcggcagcca gagttcctcc    2400
   cgcacggccc gccgacccac ggaagagcga aagagcgccc aggtggggcc gagctggggg    2460
   ccgggcccct ggagcgctgg gaagcacagc gcgctctagt caggttccct ttcctggagc    2520
   cctccgcttc cagactccct tctttcctcc ctccctcccg ccacccctct ccctcctctc    2580
   tgtgtcttct gtctctcccc ttttctcctc tctacgcaat cctacgtgat tgaggtttgg    2640
   atgagaaatt ctcagaggca gagcgaggga actgcagctt gggtctgctc cgtccggtcc    2700
   ctcccacaag agaaacacaa ccacagtggg agttaaagga ccctaggtgc gcaaagaaga    2760
   ggtgggatgg gggagctgag aaaatgcagt ccacactctc tccaataagc ttgagcacgt    2820
   agaattctct gtttagttag gaagaaagtg aacactggag aaagtaaaaa tgacctcttg    2880
   gaccttatcg tgggccccac ctatggctca ttttggaaca ggaaaaagtg tttcccttct    2940
   tcttggaacc cagatttctt ggttctgtct ggaaagctgc aaagcaggct cagtccctaa    3000
   aaagagagcc caaataagca gcctgcacag aggatgactc caggtgcggc gagggagtga 3060
   tgtggacaag gacagtcaac aacaagctgt ggaatgcaat caggtctcca gacgtgaatg 3120
   tgacgacatc tgatgttgga gacactgggc agaggagttc tccaagttaa aatgcagcat 3180
   gaagcattaa tcaccctcca tttatgctaa agtctgggag cggctattgg tttctactta 3240
   caatttctc 3249
<210> 12
   <211> 1499
   <212> DNA
   <213> Homo sapiens
<400> 12
   ggggggcgct tgggcagcgg catgaaggat gtggagtccg gccggggcag ggtgctgctg 60
   aactcggcag ccgccagggg cgacggcctg ctactgctgg gcacccgcgc ggccacgctc 120
   ggtggcggcg gcggtggcct gagggagagc cgccggggca agcagggggc ccggatgagc 180
   ctgctgggga agccgctctc ttacacgagt agccagagct gccggcgcaa cgtcaagtac 240
   cggcgggtgc agaactacct gtacaacgtg ctggagagac cccgcggctg ggcgttcatc 300
   taccacgctt tcgtgtgagt acccgcgccc cctgctatgc ccgctgcagg ggaccactgt 360
   ccctggcccc ctggggcgtg ctccgcgctc gcgcccttgg gcccccgcgc gcgtgcacac 420
   gtggtggctt ttatttcttc gcacgtgttc gtggtcttcc ttctggagcc tctcccctcc 480
   cccagcccca cttctctcat ctctacagct tgaacctttt ccccgaggac acccaatgaa 540
   ctgcccggta gcttcaggct cccggggcga gagccaggca gacgcgggac ttaggctgcg 600
   cggataattg ggagcaatta ggtcccaaga tacgtaaact tcaaccgaac ggggcgcccg 660
   ggagctaggg aatgcaaagg gaggacaggc gcccgtgtga ggcttgagag tatactggag 720
   aggttaggag gtgatggcgg ggtaggacgg ggagaagtga gggggcatcg agggctaggt 780
   cctcagtcct aggggcggag taggggaagc tgctacttgg agagagctgc taggttttaa 840
   gcgcgcccgg aaacacgcct cgccaccacc cagccaccac caacggaaaa tctgtcagtg 900
   catgtagccc ttcctgccac ggagaaggtg gccaaggtct agaggaggcc agcaggccag 960
   gcgaagcaac gctcccgcgc tgcagggggc ggggaggcag cggggaacct ggggcgcagg 1020
   aacgcgggcg gaggtgcgat agcagaagcg caaatgggtc gcctctgaca gagatcgggc 1080
   agtgggttaa gtccccgttt gtggcgcgga gtcaaagagt gtgtgtgtgt gtgtgtgtgt 1140
   gtgtgtgtgt gtgtagtaag ccttctccat ctagcagaga atgcttaatg agaaaatgat 1200
   tggaagcaaa tgtttatttt tcccttaggc atttaaaacc tttcagtggc tttaaagttt 1260
   actactgttt ttcccacaaa gtccattcat tcagtctcct attagagtta cgtttatctg 1320
   ggcattttaa ggttgttttt ataatgttac ctcgtgtcta attctttttt tcttcctctt 1380
   ctccttttgc ttcctctttt tttagtatta ttatttctgc ttcttttttg ttaagatgaa 1440
   atataaagac atcaacctta gaagaccagt agagaaagtt gcagatactc gctgataca 1499
<210> 13
   <211> 1499
   <212> DNA
   <213> Homo sapiens
<400> 13
   ggagcgggtc gaagtacctc atgcgccgct tggggtcgcc cagcagcgtc tcggggaact      60
   ggcaaagggt cttcagctgc gtctcgaagc gcagcccgga gatgttgatg accacgcgct     120
   ccccgcagca gtcctgctcg cccgcggccg gcagtgaggg cggcagcggc tcgtagcggt     180
   cgcagccgcc gccgccacag ccgccttgag gcggggcccc tccaccatcg gccacctccg     240
   gctccagcag gtggtccccg ggcaccacgg tcatgtcggg cggcagctcg cggcctgcgg     300
   cgggctccgc gtagccgtgg ttcaccagcg tgtgggcacc gccgctgctc gctgggcgct     360
   gaggagggtg ggcgcggtgg cgggctgagg gcggcggcgg cgagcgcaga aggctgaggc     420
   gctcgtccat gcggcgggga agaggcggca gcggtgaggc caggtcgctc ctcctcgcgc     480
   tccccgccct ttcgccgcct ccgcccccga gccgagccca ccgcctgttg cagccaaagc     540
   cgcgatgctc tgtctgggtc tggcgcggtc agccgggctc ccgcacgggg acgcctcctc     600
   cctccttctc gcgctctccg ccccctcccc tgcggggcgc gcgcccgcct ccgcgtcccc     660
   ttaggattcc cgcccaccgc gcgggcgcgc gtcccgctct cgggggcagc cgccgggcct     720
   gcatttcttg cagccctcaa ggcccctcgg tgtcagcgaa agagccctca tgttgtacct     780
   cggcgccccg cgggaatgcc cacccagcag agccggccca cggggagtca ggctgccggc     840
   ccgggcccct aggctccgcc cgcttctggt cagcgcccct cgcccccggc ccgcctggcc     900
   gcgtcccagt cgccagggtt ttcggcccgt gggccgggag agctcccgcc gcggccccgc     960
   gggcgccggc cccctggcct ccacacccct aggtacagcc cggggagggc aggcgggccc    1020
   agtgtccagg gagggagtgc aggccaggcg ggcgccctgg gccagaggca agcctggcgc    1080
   cggcatccca ggttcccttg agggtcgagg accgccaaac cctggggagg agcgggggtt    1140
   taaacaattt agcttctgct aggatgcgaa gccaaaggga gtaatgggtg ctgatgggct    1200
   tcgcaaacgg agtccgaagg aaatggattg ttaaaggcgt tcgggccctg ctgctttagt    1260
   gaatagttca cacccgtttt cgcagcggag atgtcggcca ctgggaagaa tcaaggacca    1320
   agtttctgat tgggattagc agtgacagcc tggtctttat ccactacaca ggtttcctgt    1380
   tggcggggaa ataagaggaa aaatgggaaa ggaaattcac gaagtcgaag ttgtgtggtt    1440
   agaaagtcca gctttatgac tcaagcctgt cgtggaaggg atgagagcag gacctgtac     1499
<210> 14
   <211> 1249
   <212> DNA
   <213> Homo sapiens
<400> 14
   tgcgggtgct cgggcgccaa ctaaagccag ctctgtccag acgcggaaag aaaaatgggc      60
   tgtgaaaaag caaaaggcct cgtctttgaa tgaaagttaa acattaaaat ctgaccctag     120
   agttgtctaa agatcgcgga attttgaagc tccggcagag cggactaaaa aacggtgcta     180
   tgagagatgg tgagaatact ctaggcatga acgtgtgcgt gtgtgtttgt gtgtgtgtgt     240
   gtgtttcatt cttcccgcaa aacaattttt tgtttttttc ctattcccgg tttgttatcg     300
   gcctagggcg ggagaaccac gcagcggctt ctgggcccta aggacaaaag agttaaaaca     360
   atgaggctca cccgggaaga gacgctgccc tgggcacaat agggtcgcct gcattactcc     420
   tccatacaca catctttaaa tgtgtccctg tgtgtgttcg ttagggtgct gtattacaga     480
   aaaagaaagg cctaaaaaca cccccagccc tggtcgcgcc tttcgctacc gcctgagtct     540
   ggagccgaca gctccacctc ttctgctccc tggaccgccg cgtctccacg ccacggcgcc     600
   ctttttacta aaagatcttt tctcatccta tcagcaaatc gttaagaaag gcttagccat     660
   tgcgggggct ccaacttaag gattcccccg gcccactaaa aggctaggcc cggcctgtag     720
   cccagctccg cagaaagcca gagggtgctg ggctttcagc ttcttcctcc tagacacttg     780
   ccccacaaat atatttcgtt ttctctaatc caaataccca tctttttctt ttttaaaaaa     840
   tgataacgta atgggaaatg accaaccgaa ctctgttaca taaagttagt tctgttagat     900
   cttccacccc acccccatcc cgcgggagcg agtaaataga attcatgagc ttagctcccc     960
   aggttcacgc tctggaatgg tttctttttg cctcattccc taagttttct ctcttctgcc    1020
   tcctgaatgg agctcaggct aaggagaacg gcagaaagag caaactctga tctgaatctc    1080
   taattatgac cccatgtatt acccatttga acataaggcc ctagacgggc tccgtgcgat    1140
   ctggggcctc ccaagagaaa acttccccgg gacaggacgt ctgccacgcg cagctaaaca    1200
   acttctgttt tttccgccgt ggggaaaata aaagaacctt acaaattct                1249
<210> 15
   <211> 999
   <212> DNA
   <213> Homo sapiens
<400> 15
   ttagacttct gtatgcctct tttttcatct gtaaaatggg tattaatagt agtacctatc      60
   tcatagggct tttgtaaggc ttaaatgagt caatacacaa agcatctaga agtgtgccca     120
   gcatatatcg gttattccct accatgataa tgctcacttg ggccactgca gtagtggctg     180
   tttcaaatca ctacagccca tctttagtat tttctcttat cgttaccgag aatgagcttt     240
   tcacaactca aatttgtctt cttgcttaga acatgtgaat aggttcccat tgctcctaga     300
   aaaaaggtag aaaagcttcg acatgccggt gacatgctgc acggcttcat ttgctgcctc     360
   gtcatcctct tactctacat gctcatggcc acatgagtca tcgttcagtt gcgcaaacat 420
   gctgtcctca ctcagacatc cccaccctac tcactggatc cttccactgg ccgtgcccct 480
   cactcacaaa cttgccttct ctctccttat cttccagtcc ttctttcaat ttcagcacac 540
   aaatcacttt ctcagggaag tgttctttga acacagcccc ctttccagac aaagagtttg 600
   tctggaaaga caaactgtca cagagaagtc ttcctttccc tcagtggcct gatcccagac 660
   aagaattgaa catttgttgg tggatttttt taaattaagt gccaccattc ccactatgtt 720
   gaattaatta aacaatattt caatataaag tagaacttat atcaaaataa cattttagcc 780
   tgcaatcttt ttattggaat ctgagagtgt aaaatataaa agatgcctta ttcctgccta 840
   atgagaatct cctgaaagtg gcgattttct ttaatcagca aacacaaaag tgtatgttaa 900
   tgagatacat atttttcaag ccccctaatt ctgcatcttc tgtgtccatt tcactccttc 960
   atctcttctg caaaggtcaa aggatcctgt ccagtgctg 999
<210> 16
   <211> 499
   <212> DNA
   <213> Homo sapiens
<400> 16
   tcttcatgat caccatcttt gtggttttca agatgattgt tagatcctta tcaaaatata 60
   aacaattggc aatggttcca attgtgtcaa caaaagccaa cttcaaacct gtaatcctca 120
   tgctgagtgg agaggctggt gccccacccg ggctgtgaca tggtggcttg ggagatgtgt 180
   gactcagata tgtcagacca tgagtgaggc acccaacctt ccctcccagt gacctttgaa 240
   gtaaggcgaa ttgaagtccg ctggtctcca gacaggcacg gtaacgtgca cgcatcggat 300
   gtggttcccg gggaatggtg ggtgattgtc catcttccta acagtcctca aatgaggact 360
   cagttccagc tcttaacgca gcacaacaga gttcttaata gtaaaagtcg tacttttcac 420
   tcaccgtgaa aagcaagtct gcacattgct agatatgtcc cagtattatt atccaagctc 480
   cagaaacgta ctcagccac 499
<210> 17
   <211> 999
   <212> DNA
   <213> Homo sapiens
<400> 17
   tcaggtgtgt tgatggttct gtttttgtat taaatagagc caacctcttc ctacttctgt      60
   gttcttgctc taagctggct agggacgagg ttaccagcga cccaattcaa tcagcagctg     120
   ctggctttaa gcgggtccag gagttcactg tgtgaatgca gccattagct ggctttagac     180
   ttggagagat aatcgatatt tttctgggcc gtcttggtct cgcctctttg gcggaagaaa     240
   gcagcaccca cacagtgtgt aacatctgat cccggtccag ctcccgcggg ctgggctctg     300
   cccgttgtga gtggccgaca gctccgccag cgcctgtttc catctgccga gccatccttt     360
   cttctgaatg tgaactgttt tcttggtttc tttctggcat cagaaagcaa caatgagtga     420
   ttatctgatg cagcatccct ggggccccag gtgctggtga ctcattcaag tctccctgca     480
   aaccaattca ttaaacctgc ttcatctggg acgtgctgag agtggaggta tatttcaaaa     540
   gcggtttggc agcaacgctg caattaaaca aggagggaag gagagcagag gcggaggagg     600
   aaggcgcgat ttagttgtga cttgaacacc gtctacacca gccaaagaag gctggtccac     660
   actggctttc agctgagggg aggggcagtg cccagatcat gtaatttttg aaattatgtt      720
   tgtaattaac ttcacgatat ctccagggaa ttctggaaag acagcaagaa aaaacactgc     780
   agtatctgtc ctatcagata ctacaaagca cctaatgagg tatccttagg acattagaaa     840
   aaacactcac tcaaaaaagg tagaattctt cctttgtatt cttggggtgt tggttagggt     900
   ggccgggttc ttcgttaagt tcatcgttaa gcaagcaggg tcttgctgcc tgtgagatga     960
   ttcacggagt tttagttttt actcttcagg cacggtctg                            999
<210> 18
   <211> 999
   <212> DNA
   <213> Homo sapiens
<400> 18
   tgacatttga aaggcatacc atgaagggac tttggctttt gttagagaac ttgagtcggg     60
   gtgagtccac ctgggcccct ggatgatact cttttaaaaa ggcaatgaga gtggccaagg    120
   ttgtgttctg gaaagtgatg gtcacaacac acaaccgggg aagtataaca ccatcttgaa    180
   ttgaaggaga aattaatcac gactcggaag tattggtgtg tagagagaag gatactcagg    240
   tggaagagca ctgacctgct ctctgcgtag atcaggcatg tatttcatct caccgtgagg    300
   ggaggaagtc atgccaggta aatctcaagg cgcttccaca cctgaaatgt tcctggcaaa    360
   tacatgggtt ccccggtgtg gaggtatgag agttcttttc tccttcaccg cagacaggca    420
   ggtctgtggc agtttcaggc tctctgctgg attcacactc ataagtggtt tgtttacttc    480
   cttcagcatg aaggaagagc tgaagaaagt gctggcatgc gcttactttt gaaattggca    540
   gtgaagtgat tgtatgaagt cattggtcag cataatgcca atttcaattg tgtgtgcgtg    600
   tgtgtgtacg tgtgtgtgtt tggtagtgaa gaaagctttc agaaaaatct gctttgctat    660
   ttgaaatgca acgtggtcct ctggatgtct ttcttgtact tacatggttt tttttttctg    720
   tatggctttt agtgtaattt ctctttaaaa cataataatt tagcaattag aaaaggaata    780
   atgcatgctt ttcttttttt aagtctgatg ttaaatcagt ccatgggttt ctggttactt    840
   cttactgcat cacagaaagg tctattgctt cataggcatt taacatgttg cgattatctt    900
   tatgataata aatctttatg atgataatga ttatgtgcta cgacaatatg accaggaaaa    960
   aaaattattt tctgaggggt ggaggcgttt tattttcca                           999
<210> 19
   <211> 999
   <212> DNA
   <213> Homo sapiens
<400> 19
   aaaaaaaata agaaacatac atacactcta acaaagaatc ccttgcggag tttatgctcc        60
   agccttttgt ggttgtgtct ttgcagccac acagggatgg tttgcaaaga atgtagcagt       120
   atttgttgca tctagcaaga ttaattggtt taagcagcag tctttcaaag cagttacaac       180
   aataatattt cggttctttc agaaagacac aaaagcagcg gaaaagcaga aaggcttttg       240
   agcggccagg agtgcagagc gccagcaaag tgcatctatg atagactgta accttaccaa       300
   aacttttctc ctttttctgc atgagttgac ttaggcgtgt ctgagttgca gcagcttcgc       360
   attgagcacc aaacccaaag gtagaagtag aagggggtct ccttgatttc gcttaagtgt       420
   ggacctggtg cgcagcctac accgccgagg accgactatt gtgaagccac tttgggagcg       480
   ggtcggagtg gcggcagggg gtgggggaag ggatgaggac ggccagacaa gacagggcgc       540
   acacacggag cccctcgcag tgtgcaaaat gatggcgaat gacaaagcca catgcttccc       600
   taactctgcc cgtaatccta aaatcccagc ggcccctttt agcttcctgg taacaaatgg       660
   atttgattaa actgtcacat gcagcgttag catagcatat catgttcaat atgaaaaaga       720
   tcataaatct gacttgtatt tcataacagc aatctgagta gtccccgtaa aaaatgtgct       780
   gcatcagttt gaatctcaat ctattaggat ataggcacct tggtccaggg accttccctc       840
   ttctagccac ttctgcccct acccgcctgc cccccccccc cgcccccatg cccaaacaca       900
   gccacttttc cacggcaaag gaacaacatt ttgttattat ggctgcgtgg agagaggcag       960
   aagcgtcaac aaggaccaaa agattgtaat atcaactct                               999
<210> 20
   <211> 999
   <212> DNA
   <213> Homo sapiens
<400> 20
   gtctcgaact cccgacctca ggtgatccac ctgcctcggc cccccaaagt gctgggatta        60
   caggtgtgag ccaccacacc cagccaacat caccaaattt ctaaataaag atcaaaacac       120
   ttctcatgtt aaacattgaa acgaatgtaa gctataccta tgtttaagaa gaattaataa       180
   aaacaggtaa gataatgatt tacccaatta tttcagttca gggtctcagg aggctggagc       240
   ctgactgagg cactcaaggc acaaggcagg taccatccct gaacaggaca ccatttcact       300
   gcagggcaca ctcacaacca cacccatacc cactcccaca cccacgctta ctcaccctgg       360
   gaccactcag tcgtgccagt taacctaaca tgcacacatc tttggaatgt gggaggaaac       420
   cgaagaacct gaagaacatc tatgcagaca tggagggaac atgcaaattt cagactgcag       480
   ccccagctag gaagcatttt ttttcctcat caacgttata aggaaacgat gttgaaagaa      540
   aggacatttt gtgaggacct ggtgtactga gattcttcta tacgtcatac agtcacactc      600
   tcctactcta gggtcaagaa agaaaccatt cagccaggct gggcatggta gcccacgcct      660
   gtaatcccag cactttggga ggctgaggcg ggtggattgc ttaaggttcg gagtttgaca      720
   ccagcttggc caacatggag aaaccccgcc tctactgaaa atacaaaaac tagccaggtg      780
   tggcagtgtg tgcctttagt cccagctgct tgggaggctg aagcaggaga atagcttgaa      840
   cccgggaggt ggaggttgca gtgagtcaag actgtgctac ggcactccat ccagggtgac      900
   acagcaaaat tccagctcaa aaaaaagaaa aagaaaaaaa aaaaagaaaa agaaagagaa      960
   aaaagggaaa gaaaagaaaa accattcagc ctctcacag                             999
<210> 21
   <211> 1749
   <212> DNA
   <213> Homo sapiens
<400> 21
   accgtgaaac taggccagag aaggggcggc cgctctctta ctagtgtctg ctgctccacc      60
   ccagggtccc agccactgaa tggcgaaggg agtggggagc atccctcagg gagccccagt     120
   aatcacccct cccctgcctt tccacctcat tcctcctttc tccctccttc agccttgcgg     180
   gcagaccctg tgggccgcct ggaccgcgcg caggagggct gggattgcgg tggctgaacc     240
   ctgcggacct ctcccatctg ctccaccccg accgcctgcg gttccgcgcc caaggctgga     300
   cagaaggcag gagaaattta taagaaacag acaagcaaaa accctggctt cttgtcactg     360
   attttaaaga acccactgag gtcactgcga tgggtggagg gaagcgagaa tggaggaata     420
   caagccaaag ggaaggaagg ggacgaaggc ggacagggag tgacctcttc ctccaacccc     480
   cgggcccgct gggagcggcg cgaggccaga ggcccttgag aggctcgggc tgtcctgggg     540
   gcctcagtcc tctgcctgta ccccatgggg gaccctgctg ccaccaggcg ccccgcactc     600
   actcgacctg cagcgtgctg ggtttaatct tcacctcaac cttgtaggag gagccggtga     660
   gcagcttgat ggtgcggttc tggccgaagc gctgcccgtc caccttgtaa aagaccgggc     720
   cgtcattagg ctggatgcgc agcgcgatgg agaggcgcac gaggcccggc aggtccccca     780
   tgtctgggcg agggtctggc gcggcggctc cggggggcgg aggacagcgc cggctgcggc      840
   cgagtggctg gagcgcgagg ggcggagagg aagcgcgggg agggtgaggg aggtggtgga     900
   gctgaggctg ccgctaggaa cccgcgccgt cgccgccgtc cgcccgggct tttgaggagc     960
   agctccttag gctgtggccc ccctccccac tcggcgagga agcgggccca agagacggct    1020
   ccaaggccgc gcgcttcccc atcccccgct ccagtgctgc gccctccacg cacccgaagg    1080
   ctcgctctgg cccgcaggcc gccgcgcaga tccgcgcagc tgggggcgag ggagttaatc    1140
   ctgtttacgc accacaatcc ccttcagctg gggaagcgga catttaggct cctcctagaa    1200
   cagccccggg caggaggagg agaggtttgg gaggcactgg gaaggcgctg gagttaagcg    1260
   accactatgc caaggagcga gacccccgga atctggatac cgcctcggcc agctacgtga    1320
   ggtggacact gctgctcgcg gatccggcgc cagccaggcg ggaggaggct gagggggggt    1380
   aaagggaggc gggaaggggg gacaggaaac cgctagccgg tgatttaaat ttcaggaaat    1440
   atgagtcttt ccaaagctta ggggaaatgg ccgaggaaag gcgcaattcc acgtgatgga    1500
   gccacgctgg atgaggaatg gatgcaagag gaagaaaata accatattca aggagctaca    1560
   tcttcttgtg ggtgtacatt tccattatac gtatgctcgt cccaaaaatg acacatacat    1620
   aaatatatgt aatgaatcac atatatttac acagattttg aagggtgagc tattaaccct    1680
   gtaaaaggca actgacatga gcctaaggca ttctggtgac aaaatggcca agaggtggga    1740
   tgggtcaaa                                                             1749
<210> 22
   <211> 999
   <212> DNA
   <213> Homo sapiens
<400> 22
   tgtgtggggg cgaccccagt gccaggaggg actacctcgg tttcccagtg gcccaggtgg      60
   ggtcggtgca tgggcgcctc ccccatccgt ggttcccgcc agccgcggcc tcgccaagtc     120
   ggctgccgaa accacgcgcc agcgcccttc cactcccccg cccgtcgtga ccacacgact     180
   gagccagcct ccaggtctag aagctcctgc cacccagtct ggtggcaacc agactgggag     240
   atcggcccga gctccctggg cttctatgca gccagcaccg agtaggcgcg tgctgtgtgc     300
   ctggcgagcg aggggagagt tgggacacct ctcctgcagt cctcttccca gccaagcccc     360
   tcgcgatccc ccgccctagc ccagccttgc cctcccgggc atgaggttgc agcgcagagg     420
   cgtctccctg agtaaggctg cacacgtaga cttgactcta gcccatcctc agcctcagcc     480
   taagctttgc cgagctggaa cctccacttc ctcgcccacc gcctggcaca tcgaagccga     540
   tgtgcctcgg gccggcgggg aggccaaaaa cctggtgctg ggctgggcag agttgcgctc     600
   tctgggcctt gtttgtggca gcgggaccat aaggggctcc tccggattct gtttgaagtc     660
   aattcctgga acatcagata ctgtcagtca aagataaata caagaacaca ttcctctgcc     720
   tgttacaatt tccccatggc tcagaatcag ctggactggg ttctgcctcc tggaacaggc     780
   agcaagggac agaggctgtt aattcccctg acagccaggc acagctgggt caggaggccc     840
   cactccaagg agaataattc tgtcttccct tcctgaggat gcaaaactga actcggaatc     900
   tctatgttcc ccatccccca catacctggc ataaacaatg ctcaaagcat gcttgtggaa     960
   tatgttctcc attcattcca ggagtgttta ctgagcatc                            999
<210> 23
   <211> 999
   <212> DNA
   <213> Homo sapiens
<400> 23
   gtgcataagt ggcctcgagc tttttcctca ttatttccag caaaccccgt ctctgtctac      60
   ttacctcctt ccttaacagc cttttcctaa ccaattcttt ctgctcccct agaaatatta     120
   cattctgcaa atgcgaaagg aaaagaaatg ggtatctgct cagtgccgat ttcagagagt     180
   atctacaaag cttttctctt ttgcacagat actgcactga agactcggag gggttgagcc     240
   gctggagcca cgcaaattca gacacctctt ccgccccagg tcactctact cgcccacgct     300
   gcctgccaca cccatccggt tgtgcgggac actccccgcg tttcttcagc gattcttatc     360
   gggctccctc cttgttcaat aaaggtgaag ggtgtggggt tttctgtgca tacgctcagg     420
   aagttgagtc ccggtgaaac gtgtcaggtt gccatttccc aggctggaaa gattttccca     480
   ggacgggtat gaatagacga tgaaagtgca cactcttacc cggctgcccg acccaggtgc     540
   caggcttctg actcaggacc atctgtgggt gcgagtgcag ggaggtgagt cactgcagcc     600
   ttgctcagtc cccctgcaga ggtcagatcc tgggccccaa aagctgctcc aggatgaaag     660
   cctgctctca gtgagactaa aatcctggtc atttgtgttc tgcagtcatg agcatgtaac     720
   cctaatgtag aacaacaact tagccaatga ctattttttc tgttcatgcc acagtacctg      780
   aaggagaatt gctgcttctc ttaatggtgc ctgccaccca acccaatagt tagcatgtga     840
   acgtcttgct tgagatcagc ttctgggtgt aaaaataaat ttaaatatag aaaattcaaa     900
   taacacccat tcatttatca aagatctcaa atgttctatg atcaaggcaa aaatctagta     960
   gccaaacaga gggttcccta gctggtttga cagccacac                             999
<210> 24
   <211> 749
   <212> DNA
   <213> Homo sapiens
<400> 24
   tagcccctgc taggccttac cttccatctc tctcctgtca ggaggaaaag cacacacgtg      60
   aaggaacctc agctagatga tctccctctc tggcctgtgc cgacatgtgt gactgacaac     120
   acgatggagc aagaagtaaa gcccgagggg taaccttaat ccctctgacc tgcgacctac     180
   tgctttcgcc cccaggagcc tttttcttct ctgggcctac tgtaagcgcc ccagtggctg     240
   gaatggaacg gtttccagtc tggaatggtt gacactaatg gccaaagagt agggggtccc     300
   acgtgcttgg ttaaaaggtg aaagtaaatg cgggagtctg gaaggacttc ctataggcac     360
   aaaatctgcc ccctcccccc caactttggg aaatatggat taccaacagg tttgtgtcaa     420
   ctcagtgttt caagcacctt gcaagtttca gtttgcgaag aaaagacttt ggtgagacca     480
   aagccactgc ttttaaaatt gtttaaaatt ttacaattag tacacaaaag ggatttatac     540
   tatgaataaa gacttcttgg gcatttatgg atcataagtt aagaacttct gcactagaga 600
   tatatagagt acagtacaga atacagtaca cagatctttc tgggacagaa gctgtatttt 660
   agtttggtca gtatcttatg gctaaactgt tatgtgaatg agaagcacca gcatattgta 720
   tagtgttcca gtaatcttct agggggttg 749
<210> 25
   <211> 749
   <212> DNA
   <213> Homo sapiens
<400> 25
   aacagatctg tatcattttt caggaagtgg gagacagtgt ctcactctgt tgcccaggct 60
   ggtgcagtgg cacaatcaca gctgactgca gcctcgacct cccgggctca agtgatcctc 120
   ccacctcagc ctcccgagtg agtagctggg aatacaggcg cgagctacca cacccagcta 180
   gtttgttaag tttgttgttg ttgttgttgt tgaacggctg ttgcccaggc tggtcttgaa 240
   ctcctggcct caagtgatcc gcccacttcc gcctcccaaa gtgctggaat tacaagcatg 300
   agtcatcgag cctggcccag atctgtatcc tgattgcggc gatgatcgca tggatggatc 360
   tacctgtgtg atacgatgac acagaaccac atacaccctt tatgccaatg tcgaactcct 420
   ggttttgata ttttactaca gttacatgag atgtcaccca gcggggaaac tgggtggaca 480
   gcaggggaca agggcatcct ggggctcctc ccctggcagc ttctactctg ggcccccatg 540
   gagctggcga gacgctgaga gctgcactac agcagaggcc cttccttcct gtcttttcct 600
   gacgtcccat ctgtactaga agtttcccct gttgtgcagc tccctcacca cgcagccctg 660
   aatgagctcc cccacttcta actgcctcct agaagcccca acttcacagg ggctacctag 720
   ggttggctgg cattaactgg gaaaggcct 749
<210> 26
   <211> 499
   <212> DNA
   <213> Homo sapiens
<400> 26
   caggaagctc ccaaacactg cctggtgtga aaggctctgt gatggagctg agcacatgag 60
   gtgtgggagc tatacccagg aaaggcatct caccagcctt ggagtccaaa tgccttcttg 120
   aatggacatt taagctaaga caagaagggg gaatggagtt ggggaggtag aatattctag 180
   tgacagggaa gcttgcatac agatctgcag gtgagactgt ggctccttca gggagacaca 240
   aggagcaggg tacagaggag gacagagtgg gaggcactga gaggagggac tgtggtgaag 300
   aggaacctga gttgccggcc gtgggagcct gctctgccag cctgacgagg ctgtactcca 360
   ccctgaggac agtagagact tactgcaaga gttttaagca gaggcgcaat cggatgtgca 420
   ttttagaaag gtcgcactgg ctgcagtgtg gacatagcgg cagtgagaga ggctggcacc 480
   ctgaagtgca gagtggtgg 499
<210> 27
   <211> 999
   <212> DNA
   <213> Homo sapiens
<400> 27
   caccctgcct gtttttgtat ttttcagtag aggcaggatt tcaccatgtt ggccaggctg 60
   gtcttgaact cctgacctca agtccacgcc ccttggcctc ccaaagtgct gggattacag 120
   atgtgtgcca ccgcgcccgg cctgtaatcc tactggccgt caaatccact ttaaaagcag 180
   taaaggcatt tgactccttc ctgtttctcg ttccttctca cccccactca ggccatctcc 240
   cctcgcccca ccacttcctc cctgcaccta cctttcttcc ttcctttctc ggtgaagtga 300
   agggtcacct ctcattgtgg aagaaggact agtaaagcca gctttaaatg aacattactg 360
   ggttggccta tgccaggcag gcgcgaggtc tctattcccc atgtgacaat caagctgggt 420
   gcgttcacgc ccaggatgct ggggttgtcc cacctctagg tttggagtgg gacgacgagg 480
   agaagcaatt tgttcaggag cagagaaagt tcgcttggct gtgactcatc gcctctccat 540
   tgagagtctc cggcgggtcc gtgatcatcg gacacgatca tgatccgtcc tcaggccccg 600
   cctgtgcaga gtgcgcggag gccaaggagt tattggcaga aaagcaagag cggaatgagc 660
   ttgcgtactt gaagtctgtg gccgtctgcc aacatctcct tcaaatatga acattcttat 720
   tttcgctctg gaagtttttg tcaggtttat tgcaaatgca agggtggtga gcagacagaa 780
   agaaaatggt atttactgag ctggaaggac tgttttctca accgtttctc aagagcacgc 840
   aaggagacgt gcactttcct gggtgacatc aggttctccg tggggatttt aatccaaatc 900
   agatatggcc ttgttttacg agggatcctc ttgggtctca gggtgtgagg attcataata 960
   agtacacgtc catccagtac atggcgaaga ccattgtaa 999
<210> 28
   <211> 749
   <212> DNA
   <213> Homo sapiens
<400> 28
   ctgtattcca gcccctgttg gccaccttga cttgtgccct tgtgtagtgt acaaccagca 60
   caaccataca taccttgttc tcaaattcct tactccaggg gccgagtcac tgacttactg 120
   gttatttttt tctggtaagg acctgaccaa gctctaggta gtcctggcca gcagaataac 180
   tgatttagtg tgcaggagac cagttagctg gaatcataaa tttccttatg gcaaagcaga 240
   agcaccgagt gtaacactca cctctagctg acctcaaagc cggacaaggc ccatctagaa 300
   atggccaggc aggtggaaga gcaagcgcag aagccctgag atgagaaaca caccagtgtg 360
   tctgaggaac agtgatcagg ctagagcaga ccagagtagc aggaaagcag tgaacgaggg 420
   gcaaatcagt cagcaggaga gtgggagcga ggttgtgtgg ggctttactg gccactgcaa 480
   agactttttt gattctgagt gagatgggag tgggaagctt tggaggattc agagcaaaga 540
   aggggtataa tctgacctga ctttcttaaa gaatacaatg gcttctctat ggagagtcag 600
   tgtcaggagc tagtgtagaa gcagggagac aggagcttgt ggatgaaaaa gccaaactct 660
   gtaaaatatt tggagagatt tattctgagc caaatctgag aaccatgacc gatgacacag 720
   cctcaagagg tcctgagaag atgtgccta 749
<210> 29
   <211> 999
   <212> DNA
   <213> Homo sapiens
<400> 29
   tcctacatag aattcgttct tctttatcct attttattac caaaattaca gaggggttga    60
   ttggcaagtg ctattctctt tatcagattt tgaaaacagt gtttctaagt ttcagtcttt   120
   tccccaagat gggaaaaggc aatgaggaga aaattccaac gcttccgatg tctgcttcct   180
   tcccgtgttt tccaccgtag caaggtaagg actgcgtcac ttagacttca atcacaaaat   240
   gagaaaccac accctgggct aaccatgagt cactaacagg aagatgtagc gatcactact   300
   aggactggag atcaaaggga aaggagtggg gttaatggaa cccgcaagct tggaatagat   360
   cccctggttc caggacttca acctcttagg agagggtaga gccaacctac cgctgaaacc   420
   tctggaattc gtagaggatc caaagaccct ctgaggcgac taagacctct gaaggtaaga   480
   tggatgtttg atggctgtgc tggtatccct ggggctgaca atgctattgg acctgggagt   540
   tatggaatat atgtaggcaa aacgtgcagg cacaaagcta ctgctgctgc caaggcggaa   600
   gctgtgtgtt actcaggtaa cattgacata aacagctatc agacacttct gtcaggtttc   660
   cggtctctct agttccccca acggcaaata ctaacagaga ggatgggcaa agaggaaatg   720
   gagtgtgtag gttcccgttc tccctgtgac aaagcgcaag gataaagagt aaaaagggga   780
   aggaggcttg gaattgaaag acagcgaatt aaaacacaca gaacccattc gtgagctgtg   840
   tctttgctca agaaacccaa gtctaatttt ataacaaata aaacactaaa attgctttaa   900
   aataatgaaa aagcaggaat aagctggcat taccttattc aatagccaac atttatgatt   960
   ctaaactata aaagaccttg gggatttctg tcagtggtc                          999
<210> 30
   <211> 499
   <212> DNA
   <213> Homo sapiens
<400> 30
   ctcttgccac gtgaggtgcc caaatatggt cggactcagg aggagccagg gagcgcttgc    60
   ctttctcctg ctaatgggga ggaggctgga acaaatgttt ggagttaaac acaatctgca   120
   ggaaagcaaa tggggactcg gactcgctcc tgggcgagct gaaagtcggc tgcagcagaa   180
   gctcctgcct tgggtgatcc atcatttaat aaaccccaga gaatccagtg tccccggcag   240
   gctttttgct cccctgctct cttgccttct gaggccctgg gtcgtccccg cagctctagt   300
   cgccctgtta gaaacgggag gcgcccgagg gccgggtggg cggctgcctg gacctgggct   360
   ggcgcgtcgc agcgcctctg gtcccggcag cctgggggca gatgctgctg cagggcgtgt   420
   ctggggctgt gctcatgtga tgaagcgagg gaaaaaccgg ggggaggggg gcggaggcta   480
   agaggtggcc ttttttttt                                                 499
<210> 31
   <211> 749
   <212> DNA
   <213> Homo sapiens
<400> 31
   acaaaactaa cattggttgg gtagaaaagt tgaatacaga gttaataact tttaatttta      60
   gcagttaaag actttaaaac aaatagatta atcacaaaaa ctcaactgct agcttcactc     120
   accatcaatt cattaaagaa aaggtccaaa ttaatgttct ttgttttgca agacctctca     180
   aagctttgct gcaacctact tttctagcct caattcaacg attcctctgc cttagcctgg     240
   ccacacggct cactgttccc ggatttccca ggtaatcact tctttggcta gaaataacca     300
   acccccacag ccacttccac ccactgctgt atttgactgg ggaactctga ctcatccttc     360
   caggtcaagt ttcttcatct gggttcccat tgcactatat atactcctct attagagcac     420
   tcgttacgtt attttatagt tatttgtgga ggtctttgtc cctttcacag gattacgaag     480
   aagggcatca tggtgagttc actttctttt ccccagcatt tagcaagcat aattaacaag     540
   cacacagtaa gctcccagta aatggcctca agtgaacaaa tcaaaggcca acttcctgtt     600
   tgtgatgtct gtattcatca gaaattttcc tgagattttg agcattgttt tcagtgtgca     660
   taattcccct gaaacccaag tttaatatta gctgaagagc agagcacaag gcacttgtag     720
   taggactcca agaagtgtgc cactccaag                                       749
<210> 32
   <211> 749
   <212> DNA
   <213> Homo sapiens
<400> 32
   gaggatggcc tgaatccagg agtcggaggc tgcagtgagc tgtgatcaca ctcctgcact      60
   ccagcctctg ggcaagtggt agtttgtggt agtttgttat ggcagccgtg gcttgccagc     120
   aaccactaga agctaggaag ggacaaggcg acagagtgag accctgactc aaaaaaatct     180
   ttgatgagct ggattgactc ctgaataatt ggaagggtgt gttagcctcc tgcggccgtg     240
   ataacaaacg accacaagct gggcagcttt aagccacaga aatcaaagtg tcacagggtc     300
   acgagtcatc cgaaggctcg agtggagact ccctccttgc acctccccag cgtctggtgg     360
   ttgctggcaa gccttggtat tcctctgctg gcagctgcac tgctccagtc tgtgtctgtc     420
   acttccctgc cttcttctct gtgtcactga gtccacattt ccctctcctt taaggacacc     480
   agtcattgga tgaggttcca ccctaatcca ctgtgacctc atcttaattg gattatatct     540
   gcacaaaccc tatttccaaa taaactcaca ttcacaagta ccttaccagg ggtaaggatg     600
   ggaacatatc ttttgggagg gccgcagctt aactaacaaa gcgtgctgtg tgtgctcttc     660
   ttctgtatct acaggtctga agcttctttg gagggactcc ttccacatgg ggcaggttat     720
   tccggaagca gctccagaac ttcaaataa                                       749
<210> 33
   <211> 499
   <212> DNA
   <213> Homo sapiens
<400> 33
   acaaaaaggc tgttcctttg actagagaat gcaagtcacc tccacggggc cccttctctc      60
   ctttctctgc tcctggcctt gcagccgtgt gtcttcagcc tgtttctgtt gaggtctcct     120
   tgtccacagt caggacaatg tatctttctc tttcccacat agtccataac tagtttacaa     180
   aatacagttg ccagaaaaaa tgccaggtac ccagttaaat ttgaattcca gataaacaat     240
   gcatagtatt ttagtataag aatgtctcat aaactattga aaaaaaatta atcaattata     300
   tttcatctta ttcataaata ataccatctc aaggggagag gtagcaagac ctgaagaggc     360
   ctgaggcctc tttaggaaga tttggttttc cattgtcttc agaagactgt gacattggga     420
   agtgtaccct tctcttctat tttttggaag agtttaaaaa ggtttagtat taaaatttta     480
   aatgtttgtt acaatttag                                                  499
<210> 34
   <211> 499
   <212> DNA
   <213> Homo sapiens
<400> 34
   aagcaagaga gacaggcgga gggagacaga gaggaactta caggttgaaa cttttcttgg 60
   ggtccagggc gttaccctag caggttctaa ttggtggatt tagagcaagc aggcatgagt 120
   tccgtggagg agtcacacag tgactgagaa gtcgtcactg cggcatatct gcagtttgtg 180
   cagggcgtgg gggccagtgg agcaagtcaa acgggttgta tctagctgtg ccgtaagaag 240
   gagatcacca agaggtggca gtgtaagaga ggtatctgga tcaaccacat ggagaaagag 300
   gaggtggaga actgtgtcca agccctgcct tcagtatgag aaagttaaac ctagattcaa 360
   aatggatact gaggcaaaat aaaatgggat gtactgcagc ctctggcttc agttgtcgtc 420
   tacagagatg ctgggcagga gatcaaggga cgcaggagag agaagtcagg tgtttgtctc 480
   cagccccact ctcctggcc 499
<210> 35
   <211> 499
   <212> DNA
   <213> Homo sapiens
<400> 35
   tgtgtttggg aaaatcgtga tcagcccggg ctgggtgaac ccacctgcag gccatgtgtg 60
   cagtgatcat gaagcatggg cggtagtcgt gaagagaggc tggaggcagc tcaggccgaa 120
   tggactttct cctccagccg ggagccgcct gggttcttgc tttcacttcg gatcagagac 180
   gctgctgcgc tgcttgacac tagacttgct ttattcctgt tgagtggaat acagcaaaca 240
   ccccaatagg tggagcaggc tcaaagcaag aggcacatgg ccccccagaa attctcatga 300
   tcctgtggag gggtgagctt ggtcagggca accaggcctg gatgcaccag ggttgcatct 360
   gagaggaagc accctggtct cctctgcctc gaaaagcata gtgagggggg agcccaacca 420
   agttggagga tgctgagctg tgcagtcggg cttccagccg tgctggcacg ttctcctttc 480
   agctgaaatc tgcattggt 499
<210> 36
   <211> 749
   <212> DNA
   <213> Homo sapiens
<400> 36
   gaagaggctg gaggggatgg aatgttctgg aagaaaaatt aaaggaagga cgttggactg 60
   gaagcaatgc aaaaataatg ataatagtgg atctggaggg gaaaaaacac aattttttat 120
   aaaaatttaa gtgatgcaat gttgaagtat gttttattta aaagtaaagc tagttagaac 180
   accacatgag ctattccgga tcagggctgt ccagccctgg tatatcatgg agagtggctc 240
   gggccacttg cacacatgcc ttcagcccct ggtaccgtct tctctccccg ggtcgcgaca 300
   ctgactcgtc aacgttaatg ggggtccgcg actgctgcgg ggacgagggc gcagagcagc 360
   ccccgccacg ggccggtcca cgcaggggcc gagaaagtgg cggagaggcg gtggccgagg 420
   cccaggggcg agcgcgggct gagctggtcc ctgctgcgtt cacgagcgac acccacccct 480
   tcgctgcgga cgccccgcgg gcgccaggct gggggccctg cgaccgaccc ctcccgcccc 540
   cgaggtaccg ccgggcccgc ctggcaggca gcgcgtcccg cgagctggag ggccgagttt 600
   cgcggggccg tggggcgtgt gggtgaaggc gacacctcgg atgcgggacg catgaatggt 660
   ggcagagcag gggtcgggat ccgttcatgg gttgggagag agatgctttt gtgagcacgg 720
   gaaagtagcg ctgccggaga acagctctg 749
<210> 37
   <211> 749
   <212> DNA
   <213> Homo sapiens
<400> 37
   ggagctgggt agggacgggg agggcaacgc ctgatgggga ctggtgagac ccgggacgca 60
   ctggcgcgat ctaggtagaa aactcgctgc tccctggctc cggggagagg cagcgcggca 120
   cagagttcgc tggcatcagc cgcctcctga agctcatctc ctcttgtttc tttcttcctt 180
   ctctttatgc tggctgctct cccggccact tgctacacgc ctccaatctt cattctctcc 240
   cagtcccgca aaggcttttc cccctccgct gcctccagat ctcgtccttc gccaatagca 300
   gctggacgcg caccgacggc ttggcgtggc tgggggagct gcagacgcac agctggagca 360
   acgactcgga caccgtccgc tctctgaagc cttggtccca gggcacgttc agcgaccagc 420
   agtgggagac gctgcagcat atatttcggg tttatcgaag cagcttcacc agggacgtga 480
   aggaattcgc caaaatgcta cgcttatcct gtgagctgag ggataggatc ctgggccggt 540
   acccaagggg agagaatggc cacagaaact caactgggag actgtggcac cacctgatga 600
   gattctctgc tctgtccacc ctcttctgat ttcccttcta cctggagatg tcccaggctt 660
   tgactcctca aagtgtccct cgttcctgcc tactccaggt cacttacttt cctttccctg 720
   aagtctgggt ccccattata acctgcaca 749
<210> 38
   <211> 749
   <212> DNA
   <213> Homo sapiens
<400> 38
   aagaagatga tcagattgat cagtgtactc tatgcccttc ttaatagtaa ctgagtgtga      60
   ttttttacat tgcatactgc cagaaatcac cacatgtagc atggcagatg gctgccaata     120
   gtcttgttat cctttcataa attatgtggc atttatgcca ttagggtgat ttttcagttt     180
   agaaaagaca actaagggtc agtcttttct atgataatgg actcacaagg gacctcaaaa     240
   ctttaccatg aacatatttt atatcttaag ttatcttcca gagactttga atgtttgaag     300
   ctggttgagg tcgggaagtc aggacagaag agggagtaga gcacacctgc tctaagtata     360
   ggcatttcaa cgttcagagg aaattagtgt ggcgtggagg ggcaccaggg gtggtagaga     420
   gttcatgctg tgctctctcg aggttggatt ctacagaagc tcagcgttgg tgtgattgtt     480
   ggttagtctg gtgtggtttg gtttggttct ttagtaggtg gggcccctaa gaacctgagt     540
   aatgtcccca tgcactagtt ctgtaaacgc ggaagcaggt ggtggcagtt aagtgactca     600
   cactcattta ggctctaagc cggccctctc attcaatatc cagcaattcg atttctactg     660
   ttgggtttac gttgctttgc tagtctgggg cctgcttcga agtgtcaaaa tagcagtgcc     720
   attgttcgtg gtgaatttcc agcaaaaga                                       749
<210> 39
   <211> 1499
   <212> DNA
   <213> Homo sapiens
<400> 39
   ctagagctgc aggagcggcg ctgcacaggt ctgacaagcc cagctcattg gcgggtatct 60
   gagccatcag tctgaaagac atttggggaa aattcataga acatagaaat tcatattata 120
   catattcata ttatacattc atattataca ttgtgtatat tatataatat atatatagtc 180
   cataaattag taaatgtgtg cggtgttttt cttgaaaccg ttagcatcct agttggtatt 240
   ggtggtactg gttgatatta acacgaatga caagtgggtg attttcaaga agcgcccggt 300
   ccctctagag aatgcgtccg aatatcagcg gagccgactg cgtatgcctc cggatgccca 360
   tctataaact ctcttgcttg tagctattcc tcgctcccca accatattga ccattcaccc 420
   ggataaggca atttcctcga aagggcgatc tgaggacgct gaccccctaa atgactgagg 480
   acgctggatc tttaggggga acatcgtgtc ttgggggtgc caaaagtccc cagcccttac 540
   ccacaccttt gtcacgacgg gcaattgggt atgtgtaggg gaaaaacagc aacgttaaaa 600
   cgcaactgtg taaatgagga tagagagtgc gaaaggaggg agaggcgagg agctgctcta 660
   tttctaggga ggttttgggg agactgatca gctccaagga cagaccgctg ggaagggaaa 720
   aacggcccac atcgaactgg atgccggatg gaaacctctc tgcgctatta gactgcgtcc 780
   agtacagcag atggcacgag cacgtgcggc gctcagctta ggctctcgga ggcagctgag 840
   ttggaaatcc cgacggaaag cacccacaag ctcccactct gcgctggccc acccgcgtgc 900
   acgcccaccc cccacgcgcg tccctggctc agaagcgcac agatgtttac tgcttagagc 960
   cggtaccgct ggggagatcg agcgacttgc gcggcgcaca gtgcggcgct ggcagggctc 1020
   tgggctcccg gtcgggggtt cgagcggcca agggatgggg gtgggggcgg ggagagtggg 1080
   gggagggcga aagaccgccg agaggagggg ggagtgggtg gactaatgat gaaaaagtct 1140
   cctccatccc agttccttaa ttaaatgcat ggaaagaacc gaggcgagca catctggttt 1200
   caatctacag ccctttgatg gcatcaaatg ttcttttccc agatcagggc tggaagttct 1260
   gggctaacta tggccgtttg gagcccagaa accatttaca cacactcgta cccttctttc 1320
   tctccagtcg agcctcttga ctataggacg aaaaaaaaaa aaagtctagc aatcaaggga 1380
   gtgcgggagt acggatgcgt gtgtgtgtgt gtgagtgcgc gtttaaagaa cataaaacgc 1440
   cacaaataag cacttaatat tttactgagt cgtcatacag taactcattt ctaatgaga 1499
<210> 40
   <211> 1499
   <212> DNA
   <213> Homo sapiens
<400> 40
   taactgggct tttcctaaac tgtttaaaag taaagtacca tttacacaaa gaacccggtc 60
   tcgagatttg taagtgacgc ctgtccagac aacgtattat tccatgcagt ttccacatca      120
   cgtgggcttt tatttggttc agcagtggcc acagtaagcc ctgccctggg gcattagctg      180
   gtgcccttgt acgcgcacaa accaagcatt ttattgcata atccaaaatg atgtagcctg      240
   tggcctgtcg ggaggcgctc ccttcttgtg gaggaaggaa ggtcaagaag gagctcccgg      300
   cagaccaggg ttcgctgcgc ccagagacct gcccagagac ctgctgcacg ccggggcgca      360
   aggccgagtc atcccaggcg tccgtgggcc gtgattccca ctcacgccgg gggcccaggc      420
   aggcagagaa gagttaatga gcgcgcaagt gcaggcggtc actcctgggc ctgaaactcc      480
   cgcgctgtgc attcagggcc ctcgtggctc tcagaggcgc gtcccagggg cgcacactgc      540
   accttgggct gggcagctcc gccgggttgt ggcgagcgga tgagggaagg acgcagaaac      600
   cagggcggag gagccgcgag gggcaggacg aggctgcatg ggccagcgag ggggtcgaca      660
   ccgagccaga gtgagcgcgg ggcctggggc gcagagcccg cccagggagc cgggagacgc      720
   cgcgcaagct ccccggacaa acgcaatgac cgaggacgcg cgggcgaggc cgtccaggga      780
   gccctggtcc ctcagctgca ccggactgag ccgcgaccgc tcagcacgcg ctgcttataa      840
   atcaggggtg cgcttcccaa gccccgggtg aggtccccta cgtcggcaca gccttaggag      900
   ctgcaaagca gcgcgcgcct ccggggctcc tgcgcgcccc ttgaaccccg cctcccgcat      960
   cctcctgcaa cagcctggag ctccctgtgc aggacgcagc gggggcgggg gggcggtctt     1020
   aggaggctgc ggggcgcact cccacctcct gcctccccga gacccccagc gccttctcca     1080
   gggtttagag cggaggtgaa ggggcctcgt cctgcaccgc cactgggcgc ctgggctgtt     1140
   catcatcggt taccgccgat tcataggaac tcctcaacac attggctcgg aaatgtacag     1200
   tcataggcaa tttataaaac tgacaaaaat tattccgcta atgccaggaa taacggagga     1260
   tattcagaaa gaaaaacagg aatattttct tgtgtaaata atagataaag aataaaaaag     1320
   taaatgagcg taatccagca gcaatcccct tagggagtaa taaaacccga aaagtccaat     1380
   ttgcgcagca agatccatta ggcaggaagt gaggaagcca gacgctgtcc tgcggccctg     1440
   aagcggggaa ctcactgtgg gagtttgatg cctcaaatca ggagctgcgg aaggaagaa      1499
<210> 41
   <211> 499
   <212> DNA
   <213> Homo sapiens
<400> 41
   agttaaaagg acaaaagtct ttcctgtgtt tcatacttgg gcggtgagtc actaggaaag       60
   gatttggttt ttagaaaaaa aacttctgat ccctgggcta aaacagagag ccccaaagag      120
   ctatgttgat cccagacaag cacgtgcgtg gattcttcaa agttcaggtc aactcaggcc      180
   cctcctcctt gcagtcagcc ctgtactcaa ggttgctgga gacatggcgc ctctatttcc      240
   tgcccaaaga agccccctta actgggggcc acgggttgag tggtgaagga ggcaactcac 300
   acctgaatta tagtgggctt gtaaacctga acagggcaag tcacaacttt gggaggctga 360
   ggcaggagga tcacttgagg ccaggagttc aagaacagcc ctgacaacat agtgagaccc 420
   tgtctacaaa tgaaaaaact agccaggtgt ggtggtgcat gcctgtgcta cttggaagac 480
   tgaggcagga ggatcactt 499
<210> 42
   <211> 499
   <212> DNA
   <213> Homo sapiens
<400> 42
   ctgtaataaa tgctttacaa aattcgcacc caaacctcaa agtggcacac aggaggcact 60
   cttcttatcc ctactttgca gatgaggaaa ttgaggcaaa ttgccggttt cagttcattg 120
   ttcagggtca ttggtggcaa agggcatctg ggccagactt tccagtctcc tcagagatgt 180
   aggccacagt gccagtgccc agggtggggg tggtgggagg ggcccagcaa acaagtgcat 240
   gtgtgccacg ggacccttca gagggacacc ccttcccact cctccactcg cttctcgcca 300
   cagtcctcag aggcccagac cctgtttctc cagcgtcagc actttccacg tggacagtga 360
   gcactgaaca cagccctggc acccacacag gagaagcttg taaccatgcc gcccccaggc 420
   ccgggagcta gggaaccaag gcagcattca gggcgtgggt gtaagtgaga aactagggag 480
   gaccagccta gcacccccg 499
<210> 43
   <211> 499
   <212> DNA
   <213> Homo sapiens
<400> 43
   acaaagtagg agtgtctgga ccactaggaa agaatctgaa ggatttatga ggtcagactg 60
   cagttgaggc ctgaaaagga ccatttgatt cttataaacg tgagccactt ccacgagccc 120
   tcaagaagca gagaggaacc cagagggttg agaataagca tagtgttcat tgagctcctt 180
   tcatctgggt tgagttgact gagtggcagg aaattcatgg atgatatggc taaactgtaa 240
   acagctgtgt caatctaaaa tacgattgaa aattttttga gactccttcc attgagaggt 300
   gtggtccatg tttcctcacc tcgaatctga gaagatccat gactacctag aacaatcaag 360
   catggtgcta tgtgaagtgg tgctatgtga tttctgaggc taggtcataa aaggtcatgc 420
   ccagttttct tgggacatga actgctatgt aaactatctg actactttga gatacccagg 480
   atggagaggc catgtgaag 499
<210> 44
   <211> 499
   <212> DNA
   <213> Homo sapiens
<400> 44
   cctttgctca aataaagcct atgctgatga tctcttctag aattgcaact cattctgcct        60
   ctaccactcc aagtattcca aatccccttc tcccgggttt acctttcacc ttgtaacata       120
   cagtataatt tctgtgttat gttcttggct attgtctgtt tccacaaagg tagggatctt       180
   tgttcactga tgcctcccac ccacttagga cgtgcctagt gtgtgctgga gtcctggaag       240
   tagctgtcag gtgaatgaaa agtgtcatag gactggaggg tggagccttg tggaggagcg       300
   caagtgatga ggatgcagaa ggaggaacag ataacttggt ttctttgtgt caacctgtga       360
   catgcaagct tgcactccaa gggccaacta caggaggtct tagaggttta ggcaggcatg       420
   tggcataatc ggatctccac ttagttctcc tgccccagag cagagagcag actggggtag       480
   gataggatca gaggtagga                                                    499
<210> 45
   <211> 999
   <212> DNA
   <213> Homo sapiens
<400> 45
   caacatccgg aacctcaggc cccacccaga cctattggat gaagtgctgc agcttaataa        60
   gatcccaggt gacttttatg cacattgaag tctgggaaac agagtcttac aacgtgagtc       120
   aatggccttc caaaagggtg ctggcactgt aagaaataaa acctgagaga tcagatactc       180
   ctgggagagt tagggaggaa aagctttgct aaaagctgct ggaagtagtg ggtgtctatt       240
   tgtggatcat attttgtact gaattgttat gtttccccta cttactgaaa acatgagctg       300
   aattccagaa agtaacagga agaaggatga ggatggaaga gttaaaaaat aacacagaag       360
   tcctgagttc ttgcaggagc atccccttgc aagatactca tcatgacctt gggccccatt       420
   gcgccacagt tttctccact ttgacaagcc cagatcactt cctagggcct gcaggattcc       480
   tacattagtg cttctcaagg gctccgaaag cctgggatga acgtcatggc gcatgcgtga       540
   agcttatcag ggtcgcgcta tgagttccag gctggctcct aattccgcag cctcctcgca       600
   gctggggagc agtgtgccca cttttatctc agctcctggc ttctacagag gacgagatgg       660
   ggagggcgta gggcgaggaa ggaggagata aagcggtttg gtgcatggat gagtcagagc       720
   ccgggcactc ccacccatgg ctgaaaagag catgagtttc ccacgtccct gttctgctgt       780
   gagaggggac cgcgtatcca cgtcccccag ctgcactgtg ggagggttaa ttgcagaaag       840
   acattattaa acagcagatt ggctgtcaca cgtgtcaaca cgtagcgatg gaggtgagta       900
   aatgactatg actcaaagta atttttagaa acaagcacaa aataaaatgt ctgtgaatgg       960
   gactattaca gaatctcctt gaggaagtgg ttgcaaatg                              999
<210> 46
   <211> 499
   <212> DNA
   <213> Homo sapiens
<400> 46
   actttctaag gctgggatct gagaaaccct gtgaaggggg atgaatggca ttgagagcac        60
   tgtttcctag taggtaacaa ctggtatctc tacttcctag acaccaatcc ctggcccagg       120
   atctatggct ttgggttcat gagtctacat ccaagggaat ttaagtacct gcaggagagc       180
   acgaaattgt aggtgccagc caggcgcaga ggctcacacc tgtaatccca gcactttggg       240
   aggccgaggt aggtggatca cttgaggtca aggagttcga taccaccctg gccaacatgg       300
   tgaaaccctg tctctattaa aagtaacaac acaaaagtta gctgggcgta gtagcagatg       360
   cctacaatcc cagctactcg ggaggctgag gcaggagaat tgcttgaacc cgggaggcag       420
   aggttgcagt gagccgagac tgcaccactg cactctagcc tgggtgacaa gagtgaaact       480
   tttgtctcca aaagaggaa                                                    499
<210> 47
   <211> 499
   <212> DNA
   <213> Homo sapiens
<400> 47
   taaacaccag aaacttttcc atcaacttct aaacaccaga aacttttcca tcaatttcta      60
   aacaccagaa acttttccat caatttaatg cagtttgctt tgggtcctcg ggtctgagct     120
   gtgtgggaaa cactggttga tagtctggcc tcagtttttc caactcttac cgttcaagag     180
   atctgagccc tgaagacctt tgcagcttcc tgagacactc aggaggacct ctcctcggtc     240
   ctgtttagtt tcctggggcc acatgggaac aaggagaaag acttgggtag aaacccagac     300
   tcgttaccat ctaaagatgc ttaatttcca agatatgaat cgattttcca caaacccatc     360
   taccccgggt acccaaaact agtgcatttc gtctctggga taggactgaa cactgatacc     420
   ttggcgaggg gtagggagaa ggatttgctg ccaggaaaat gaccaaaact ttcatttggt     480
   gttaagtgta tccagagag                                                  499
<210> 48
   <211> 999
   <212> DNA
   <213> Homo sapiens
<400> 48
   caccgagaat acttcatcag caacatccag attgtgggaa atttcacagg tcaaaggatt      60
   caggtttttc aacagataaa ttgtcagaat aagaaagaga ggggaaactt gtagcttcgg     120
   agacttaaaa tcataactaa ttttcaaaaa atagagatgg ggtcttgtta tggtgctcag     180
   gctggtctcc cacctctgcc tcagcctccc aaagtgttga gattacaggc atgagccacc     240
   acgcccagcc acgacttttt taactggaca accatagagt cccaggtgtt cactataaag     300
   aaacacgacc aagtgattgt cttcgtggtt tcttggcggc ggtggtggtt gggacgtgat 360
   aggggtgggg cccgtggatg gtcttctcag tggcttgcaa agttctattc cttgacctgg 420
   atggtagtta caagggtgtt tgccttcatt acgctataca ttcatttttg tatgattttc 480
   tgtatttatg ttttattttc caaaaaaaaa aagctttaaa agagtataaa gaaagtagat 540
   ggcagaaatt tgtggaatct tcccccagca acataaaaac gcggtgggtt tttgtaactt 600
   ggtttttaca ctttacaatt aatattgtaa tgagaaatac tcgtgtggac cactagggcg 660
   cacaatttgt tgcccgcagc atctgcggca ctggcactga tgaaaggggg atgctaaatg 720
   cttcagtaac gccacctgag tctcgggatg aagcaacatt attcatcgcc ctttgatcat 780
   gagctacata tgtgagtgcc aatgctggcg aatcgtattt ggaaagtcgg gtccaacatg 840
   tgatgtgtac atacagggta tacactgaaa ataccgtagt tttatcctct tttaagataa 900
   gcttcaattt atttgagtta ttagaacaaa gcctcataaa ccacggtaaa aagaacctta 960
   aaactttttt ttttattttt gaggaagtct tgctctgtt 999
<210> 49
   <211> 499
   <212> DNA
   <213> Homo sapiens
<400> 49
   ggccttggga cactgaaacc ttcatccgta gaaaatcagt taagtcttca caggctagaa 60
   gagagggtgt gtgtgattag taggcaaagc aaagaaagat cagtacaagt tgtctggcag 120
   ctggataaaa ccttacacct gcgcaaaaat aagcctccct cataagaaag cccaaagatg 180
   tccggggtcg gggaggagga aagtgtctct catctgtccc atcaacgaaa attagtgaaa 240
   tctgcctcag atgaagtgca aaggccagtc tgcagggata gtttcaacct ctccccacgc 300
   gatgggctac acatcacctg cccaagctct ctcccgacct gctagagcct agagggcgga 360
   ggccggagag gctgcagccg ggagtagcac cgcacatccg ggaacgccag cagcgggctg 420
   agggctgcat aactgatgga aggccgggcg cggtaagagc gtctcgggga gtagggcaag 480
   gcggccgggc ccctcccat 499
<210> 50
   <211> 499
   <212> DNA
   <213> Homo sapiens
<400> 50
   ggccaagctt gtgtttgttt aaaaaacaaa aaagtttgac tgagacttaa ctgccctagg 60
   tacctcttcc tatgttcatg ttttaatggg cggaaaaaaa gctcatgaaa atgtaaagaa 120
   ctggtcacag ggacctggct ggccccaccc agaaggtggg ggttgggtga gttgccggga 180
   aggaacttgg aaggggctgt gaaggacaga gagggctaga attgggctgt gtggagcctg 240
   tgttctctaa gacttcaggc cccacagacc tgttgagtgc ctcattgatg tgatcagtgg 300
   cccagaagat agtatcccaa atgtttaggg gtccacaggg tccacctctc ccatctgatg 360
   ccagcctgca tggaaaggag ccctctaggg agaggggcag gtgaaacacc tgcgtttcta 420
   aacaggcttt tgaaactcca gctggtctcc tttccacctc ccaccaccac tcccaagacc 480
   ctccccagat gactagagt 499
<210> 51
   <211> 499
   <212> DNA
   <213> Homo sapiens
<400> 51
   cattagttta gtctaaaata acttagctca ttcattttta tgaccaaaac atctgggaaa 60
   aaccaggcat ttctgttgca ttttaacagg gtaagtgaat ttaattcgta tttcctgcag 120
   ctgtgatttc ccctcctact gggttcttcg gcattcattc cacaccaaca caacacgact 180
   tcatcacacg gtttttaaga gtaagctttt tttcccattt tcaagcagct cagcaggaac 240
   ctgtaattct acaaggtgtg taagcacaaa tgagcaagtg aggtcttagt caaggtgacc 300
   cagacagttc aaggccagag gctgagattt gacaaagaat cttcaataaa aagatccaga 360
   acttgctttt ctacttctct catctccagg ttgtccaaat caaatgggtt tactccttta 420
   taaatcatct tggaggagct ctctgtggtg ctgacattac agacattgct gtttcttttt 480
   acttgaaacg gtttctagg 499
<210> 52
   <211> 1249
   <212> DNA
   <213> Homo sapiens
<400> 52
   attatacaaa ggtcttctgc tccacctgca tctctcagga actcaggcaa aggtggcctt      60
   ccatccagcc gcaccgccat ccggcagggg agggcacagg caccctccca cccgcatccg     120
   cccccgcccc ctcgcccagc agcgtcagtc tctgacccca ctggatccgt acaggagacg     180
   actcacaatc ggtcggaagc tgcttttgcc cccccacccc accgcaaacg ggggtttgct      240
   tggatcattt atctatcttg tgtgcattaa gaaaccagca ttagctgcta gtgggaggcg     300
   ctactctgcc cgaatcccag cccgccgcgg cgattctgca cacacacacg caccagcctg     360
   gcagccagag cccgtctgga gacgccctca gcccggggtc tgcgttcccc gggacccccg     420
   acgcagtctc ccgcttccgt cccccacgct caaccgggca gggcgccggg gcgtgatttc     480
   cgatcctctg cctgcttgtt gggtccctcg gaggcgggtc agaccgcacc cgccgcgggc     540
   gcccggtgcg cccccagccc ctggctcgcg gcggcgacag cggcgctgtt cgctggagtt     600
   tgactctccg gcggcggcgg cagcggcgcg cagcagcgaa cggctggagc aaggcgagcc     660
   gggccgctag ccctccgcgc tgcgctggga ttggtctctc cagaagagtg ctggccgagg     720
   gttggctgcg ggccggctga agaacaggtg cacctcaccg cccgggctcg cggagcagcc     780
   gccgaagatc gcggcggcca ggcaggccct ctgtgtcgga atgcgggtgg cgggcacccg     840
   gcaccccgcg accggccgcc ggggccactg aaggcggcgc gaggcccagg cgcggcgcga     900
   gcgggcgccc cagggagcgg gctgggcgcg gtgccccgag gatgtcggcg ctcctggagc     960
   gcacgcaggc ggcgggcagc agcagcagca gcgggcgcgg ggacccggcg cgcaggaggc    1020
   ggcttggagg gctgcagacg cgccccgccg ctctctgacc gaccggaggc gccgggggcc    1080
   cgtctcgccc ctcttccgag ctccttaccg ccccctcccc ggccccgtcc cctcccccgc    1140
   tcctctcctc cccgcccgcc gcccgcctct cggggggagg ggcgtggggg cagggagcgg    1200
   atttgcatgc ggccgccgcg gccgctgcct gcgcccgagc ccgccgccg                1249
<210> 53
   <211> 1749
   <212> DNA
   <213> Homo sapiens
<400> 53
   aaattacgtg gacttggcat ggctttttaa tattaaagac aaacgacctt tggaaaatat 60
   acactgttaa agtcaaacca tttggagaga cacccagcaa tttacctcct caaactcctc 120
   ggaaccccaa gaatgaggaa aggaaatgga aaatgcgctt aacccggggg tggtggggga 180
   atcgataacc agaacaggtt tgaaaaaaaa agcccccctc ccgccccctc cgtagagacc 240
   gctagctgag gctgcaacac ctgccccggc aaagcgtctc cgcagccttc ccggcttgcc 300
   cgactcggct tcctccgcct ctgccccggc tgcggcacca cttcttggag ccacgtctcg 360
   gcgagcgggg gccgcggagc gagggggccg ctgtgccgct actcacccga gccgctcggg 420
   ctggccgcga gccgggatcc gcgagggctg gcgggctctg gcccccgagg acgcagacat 480
   gtggcttgaa cctccgctcc cctagccgtt gcctctgtgc atctttctgg gcgcccccag 540
   cgaatgcgag cggcgaggcg agggcgagcg cgccgaggaa gggcgggaga ggcgcggagc 600
   ttggccgcgc cgcgctgcgc cgagcgccgg gctctccccg cgagctcccc gggcccgcgc 660
   gcgcgccccc cactgccccc gccccccgcg cggcgcgtgc cccccacccc ccgccgcgcg 720
   ccctcgcacc cgcccggctc cacgcggcgc gcgcctgccc tggcggcagc ggcggcggcg 780
   gcggcgcgtc ctcccccgaa cgccgtctcc agggctgctg gctgcgctct ccattgttcc 840
   gcggctgctg cccggggtgg gcggcgaggc gggggggagg tgtcggcttg gccgccgggg 900
   agggcttacc gctcgggcgg accctcactg cgagagcgat gcgggcccag gcgcggcgcg 960
   cgggggctgc agggcgccta gcactggggg ttgccggcgc gcgggggcct cctcctggct 1020
   cccaggcact cgctgctgct gggcgcccct cgcatcctcg gttactatgg atatctcgct 1080
   cctccgccgc cccctccgcg cactccggga ggccgccggg gcggtagcag cggcgcggct 1140
   ccgcgggtgc ccaggtgacc ggctcggcag cggcagagca gtggcagcag ccgccactgc 1200
   cgctgttact gcggtcgccg ccgctggaga gaggaggacg aggagggcaa ggggcagaag 1260
   caggtcctgc tctgtctgcc ccagaggcca cctcgggttt cttctcacta accaagcgac 1320
   ttcgtgttta cctcgcagga gacgcctcgg cagtcctcaa cttgtgtgcg ccggtggccc 1380
   tctcctgtgg gacttgcgtt ccagctgttc tcagagcggg tatgatcggc ctccagtaga 1440
   cttggagggt cacgggtgag attttgataa ggttcaaata ctcctcactc ctgcctccgg 1500
   tttccaccaa agttaccatt gtactactac caacagttgt ggaaatttac tttggcaaag 1560
   gttttgtgtt tttgtttgtt tgtttttccc cccaaaaatt atgccaatta aatccgacct 1620
   taaatgacaa ggcttttctc tcatgtttaa aatcccattt ttttcccctt gccataaata 1680
   aataaaaata acttgtggct tacaggctgc ttaataccac aacattttaa tgagcatgtc 1740
   aggtaaccc 1749
<210> 54
   <211> 499
   <212> DNA
   <213> Homo sapiens
<400> 54
   cggccgggtg gggagggcgg cggtggcatc gctgcgcggg gcgcattgtg ggccgcgctc      60
   gcctccgcgg gggaccatct gctcgctgtc aatgcatcac ctgctcgtct gggccgtcgc     120
   cggggcaacg gggggcgggg gattaaggag cgtgtgcgtc tcggtccggg ccgaggcggc     180
   gaggtggggg ttggggcggg ggaggagagc tccttggccc cccacccccc tgccccgaga     240
   cgggtcgacc cgctcggggg ccggcgacca ccgcgacggg ttccgccgct tgcctccgct     300
   ccttggcctt tgctgccgtg ctgcctcttc tcacgggcgc ggctggagtc ccggggagca     360
   gcagagagca aacggtccgg ctctacctca ccctgccagg gggcgagtcc cgcgctccct     420
   gcgtctacct ggagctgcag ggtccctatc ccggggcgcc gcccgcagcc tcctccgcgg     480
   gagctggagc actctgctg                                                  499
<210> 55
   <211> 1499
   <212> DNA
   <213> Homo sapiens
<400> 55
   tcccggagga gtactatgcc ttgacacctt cgtttcaccg ccccaaagct ggcctggggc      60
   tccgtaggga gtggcctgca tggggagggc ccgcgtgctg tgtttctggg aggggtaaga     120
   gagtgggggc gcagggggcg ggccaggtcc ctgggcgcgg cgcgggctcg ggggacccgc     180
   gcggctgacg tcaggccact ccttaaatag agccggcagc gcgctccgct cggcatttcc     240
   cgaagagcca gatcgcggcc ggcgccagcg ccaccgtccg gtccacccgc cagcccgcac      300
   agccgcgccg ccgccgagcg tttcgtgagc ggcgctccga ggatcaggaa tggggcttcg      360
   ggcgctgggc gcgctccgaa cccggcgcac gtaagagcct gggagcgccc gagccgcccg      420
   gctgcccgga gccccatcgc ctaggaccgg gagatgctgg aaatgcaacc gcctgttccc      480
   cgaggagccg ctgcccccgg gaccccctgg cactgtgcgc accctggtca gcagcccccg      540
   gagaagacgg cgcccccaac gcccgacccg cgtggccgtg gcagcgccac gcgagccctc      600
   taggcgaccg cagggccaca gcagctcagc cgccggtgcc ccctcggaaa ccatgacccc      660
   cggcgcgggc ccatggagcc atggcctata gggtcctggg ccgcgcgggg ccacctcagc      720
   cgcggagggc gcgcaggctg ctcttcgcct tcacgctctc gctctcctgc acttacctgt      780
   gttacagctt cctgtgctgc tgcgacgacc tgggtcggag ccgcctcctc ggcgcgcctc      840
   gctgcctccg cggccccagc gcgggcggcc agaaacttct ccagaagtcc cgcccctgtg      900
   atccctccgg gccgacgccc agcgagccca gcgctcccag cgcgcccgcc gccgccgtgc      960
   ccgcccctcg cctctccggt tccaaccact ccggctcacc caagctgggt accaagcggt     1020
   tgccccaagc cctcattgtg ggcgtgaaga aggggggcac ccgggccgtg ctggagttta     1080
   tccgagtaca cccggacgtg cgggccttgg gcacggaacc ccacttcttt gacaggaact     1140
   acggccgcgg gctggattgg tacaggtaag gaccaggagc tccgctccgt gcgccgggtc     1200
   tctgatcgct tccattggga gagccatccg tctcttgtgt tttctctttc ttttaaccca     1260
   actcattgta tgggttcagg ctgacacaca gggccatggg gggctatagc agaatttacc     1320
   cagaacttcc cagtgataat ctagacgggc agtttctgga actgcaaagg gcgttccctc     1380
   gtcactggag tcgttggaaa aggattatct ccagtcaaac ctaagtgcca gctaaagggc     1440
   taactccctc tgtgaccagc ccttagggtg cccaaggaag ggacaggcga ggacctgtg      1499
<210> 56
   <211> 999
   <212> DNA
   <213> Homo sapiens
<400> 56
   atgagaactg cattgcccag aaacctgtgc gccgcccggc ggcggcactc ttaggggcgt      60
   ctccctgcgg acggaagctc tctgggcggg acttccggta tcttcctcgc ggtggacatc     120
   ttgtcggctc ttaggtggaa ccatcggagc agaagctcgg ggttgctggg cggttccgag     180
   gtgacggaag cgggagggtg cgggagaagt cgctgttcgc tctgcggagt ggctcgccag     240
   cgaagacccc gcctgcgccc ccggggacgg acgaccgcgg tgccagggtc ccgcgacctg     300
   ggaccccctc gcggctccgg gtggtctacg aactgtgatg gcggcggccg cggtgatggg     360
   cccggcgcag gtgggtgctg cctttcccag actttcgccc gccccaaatc ctgaagttcc     420
   aaatgaggag cgcctgtctg agtccctgca gcgcaggccc cagtgtccaa ggcagcgggg     480
   cgctggtggg tgggggcgag tgtgactggc agaggggcag cctgagcata ggtttggagc     540
   tggactgagc ccgtagcagt cgggagcgtg tgtgaaccgt agtcaggcct gcaatgtcga     600
   ggggagaagt tgctccttca ttgcgaggac gataggagcc atggcgggtt ttgaatggtg     660
   gagggaaggg atccgaaaaa ggatttttaa agtattccaa tgtttgctga ggaggaaacc     720
   gactacagtg aggtagaaac gatgaggatg gaggcaagga gacgtttgag gaggtccctg     780
   caacaaactc cagaagtgtt gcggtggtgg ctgggccaga gcagtggcag gaggggttgg     840
   gtggggaagt catgagattc tgggtagatt tttaaagatg gaaccaatgg ggtttcctgc     900
   cgcatcagat gtggtcgtga gtgaatgtag ggaggaaagg gctatccagg gtttttttgg     960
   cctgttttcc ttcctgaacg tgtgaaagaa tggaaattg                            999
<210> 57
   <211> 999
   <212> DNA
   <213> Homo sapiens
<400> 57
   gaggtatccg gcggcgccca tttgggggct tctaactctt tctccacgca gcccctcttc 60
   tgtcccctcc cctctcgctc ccttttaaaa tcagtggcac cgaggcgcct gcagccgcac 120
   tcgccagcga ctcatctctc cagcgggttt ttttttgttt gtcgtgtgcg atcctcacac 180
   tcatgaacat acacaggtct acccccatca caatagcgag atatgggaga tcgcggaaca 240
   aaacccagga tttcgaagag ttgtcgtcta taaggtccgc ggagcccagc cagagtttca 300
   gcccgaacct cggctccccg agcccgcccg agactccgaa cttgtcgcat tgcgtttctt 360
   gtatcgggaa atacttattg ttggaacctc tggagggaga ccacgttttt cgtgccgtgc 420
   atctgcacag cggagaggag ctggtgtgca aggtaaaggg ccagtgggtt gctttttgtc 480
   tttggaaggg gcccgaggga gcgggagggc gccaggccct cgagtctggg agagggagat 540
   tcgcgggata attaccgtgg ccttattaaa tgggtttatt tatttatttg ctcaggttcg 600
   gtaagttgcg aagtttttag accgtttcag acaatggggc gggcggcagt gggggcgttt 660
   cggggagagc ccggggagga gagggcggcg ggactgcgcg ggggccacgg acacgcgtgc 720
   accgaaggct ccaggagctc tctgcgcgag gccgggtccc gctgcccggg ggggatttct 780
   tcctgtgtct agccccctcc ccttccaaca aggattaggg aatcccccgg taattttaag 840
   actgatgact tcgttctttt cgcagccatt gttcttagca gcgggcaggt gttaaacctt 900
   tgttccgaag gtgcccttta aaacagacac acaaaggtgc ccccttcggc tgagcccagg 960
   ggcccagcgc agggaaggag tttacaaaga cctttcttc 999
<210> 58
   <211> 1249
   <212> DNA
   <213> Homo sapiens
<400> 58
   cgaaggagag gtgggggagg aagaagagga ggaggaggag tcccttgtgg ccaccccgaa 60
   gggagggagg gctaccgtag agacttggtc gagaggcgcg ggacaagcct ggccgctggg 120
   actgtgcgct gaggtgcacc gaccgtcggg ccgcgagctc cccgcagacc ctcgcggaat 180
   gagctggggg gcggcgcgcg aggcggcgga gcggaaggcg cactgcgacc ccggcgggct 240
   acagcctgcg gcgcttgcag ggcgctggtg gggcgcgccg agcaggggct gccctggggc 300
   tgccccagtc ccaccaggtc ggggctcagc tggcggcggc ggcggcggtg gcggcagcgc 360
   gtcccatccg ggtccgagta accgccgccg ccgccaaaac tcgccaacgt ggcggacccg 420
   gaggctgtgc tggcagatgc cagttacctg atggccatgg agaagagcaa ggcgactccg 480
   gccgcccgcg ccagcaagag gaccgtcctg cccgatccca ggtaccagct gccccggccc 540
   gcgctggtcc ccacgccgcc gtccccaagc ggccgtcagc gacctcctgc gtccgggagg 600
   gtcgggcatt gagtcgtcgc tgtcctgggt gcgggtgaca ccgcggaact ggcgatgcgg 660
   ggccggcctc cccgttccag tctctgaaat ggggcatcgg atggccggtg ggggggactc 720
   cgggagagag cgctccaaag tgcccagcgc ggcgccctgc gcgcagcgag cgccccaggg 780
   aggggctggt tatgacttgg ctggaccagc tccatccctg tcgccccctc cccccggccc 840
   tgtcctgtcc tgtcccatcc ccgtggttct tcctgttgca ttggtgtggt ccctgtgggc 900
   tcgttgcctg tcactccttt gcgctccttc ttggtcgctg cttcttcccc ggctctgtgg 960
   tccccctttc caactccatc ccctcagctc cctctgggcc gcttatctgg ggactgcagg 1020
   cttgttgctt actgtccgag gtagttaaac tgctgttttc agtgcttgtt cttcttgaag 1080
   tccctaagtc tagtcacctt cttaggctct tcttctattt tgtgcccagg caggattttt 1140
   gacccactca ataatctttt tggtgccacg tgtgtcacct gagctgcttt tctcaacttg 1200
   cagatctact ggtggcactt tattaaaaaa ttgaaatggg attcattta 1249
<210> 59
   <211> 749
   <212> DNA
   <213> Homo sapiens
<400> 59
   tacagatgag gttttctaaa ctccagggga agcaggatcc aacttcccct tgtaggtaaa 60
   aagacttagt gcctccgata tatctttttt ttttccaacc aagtgtacaa taatttttaa 120
   agatacctcg gccctttctt tacctccact cctcattcca ttccactcaa agttggtggg 180
   aaatgctggg ctgctagact cagacttgtt gatgggaaca gaacaattaa ttttttttcc 240
   gaatttatat ttcccggcac aagcacaaat gctcagccag gtcccttcag gcaccgggaa 300
   atcatcccgg atacccaagc cgacttttga gcaagcacag cccatggaaa gggcagtccc 360
   gccggccagc cccaagcgag aatctagttg gtgagaagac cagaaaacca gaaaggcgag      420
   gagcggcgga cgctgaccct gccttcctcc agcccgtgca gtcagcgctg gcgtcagggc      480
   aaaaaatata ttcattttca ttttcctctc gctggggcac ggtgagtttc ctaaccgggc      540
   cgcctatgaa aggatgagtt gaggtttctt tgtttggaaa aagagtttag ggctttgatt      600
   cagctgcaaa gaagccaaat gaagttagaa acaaagggta aattgaagga ttccgactct      660
   tggctttttg tgttttcctt actagaaaat aattagacct aatgaatatg cagacgcttc      720
   agctaaagcc tcggccagga ctgctgggt                                        749
<210> 60
   <211> 1999
   <212> DNA
   <213> Homo sapiens
<400> 60
   agtccccact cagtcttcgc agcagctctc atcctccact tggcctcttg gagttcctcg       60
   ccggagtgct gactagtgga tatttctgcc cggctgcggc ggcccgactg cccttttgtc      120
   ttttctgcgt gacctcgggg caggtcctgg tgcagagcgt cgccaaggac gccgagcggg      180
   aggcgggatt gcccagacat ccttcagcga agtgcatgtg tgtttgtaaa ccatcgttgg      240
   ctgtcgggag accgcgagga ccggtccagg ctgcggcgga gtcgagggcg agggagaggc      300
   cgcgtgagtg agcagagtcc agagccgtgc gcccccagaa ctgcgcgtcc gccccgtgca      360
   cccccgcgcg ccatgcccag ttgccccgcg cgctctgcta cgggcccgct gggcttccgc      420
   gccttctagc ttccggagcc cactttgatc ggggccataa tacctattga gatcccctct      480
   tctgtcttgt accttcgcca ctggcatcgg atttgcagaa gcgtgcgtgg gatcagagga      540
   ccgccctccc cacaacaacc ggcccctgca tcttagcagc cgttggaagc cccagctctt      600
   ttaccgccaa gttcatcctt gggagacaga agacgcgtga tctcctctcc gctgctcttg      660
   gggtctcctt gcagccctgg ccaggcggat tcatcctcag gacctaaagt tgcccaagga      720
   gctcctgctc tgccagagga gggtggagag ggcggtggga ggcgtgtgcc tgagtgggct      780
   ctactgcctt gttccatatt atttggtgca cattttccct ggcactctgg gttgctagcc      840
   ccgccgggca ctgggcctca gacactgcgc ggttccctcg gagcagcaag ctaaagaaag      900
   cccccagtgc cggcgaggaa ggaggcggcg gggaaagatg cgcggcgttg gctggcagat      960
   gctgtccctg tcgctggggt tagtgctggc gatcctgaac aaggtggcac cgcaggcgtg     1020
   cccggcgcag tgctcttgct cgggcagcac agtggactgt cacgggctgg cgctgcgcag     1080
   cgtgcccagg aatatccccc gcaacaccga gagactgtga gtatgcgctc ttcgtcttcc     1140
   cctctcccca tccgggccgc gcacccctgc ctccactgga ggaacctgtc agctcagggt     1200
   cctgtgcctg gggcagccct cgctagctct cccccatgca catcctgggg ttgagctctc      1260
   cgggagggca ctggccaggg aagggcctct gtccaaggag gggcgggtcc gctggcagct     1320
   gcgctagttc tccctcccct gctctcgtcc cgccactcgc agctccttgc tggctagttc      1380
   tctggggctg gggagcgggt agatagggga caagtactgg aggatgcccg gggcaagtga      1440
   gacgccactt tgttctccag agtccataaa cggagtcacc ttgcgattgc cagcatccag 1500
   gtcggtttca gagcccagtc ctcgctcttg tcgcaggctg gcgcggaggg gatagcaggg 1560
   agactcaaaa gagagaaact tgccttcccc gattttttgt caccctcctg ggggcgaagg 1620
   ttaggaagaa ggggtcatgg agtgcctggg ggtgcttctc acaggtcgcg gggagaaggg 1680
   tgccccagga cggcgacacc tcgcatagta gcctcgcgca gccccccgcc ccccacttct 1740
   ccggggaggg gaagacggcg tcaggcccct agggacttgt ctcagcgggc gactgcgagg 1800
   gaggaccgtg tcccatccgt taagcgaagt tagcactggt tctccagcgc aaaccagccc 1860
   aaccaggtct taccactgcg gcgacccggc ggtgcccggc tgccccctcc ggcccttcct 1920
   gctgaacccc tgcgtcccca tccacctttc tggcagtttc tgcgcccctt cacgtggcag 1980
   cagttcccct gccttcccc 1999
<210> 61
   <211> 999
   <212> DNA
   <213> Homo sapiens
<400> 61
   ggctgattag gaaactgtgg agaaaagtcc ttgtcattgc cccaggtaga gccgacctgg 60
   gaagcagcat cgtcattgga ttatctcggt cgttcccgct cacttaggcc aagcaggcga 120
   tgggtgtctc ggttctgcct ggaactgccg tttttcggag ggtgggccgc accccgcagt 180
   gcgtccaact ctcccagctg cctagatgtt ccttgggctt gggacaaagc ccccacagct 240
   tccaggtggg cccggggcgc accctagccc aggatggggt ggccagcttg ctccctgccc 300
   ctctcaaagg ctgcccattc gtccttaatc tttctggcag attccaccag gactccttta 360
   ccatgaattg tcccaccggg ggcccctgtg cctttccgtc gctggcaccg aactgcgtgg 420
   cgagagctgg gacaaaacgc cggagcggcc cggcggggga cgcacaggcg agtctcaggg 480
   ccccgccctc tcccgtgtcc ccctgttctg cgcgggcggg ctgtgcgggc ctggccagga 540
   gccgggtcgg aactccgtgc agcgatggca gctcgggcgc gcgccttgag gagccggtgg 600
   ggtgctgggg gacggagaag gtcccaaggt ccggggcgcg cgctttgctg ccgctggaag 660
   cgcgccccaa ttgtcgcgcc gcgtggttcg ctcggttaaa gccccgaccc gagggttatc 720
   gagctgcttc cgcccagtgg atacgaaccc ggactgtcct gagtgcattt ttttcctccc 780
   ttatagtctg ttaaattgac taataaaccc aacgcagcgt tctctgtgca gcttcaaaaa 840
   actcagtaat ttcgttagaa aacgttgaaa tccgacccca aagtattcag cccaaatgtt 900
   tagttaaagt aaccccgtgg gttaataaac taaacaaagg caacccatgc aaaaccggag 960
   caatgaaaac caggctacat aaacgaaggg aagtttata 999
<210> 62
   <211> 1999
   <212> DNA
   <213> Homo sapiens
<400> 62
   ttggagccag cgctcacagg gcagaaccag acgagcctca ctggaggcaa actgggaggt      60
   aggcgtgcgc tgtccgtggt gctgaaagct tgaccggcgc gagctggagc cgccaccggc     120
   tgcctcgggg tctcgccggg ccttacctgc tccgcgccct ggaagcagat cttgcagatg     180
   ggctggtggt gctggtgctg gtgcccagcg cgctggtcgc cgccgccact gctgctgctg     240
   cggctgctgc acaccgagcg cgtctcgggc tggtctccgg cgccccgccg ctcgcgctcg     300
   ccgcccgcgc cggcctcaga ctccccgggg ccgcctttcg ctgctgccgc ctccgggagg     360
   cgcctcggac cttccccgga gtcgccggcc gccgccactt cctggccggc gggctgcagg     420
   ggcaggggcg gaggcggcag ctcgtccgct cccctgcacc gcggggccac ctcccctagc     480
   ggctcgcttg gccccgcggc gcgctcgggg gtctcggggg acgcgggcag cggcggcagg     540
   tagcgcgggg ccgcggggac cggggccggc tctcccggcg gcggcgtcgg cggcggcggc     600
   gggggaggtt gcgggggagg ctcggcgtcc ccgctctccg ccccgcgaca ccgactgccg     660
   ccgtggccgc cctcaaagct catggttgtg ccgccgccgc cctcctgccg gcccggctgg     720
   cgggccgggc tctggctgca gggaaagaga gcgcggaggg ggcgggaggg agaggggaaa     780
   aggagggagg gggcccggac gcctggggct agggggcggg acggggaggg gatgcggaag     840
   gttctgcagc tgcggcggcg gcaggcgcgg ccgttcggtg gagccgccgg ctcggctctg     900
   atggaggcgg cgccgaattc ggctgcgcgt gagagccgcg ccgcggaagg gggggccgga     960
   gaagcgaggg ggcgggaggg aggagcggcg cggcgggggt gacggggcgc gggcgcgggg    1020
   tgggctgggg gcgcggatca gtgggacgga gttcggggtt cggctccgag cgggcgggct    1080
   ggaagtgggg gatccctcag ccgcctccac gggccggccc cgcgctcacg tcggttccgg    1140
   ggcggatgac ccctctccaa acggcgcagc gctgcggctc tcgtgagctg ggaagtaggg    1200
   ggcaggggag aggccgcggg tccagaaacc gttactggat gggccggtgg gatgtggcgc    1260
   gggccgggtg gggcgcgaca gtctgagccg agacccgcgt gggcttaagg gtgcgcgagg    1320
   cgggtgccct gggcgcgccc gaactggctg agcagtggag cgggaaaggg cgcgggaccc    1380
   gggactgtaa ccgccacttc caggccctcg ctccccgcgc ttggagccct caagggcact    1440
   ctcagggatc ctcgagagcc ttaaaacaga agtctctgga acctgtgtcc tctccctgtc    1500
   tgtcccgccc tcgaatccct gtgtcctcct cacccgctcc ctcctgcagt gagcatcccg    1560
   ggttgttggt aaagatcttg gtgcctggga ggtcggagct tcgtctcctg aaatggttta    1620
   tactagtgaa ccctggcgcc acgttctgtg gcttataatc actttcgtcg ttgccgcatg    1680
   aggaagcaaa tgacaccgcc ccttaccctg gaaaagtggc tgcagccttc cccggatctt 1740
   agttttactc accccgaagt caatttctcg gtaactccac cctgcaaaac ctctgtggga 1800
   ctcatcttca gggcagagct aacagttttc tttctggaaa aaaaaaaaaa tccctcacct 1860
   gcagggaact aggctgagaa tcgtgcacat gcagtagttt ccaaatccgt gcagtgtgag 1920
   atcataaagc accggattta tatgcggcag tgtgtctatc cgaattttca ctgatgtgac 1980
   gctttcagtc tttgacaca 1999
<210> 63
   <211> 499
   <212> DNA
   <213> Homo sapiens
<400> 63
   gctccacagt ttgtgatgtc taagaacccc ggccgtgcac cgacgctggg catgctgccc 60
   ccgcccccgt cgcccagctc gttaatctag agctatgccg gagcccgggt gggggccgcg 120
   gcgggccggg gcgcgcgcgg gccgcggggc tcagttgtgc tgctgttctc tccgcaggga 180
   cggcggctcc cggctggcgg cggcgcgccc ccgggctgtg aatgcgactc gcccctcggc 240
   cgcgctcccc gcccgcccgc ccgccgggac gtggtagggg atgcccagct ccactgcgat 300
   ggcagttggc gcgctctcca gttccctcct ggtcacctgc tgcctgatgg tggctctgtg 360
   cagtccgagc atcccgctgg agaagctggc ccaggcacca gagcagccgg gccaggagaa 420
   gcgtgagcac gcctctcggg acggcccggg gcgggtgaac gagctcgggc gcccggcgag 480
   ggacgagggc ggcagcggc 499
<210> 64
   <211> 1749
   <212> DNA
   <213> Homo sapiens
<400> 64
   ttttggtaca ggagtcattt attctgctat ggatatttcc tttatgaaat gctgctattt      60
   aagcatgaat gaaaaccttc catttgaaat gggcaagaca ttgttcatac ggatttaggc     120
   tgtggcgatt ttcgtctgca taaaggcact ctggttgctg ttcagtagcc aacatgattg     180
   attagggaag tggtggttca atcagaataa agtattcccc aagtattctg gatccctaag     240
   gaccagtgct tccaggaata cggtactgat gattccattt tgtggctatt ttttgacagt     300
   cctcagactg tcaaatagaa tctggcctaa aaggaggaca aggctctctg aagtgcagcc     360
   cttcgggcag ctgaaggtct ttctgcagat aacttttctc agatcgaatt ttttggctac     420
   attgatactc ttcggctctg tccttgccag aagttcggaa ggattccagc gccccacacc     480
   ttgcttgatt caccctcatc ccctccccta actggagaag ccgctgggtc ccgcgccagg     540
   ctcgcggtgg cttcagagta gcaggggagc aggcggctga tccggaggcc agtgtggggc     600
   cggcaagcgg tgactgtctc cagaggagca aaggagccga gtcttgtttt tcttggatca     660
   ggtttgggac ttttattctg tctgaccatt tccaccactt gcctcacaag agtctctgtc     720
   tcgaagcaca ggaccgaagc aaaatgccta atgaagcgtg cctgaggaag gggcaggggc     780
   ttgcaagtga cttgggaaga aggactgggg cgaagggaga aaggaggtta cgagttcgca     840
   cgttctcaca aaaccatttg aaaacatgag ctggagacgc caaattctgg gacccacgaa     900
   aggctttgga gctcgctcgg gctcctcgaa gttgggcgtg cgtcgcagaa cagtgctggg     960
   cgctctcttt cagcattttc ggcttttttc aagcccttgc gtagggtcgg gaaggccgtg    1020
   ggtgggctca gtcaggcttt aggtcgccag gaacccggct ggtcctctct cgacttctta    1080
   gcgtggggtc ccgccggccc tgccgcccgt ggccgccgaa gttcccgccc tcgccgaggg    1140
   ccctcgctcc ggagtggggc gcagacgcgg ccgccggccc gcagtccccc gcaggtgccg    1200
   cccaggacta gctgcccggc ggaggccgag cacgcttggc ggcagctgag cctccacccc    1260
   aagccccagc cggaggggcg cgtcccctgt cctcctcccg agcgagacga acgctcagca    1320
   gctcgttccc tgggcgccaa gaccgatttc caagtcgccc actttccccc tcgagggagc    1380
   tgttggcgct tctccagaag cctcctcggc tcccagctcc agcccctaaa ataaaagcac    1440
   cttgccagag agcgggggag gggagcagct gaacgaggag aatgaaaata ctgggagaac    1500
   gaccccattc tccaggaaaa ggtaatgagg ggaagtgaaa cagtgtgaac ttactcggaa    1560
   atgcaaaccg agttcaactc acccaggagc aaacaaacga cagcaagaca aatcagccac    1620
   cgcactcgcg gcttcccaga aagggcctca tgaatgagaa tgggttgcta ggtttccttc    1680
   cctctctcct gacaatcgct tcccacaaga cttccaccgc cgaaagaata caggccgggc    1740
   ctggtgact                                                            1749

## Description

### FIELD OF THE INVENTION

The present invention is in the field of biology and chemistry. In particular, the invention is in the field of molecular biology. More particular, the invention relates to the analysis of the methylation status of genomic regions. Most particularly, the invention is in the field of diagnosing colorectal cancer.

### BACKGROUND

Colorectal cancer (CRC) is the third most common cancer in males and the second in females, with over 1.2 million new cancer cases and 608,700 deaths estimated for 2008. Colorectal cancer, commonly known as bowel cancer, is a cancer from uncontrolled cell growth in the colon or rectum (parts of the large intestine), or in the appendix. Symptoms typically include rectal bleeding and anemia which are sometimes associated with weight loss and changes in bowel habits.

Most colorectal cancers occur due to lifestyle and increasing age, a genetic predisposition is known for the HNPCC (hereditary non-polyposis colorectal cancer) subgroup. It typically starts in the lining of the bowel and, if left untreated, can grow into the muscle layers underneath, and then through the bowel wall. Regular endoscopic control screenings are recommended starting at the age of 50.

It is therefore clear that there has been and remains today a long standing need for the identification of biomarkers which facilitate accurate and reliable diagnosis of colorectal cancer.

Multiple genetic and epigenetic mechanisms contribute to functional alterations of the tumor genome. Epigenetic modifications such as DNA methylation, have been found to occur already at the early stages of cancer development making them highly attractive for biomarker development. Hypermethylation within promoter regions is thought to induce tumor suppressor gene inactivation, whereas hypomethylation has been shown to lead to oncogene activation. In addition, hypomethylation of satellite regions might induce genomic instability.

The influence of copy number alterations (CNAs) on gene expression have mainly been shown to positively correlate, e.g., amplifications leading to an increase in gene expression. However, until now, the correlation between DNA methylation and gene expression, and in particular the influence of cancer differentially methylated regions (cDMRs) on gene expression patterns, have only been examined to a limited extent. Main limitations are the applied detection methods that allow the parallel analysis of methylation modifications only at selected genomic locations like e.g. CpG islands within promoter regions, or by the fact that studies have been performed on single genes. Moreover, long-range epigenetic mechanisms influence the cancer transcriptome. Such mechanisms, involving DNA methylation and histone modifications over large chromosomal stretches have been found in both copy-number dependent and independent regions.

WO 2012/034170 discloses a number of genes and chromosomal regions shown to be differentially methylated between caner and normal cells (Table 3).

To date, the most prominent differentially methylated genes in colorectal cancer and, therefore, be used as a biomarker for the detection of colorectal cancer, are, as recently reported, MLH1, APC, SEPT9 and ALX4 (Banerjee et al., Biomark Med 3, 397-410 (2009)). MLH1 and APC are not methylated at all or only in a distinct subgroup of cancers. SEPT9 and ALX4, which are located in a region that is subject to somatic copy number alterations (CNAs), show a variable performance for being used as a biomarker for colorectal cancer.

Accordingly, there is a need in the state of the art of studying genome-wide aberrant DNA methylation that can be associated with high confidence to colorectal cancer and identifying biomarkers for colorectal cancer diagnosis based on the epigenetic cancer information. The inventors hypothesized that enhanced biomarkers may be found in CNA-free regions, i.e. regions which are not subject to copy number alterations.

### SUMMARY OF THE INVENTION

The invention encompasses the identification and selection of a novel genomic region which is hypermethylated in subjects with colorectal cancer compared to subjects without colorectal cancer so as to provide a simple and reliable test for diagnosing colorectal cancer. Nucleic acids which selectively hybridize to the genomic region and products thereof are also encompassed within the scope of the invention as are compositions containing said nucleic acids and nucleic acids for use in diagnosing colorectal cancer. Further encompassed by the invention is the use of nucleic acids each thereof selectively hybridizing to the genomic region or products thereof to monitor disease progression or regression in a patient and the efficacy of therapeutic regimens.

For the first time the inventors have identified differentially methylated regions (DMRs) in a set of heterogeneous colorectal cancers by genome-wide approaches based on high throughput sequencing (methylated DNA immunoprecipitation, MeDIP-Seq) (Table 1) and thus, by quantifying the methylation status of specific genomic regions, permit the accurate and reliable diagnosis of colorectal cancer. The inventors found that CNAs influence DNA methylation patterns and mask the effects of DNA methylation marks on gene expression. They assume that CNAs do not only introduce a serious bias to biomarker discovery but also distort confidence of diagnosis. Therefore, in contrast to the known biomarkers, the herein described biomarkers are located in CNA-free regions.

The present invention, thus, contemplates a method for diagnosis of colorectal cancer, comprising the steps of analysing in a sample of a subject the DNA methylation status of at least genomic region 4 (GR NO. 4), wherein, if the at least genomic region 4 is hypermethylated, the sample is designated as colorectal cancer positive. The genomic regions are defined according to the UCSC hgl9 human genome.

**Table 1: DMRs in colorectal cancer positive samples. Column 1: genomic region number according to GR No.; Column 2 to 4: locus in genome (human genome: UCSC hgl9) determined by the chromosome number and start and stop position of the sequence; Column 5: length of sequence; Column 6: associated or nearby gene; Column 7: differential methylation status found in colorectal cancer positive sample.**

| **GR NO** | **SEQ ID NO** | **Chromosome** | **Start** | **Stop** | **Size of DMR** | **HUGO gene name** | **Differential methylation status +: hypermeth. -: hypometh.** |
|---|---|---|---|---|---|---|---|
| 1 | 1 | chr12 | 95941501 | 95943500 | 2000 | USP44 | + |
| 2 | 2 | chr2 | 115919751 | 115921250 | 1500 | DPP10 | + |
| 3 | 3 | chr3 | 192231751 | 192233750 | 2000 | FGF12; RP11-91M9.1 | + |
| 4 | 4 | chr1 | 99469501 | 99471250 | 1750 | RP11-254O21.1; RP5-896L10.1 | + |
| 5 | 5 | chr10 | 7453501 | 7455500 | 2000 | | + |
| 6 | 6 | chr1 | 200010001 | 200011500 | 1500 | NR5A2 | + |
| 7 | 7 | chr12 | 3602001 | 3603000 | 1000 | PRMT8 | + |
| 8 | 8 | chr4 | 144621001 | 144622500 | 1500 | FREM3; RP13-578N3.3 | + |
| 9 | 9 | chr7 | 24322501 | 24325500 | 3000 | NPY | + |
| 10 | 10 | chr12 | 5018001 | 5020750 | 2750 | KCNA1 | + |
| 11 | 11 | chr3 | 192125501 | 192128750 | 3250 | FGF12 | + |
| 12 | 12 | chr6 | 73332001 | 73333500 | 1500 | KCNQ5; RP3-474G15.2 | + |
| 13 | 13 | chr1 | 111217001 | 111218500 | 1500 | KCNA3 | + |
| 14 | 14 | chr1 | 119527501 | 119528750 | 1250 | TBX15 | + |
| 15 | 15 | chr6 | 11143751 | 11144750 | 1000 | | - |
| 16 | 16 | chr10 | 115860001 | 115860500 | 500 | | - |
| 17 | 17 | chr5 | 1973501 | 1974500 | 1000 | | - |
| 18 | 18 | chr2 | 7100501 | 7101500 | 1000 | AC013460.1; AC017076.1; RNF144A | + |
| 19 | 19 | chr12 | 16757501 | 16758500 | 1000 | LMO3 | + |
| 20 | 20 | chr12 | 101916501 | 101917500 | 1000 | | - |
| 21 | 21 | chr2 | 68545751 | 68547500 | 1750 | CNRIP1 | + |
| 22 | 22 | chr6 | 36808251 | 36809250 | 1000 | | + |
| 23 | 23 | chr10 | 3805001 | 3806000 | 1000 | RP11-184A2.3 | - |
| 24 | 24 | chr2 | 22410751 | 22411500 | 750 | AC068044.1; AC068490.2 | - |
| 25 | 25 | chr7 | 6324251 | 6325000 | 750 | | - |
| 26 | 26 | chr2 | 69428251 | 69428750 | 500 | ANTXR1 | - |
| 27 | 27 | chr16 | 4000001 | 4001000 | 1000 | | - |
| 28 | 28 | chr1 | 38838251 | 38839000 | 750 | | - |
| 29 | 29 | chr4 | 188666001 | 188667000 | 1000 | | - |
| 30 | 30 | chr6 | 151561001 | 151561500 | 500 | AKAP12 | + |
| 31 | 31 | chr1 | 181638251 | 181639000 | 750 | CACNA1E | - |
| 32 | 32 | chr4 | 185000501 | 185001250 | 750 | | - |
| 33 | 33 | chr2 | 4816001 | 4816500 | 500 | | - |
| 34 | 34 | chr5 | 61041001 | 61041500 | 500 | CTD-2170G1.1 | - |
| 35 | 35 | chr3 | 196363251 | 196363750 | 500 | | - |
| 36 | 36 | chr4 | 183369001 | 183369750 | 750 | ODZ3 | + |
| 37 | 37 | chr1 | 158151001 | 158151750 | 750 | CDID | + |
| 38 | 38 | chr7 | 145833251 | 145834000 | 750 | CNTNAP2 | |
| 39 | 39 | chr1 | 170629751 | 170631250 | 1500 | | + |
| 40 | 40 | chr2 | 467501 | 469000 | 1500 | | + |
| 41 | 41 | chr16 | 72911501 | 72912000 | 500 | ATBF1 | - |
| 42 | 42 | chr22 | 48575751 | 48576250 | 500 | | - |
| 43 | 43 | chr3 | 113968001 | 113968500 | 500 | | - |
| 44 | 44 | chr2 | 55062251 | 55062750 | 500 | EML6 | - |
| 45 | 45 | chr6 | 7468251 | 7469250 | 1000 | | - |
| 46 | 46 | chr16 | 8172251 | 8172750 | 500 | | - |
| 47 | 47 | chr7 | 154657251 | 154657750 | 500 | DPP6 | - |
| 48 | 48 | chr1 | 244964001 | 244965000 | 1000 | | - |
| 49 | 49 | chr1 | 121260501 | 121261000 | 500 | | + |
| 50 | 50 | chr10 | 120683751 | 120684250 | 500 | | - |
| 51 | 51 | chr10 | 106905251 | 106905750 | 500 | SORCS3 | - |
| 52 | 52 | chr10 | 83633751 | 83635000 | 1250 | NRG3 | + |
| 53 | 53 | chr12 | 99288001 | 99289750 | 1750 | ANKS1B | + |
| 54 | 54 | chr12 | 103889251 | 103889750 | 500 | C12orf42 | + |
| 55 | 55 | chr16 | 22825251 | 22826750 | 1500 | HS3ST2 | + |
| 56 | 56 | chr19 | 58125501 | 58126500 | 1000 | ZNF134 | + |
| 57 | 57 | chr2 | 12858251 | 12859250 | 1000 | TRIB2 | + |
| 58 | 58 | chr22 | 25678501 | 25679750 | 1250 | CTA-221G9.9; RP3-462D8.2 | + |
| 59 | 59 | chr3 | 147124751 | 147125500 | 750 | ZIC1 | + |
| 60 | 60 | chr4 | 20254501 | 20256500 | 2000 | SLIT2 | + |
| 61 | 61 | chr5 | 72593751 | 72594750 | 1000 | | + |
| 62 | 62 | chr5 | 16179001 | 16181000 | 2000 | MARCH11; RP11-19O2.2 | + |
| 63 | 63 | chr7 | 49814751 | 49815250 | 500 | VWC2 | + |
| 64 | 64 | chr8 | 54788751 | 54790500 | 1750 | RGS20 | + |

The invention also relates to the use of nucleic acid molecule that hybridizes under stringent conditions in the vicinity of one of the genomic regions 4, wherein said vicinity is any position having a distance of up to 500 nt from the 3' or 5' end of said genomic region, wherein said vicinity includes the genomic region itself, for the diagnosis of colorectal cancer.

The following detailed description of the invention refers, in part, to the accompanying drawings and does not limit the invention.

### DEFINITIONS

The following definitions are provided for specific terms which are used in the following.

The articles "a" and "an" are used herein to refer to one or to more than one (i.e. to at least one) of the grammatical object of the article. By way of example, "an element" means one element or more than one element. In contrast, "one" is used to refer to a single element.

As used herein, the term "amplified", when applied to a nucleic acid sequence, refers to a process whereby one or more copies of a particular nucleic acid sequence is generated from a nucleic acid template sequence, preferably by the method of polymerase chain reaction. Other methods of amplification include, but are not limited to, ligase chain reaction (LCR), polynucleotide-specific based amplification (NSBA), or any other method known in the art.

As used herein, the term "biomarker" refers to (a) a genomic region that is differentially methylated, e.g. hypermethylated or hypomethylated, or (b) a gene that is differentially expressed, wherein the status (hypo-/hypermethylation and/or up-/downregulated expression) of said biomarker can be used for diagnosing colorectal cancer or a stage of colorectal cancer as compared with those not having colorectal cancer. Within the context of the invention, a genomic region or parts thereof or fragment thereof are used as a biomarker for colorectal cancer. Within this context "parts of a genomic region" or a "fragment of a biomarker" means a portion of the genomic region or a portion of a biomarker comprising 1 or more CpG positions.

As used herein, the term "composition" refers to any mixture. It can be a solution, a suspension, liquid, powder, a paste, aqueous, non-aqueous or any combination thereof.

The term "CpG position" as used herein refers to a region of DNA where a cytosine nucleotide is located next to a guanine nucleotide in the linear sequence of bases along its length. "CpG" is shorthand for "C-phosphate-G", that is, cytosine and guanine separated by a phosphate, which links the two nucleosides together in DNA. Cytosines in CpG dinucleotides can be methylated to form 5-methylcytosine. This methylation of cytosines of CpG positions is a major epigenetic modification in multicellular organisms and is found in many human diseases including colorectal cancer.

As used herein, the term "diagnosis" refers to the identification of the disease (colorectal cancer) at any stage of its development, and also includes the determination of predisposition of a subject to develop the disease. In a preferred embodiment of the invention, diagnosis of colorectal cancer occurs prior to the manifestation of symptoms. Subjects with a higher risk of developing the disease are of particular concern. The diagnostic method of the invention also allows confirmation of colorectal cancer in a subject suspected of having colorectal cancer.

As used herein, the term "differential expression" refers to a difference in the level of expression of the RNA and/or protein products of one or more biomarkers, as measured by the amount or level of RNA or protein. In reference to RNA, it can include difference in the level of expression of mRNA, and/or one or more spliced variants of mRNA and/or the level of expression of small RNA (miRNA) of the biomarker in one sample as compared with the level of expression of the same one or more biomarkers of the invention as measured by the amount or level of RNA, including mRNA, spliced variants of mRNA or miRNA in a second sample or with regard to a threshold value. "Differentially expressed" or "differential expression" can also include a measurement of the protein, or one or more protein variants encoded by the inventive biomarker in a sample as compared with the amount or level of protein expression, including one or more protein variants of the biomarker in another sample or with regard to an threshold value. Differential expression can be determined, e.g. by array hybridization, next generation sequencing, RT-PCR or an immunoassay and as would be understood by a person skilled in the art.

As used herein, the term "differential methylation" or "aberrant methylation" refers to a difference in the level of DNA/cytosine methylation in a colorectal cancer positive sample as compared with the level of DNA methylation in a colorectal cancer negative sample. The "DNA methylation status" is interchangeable with the term "DNA methylation level" and can be assessed by determining the ratio of methylated and non-methylated DNA of a genomic region or a portion thereof and is quoted in percentage. For example, the methylation status of a sample is 60% if 60 % of the analysed genomic region of said sample is methylated and 40 % of the analysed genomic region of said sample is not methylated.

The methylation status can be classified as increased ("hypermethylated"), decreased ("hypomethylated") or normal as compared to a benign sample. The term "hypermethylated" is used herein to refer to a methylation status of at least more than 10% methylation in the tumour in comparison to the maximal possible methylation value in the normal, most preferably above 15 %, 20 %, 25% or 30% of the maximum values. For comparison, a hypomethylated sample has a methylation status of less than 10%, most preferably below 15%, 20%, 25% or 30% of the minimal methylation value in the normal.

The percentage values can be estimated from bisulphite mass spectrometry data (Epityper). Being obvious to the skilled person, the measurement error of the method (ca 5 %) and the error coming from preparation of the sample must be considered. Particularly, the aforementioned values assume a sample which is not contaminated with other DNA (e.g. micro dissected sample) than those coming from colorectal cells. As would be understood to the skilled person the values must be recalculated for contaminated samples (e.g. macro dissected samples). If desired, other methods can be used, such as the methods described in the following for analyzing the methylation status. However, the skilled person readily knows that the absolute values as well as the measurement error can differ for different methods and he knows how to compensate for this.

The term, "analyzing the methylation status" or "measuring the methylation", as used herein, relates to the means and methods useful for assessing and quantifying the methylation status. Useful methods are bisulphite-based methods, such as bisulphite-based mass spectrometry, bisulphite-based sequencing methods or enrichment methods such as MeDIP-Sequencing methods. Likewise, DNA methylation can also be analyzed directly via single-molecule real-time sequencing, single-molecule bypass kinetics and single-molecule nanopore sequencing.

As used herein, the term "genomic region" refers to a sector of the genomic DNA of any chromosome that can be subject to differential methylation within said sector and may be used as a biomarker for the diagnosis of colorectal cancer according to the invention. For example, each sequence listed in Table 1 and Table 2 with the corresponding genomic region numbers 1 to 64 is a genomic region according to the invention. A genomic region can comprise the full sequence or parts thereof provided that at least one CpG position is comprised by said part. Preferably, said part comprises between 1 to 15 CpG positions. In another embodiment, the genomic region can comprise at least 1, 2, 3, 4, 5, 6, 7, 8, 9, 10, 11, 12, 13, 14, or 15 CpG positions.

Genomic regions that occur in the vicinity of genes may be associated with the names of those genes for descriptive purpose. This may not mean, that the genomic region comprises all or a part of that gene or functional elements of it. In case of doubt, solely the locus and/or the sequence shall be used.

As used herein, the term "in the vicinity of a genomic region" refers to a position outside or within said genomic region. As would be understood to a person skilled in the art the position may have a distance up to 500 nucleotides (nt), 400 nt, 300 nt, 200 nt, 100 nt, 50 nt, 20 nt or 10 nt from the 5' or 3' end of the genomic region. Alternatively, the position is located at the 5' or 3' end of said genomic region, or, the position is within said genomic region.

The term "genomic region specific primers" as used herein refers to a primer pair hybridizing to a flanking sequence of a target sequence to be amplified. Such a sequence starts and ends in the vicinity of a genomic region. In one embodiment, the target sequence to be amplified comprises the whole genomic region and its complementary strand. In a preferred embodiment, the target sequence comprises 1, 2, 3, 4, 5, 6, 7, 8, 9, 10, 11, 12, 13, 14, 15 or even more CpG positions of the genomic region and the complementary strand thereof. In general, the hybridization position of each primer of the primer pair can be at any position in the vicinity of a genomic region provided that the target sequence to be amplified comprises at least one CpG position of said genomic region. As would be obvious to the skilled person, the sequence of the primer depends on the hybridization position and on the method for analyzing the methylation status, e.g. if a bisulphite based method is applied, part of the sequence of the hybridization position may be converted by said bisulphite. Therefore, in one embodiment, the primers may be adapted accordingly to still enable or disable hybridization (e.g. in methylation specific PCR).

The term "genomic region specific probe" as used herein refers to a probe that selectively hybridizes to a genomic region. In one embodiment a genomic region specific probe can be a probe labelled, for example with a fluorophore and a quencher, such as a TaqMan® probe or a Molecular Beacons probes. In a preferred embodiment, the probe can hybridize to a position of the genomic region that can be subject to hypermethylation according to the inventive method. Hereby, the probe hybridizes to positions with either a methylated CpG or a unmethylated CpG in order to detect methylated or unmethylated CpGs. In a preferred embodiment, two probes are used, e.g. in a methylight (qPCR assay) assay. The first probe hybridizes only to positions with a methylated CpG, the second probe hybridizes only to positions with a unmethylated CpG, wherein the probes are differently labelled and, thus, allow for discrimination between unmethylated and methylated sites in the same sample.

As used herein, the terms "hybridizing to" and "hybridization" are interchangeable used with the term "specific for" and refer to the sequence specific non-covalent binding interactions with a complementary nucleic acid, for example, interactions between a target nucleic acid sequence and a target specific nucleic acid primer or probe. In a preferred embodiment a nucleic acid, which hybridizes is one which hybridizes with a selectivity of greater than 70%, greater than 80%, greater than 90% and most preferably of 100% (i.e. cross hybridization with other DNA species preferably occurs at less than 30%, less than 20%, less than 10%). As would be understood to a person skilled in the art, a nucleic acid, which "hybridizes" to the DNA product of a genomic region of the invention can be determined taking into account the length and composition.

As used herein, "isolated" when used in reference to a nucleic acid means that a naturally occurring sequence has been removed from its normal cellular (e.g. chromosomal) environment or is preferably synthesised in a non-natural environment (e.g. artificially synthesised). Thus, an "isolated" sequence may be in a cell-free solution or placed in a different cellular environment.

As used herein, a "kit" is a packaged combination optionally including instructions for use of the combination and/or other reactions and components for such use.

As used herein, "nucleic acid(s)" or "nucleic acid molecule" generally refers to any ribonucleic acid or deoxyribonucleic acid, which may be unmodified or modified DNA. "Nucleic acids" include, without limitation, single- and double-stranded nucleic acids. As used herein, the term "nucleic acid(s)" also includes DNA as described above that contain one or more modified bases. Thus, DNA with backbones modified for stability or for other reasons are "nucleic acids". The term "nucleic acids" as it is used herein embraces such chemically, enzymatically or metabolically modified forms of nucleic acids, as well as the chemical forms of DNA characteristic of viruses and cells, including for example, simple and complex cells.

The term "primer", as used herein, refers to an nucleic acid, whether occurring naturally as in a purified restriction digest or preferably produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. For example, for diagnostic applications, depending on the complexity of the target sequence, the nucleic acid primer typically contains 15-25 or more nucleotides, although it may contain fewer nucleotides. The factors involved in determining the appropriate length of primer are readily known to one of ordinary skill in the art. In general, the design and selection of primers embodied by the instant invention is according to methods that are standard and well known in the art, see Dieffenbach, C. W., Lowe, T. M. J., Dveksler, G. S. (1995) General Concepts for PCR Primer Design. In: PCR Primer, A Laboratory Manual (Eds. Dieffenbach, C. W, and Dveksler, G. S.) Cold Spring Harbor Laboratory Press, New York, 133-155; Innis, M. A., and Gelfand, D. H. (1990) Optimization of PCRs. In: PCR protocols, A Guide to Methods and Applications (Eds. Innis, M. A., Gelfand, D. H., Sninsky, J. J;, and White, T. J.) Academic Press, San Diego, 3-12; Sharrocks, A. D. (1994) The design of primers for PCR. In: PCR Technology, Current Innovations (Eds. Griffin, H. G., and Griffin, A. M, Ed.) CRC Press, London, 5-11.

As used herein, the term "probe" means nucleic acid and analogs thereof and refers to a range of chemical species that recognise polynucleotide target sequences through hydrogen bonding interactions with the nucleotide bases of the target sequences. The probe or the target sequences may be single- or double-stranded DNA. A probe is at least 8 nucleotides in length and less than the length of a complete polynucleotide target sequence. A probe may be 10, 20, 30, 50, 75, 100, 150, 200, 250, 400, 500 and up to 2000 nucleotides in length. Probes can include nucleic acids modified so as to have a tag which is detectable by fluorescence, chemiluminescence and the like ("labelled probe"). The labelled probe can also be modified so as to have both a detectable tag and a quencher molecule, for example Taqman® and Molecular Beacon® probes. The nucleic acid and analogs thereof may be DNA, or analogs of DNA, commonly referred to as antisense oligomers or antisense nucleic acid. Such DNA analogs comprise but are not limited to 2-'O-alkyl sugar modifications, methylphosphonate, phosphorothiate, phosphorodithioate, formacetal, 3'-thioformacetal, sulfone, sulfamate, and nitroxide backbone modifications, and analogs wherein the base moieties have been modified. In addition, analogs of oligomers may be polymers in which the sugar moiety has been modified or replaced by another suitable moiety, resulting in polymers which include, but are not limited to, morpholino analogs and peptide nucleic acid (PNA) analogs (Egholm, et al. Peptide Nucleic Acids (PNA)-Oligonucleotide Analogues with an Achiral Peptide Backbone, (1992)).

The term "sample" or "biological sample" is used herein to refer to colorectal tissue, blood, urine, semen, colorectal secretions or isolated colorectal cells originating from a subject, preferably from colorectal tissue, colorectal secretions or isolated colorectal cells, most preferably to colorectal tissue.

As used herein, the term "DNA sequencing" or "sequencing" refers to the process of determining the nucleotide order of a given DNA fragment. As known to those skilled in the art, sequencing techniques comprise sanger sequencing and next-generation sequencing, such as 454 pyrosequencing, Illumina (Solexa) sequencing and SOLiD sequencing.

The term "bisulphite sequencing" refers to a method well-known to the person skilled in the art comprising the steps of (a) treating the DNA of interest with bisulphite, thereby converting non-methylated cytosines to uracils and leaving methylated cytosines unaffected and (b) sequencing the treated DNA, wherein the existence of a methylated cytosine is revealed by the detection of a non-converted cytosine and the absence of a methylated cytosine is revealed by the detection of a thymine.
As used herein, the terms "subject" and "patient" are used interchangeably to refer to an animal (e.g., a mammal, a fish, an amphibian, a reptile, a bird and an insect). In a specific embodiment, a subject is a mammal (e.g., a non-human mammal and a human). In another embodiment, a subject is a primate (e.g., a chimpanzee and a human). In another embodiment, a subject is a human. In another embodiment, the subject is a male human with or without colorectal cancer.

### DETAILED DESCRIPTION OF THE INVENTION

The practice of the present invention employs in part conventional techniques of molecular biology, microbiology and recombinant DNA techniques, which are within the skill of the art. Such techniques are explained fully in the literature. See, e.g., Sambrook, Fritsch & Maniatis, 1989, Molecular Cloning: A Laboratory Manual, Second Edition; Oligonucleotide Synthesis (M. J. Gait, ed., 1984); Nucleic Acid Hybridization (B. D. Harnes & S. J. Higgins, eds., 1984); A Practical Guide to Molecular Cloning (B. Perbal, 1984); and a series, Methods in Enzymology (Academic Press, Inc.); Short Protocols In Molecular Biology, (Ausubel et al., ed., 1995).

The invention as disclosed herein identifies genomic regions that are useful in diagnosing colorectal cancer. By definition, the identified genomic regions are biomarkers for colorectal cancer. In order to use these genomic regions (as biomarkers), the invention teaches the analysis of the DNA methylation status of said genomic regions. The invention further encompasses genomic region specific nucleic acids. The invention further contemplates the use of said genomic region specific nucleic acids to analyse the methylation status of a genomic region, either directly or indirectly by methods known to the skilled person and explained herein. The invention further discloses a composition and kit comprising said nucleic acids for the diagnosis of colorectal cancer.

To address the need in the art for a more reliable diagnosis of colorectal cancer, the peculiarities of the DNA methylation status across the whole genome of colorectal cancer positive samples were examined in comparison to colorectal cancer negative samples. The inventors found genomic regions, that are subject to an differential methylation status. The invention in particular teaches the analysis of genomic region 4 that is hypermethylated in samples from patients having colorectal cancer. Superior to current diagnostic methods, the invention discloses genomic regions, wherein most astonishingly a single genomic region is able to diagnose colorectal cancer with high confidence. If at least genomic region 4 is hypermethylated, the sample can be designated as colorectal cancer positive. The inventors found that the identified genomic region is located in a CNA-free region. CNAs are alterations of the DNA of a genome that results in the cell having an abnormal number of copies of one or more sections of the DNA. The inventors partly attribute the superiority of the new biomarker to the fact that it is located in a CNA-free region and, therefore, is not subject to distorting effects of CNA regions.

Accordingly, the invention relates to a method for diagnosis of colorectal cancer, comprising the steps of analysing in a sample of a subject the DNA methylation status of at least genomic region 4 of Table 1, wherein, if the at least one genomic region is hypermethylated, the sample is designated as colorectal cancer positive.
Significantly, the inventors found that a minimum of one genomic region is sufficient to accurately discriminate between malignant and benign tissues. The extension with additional sites even increases the discriminatory potential of the marker set. Thus, in another embodiment, the invention relates to a method, wherein the methylation status of a further genomic region and/or a further biomarker is analysed.

In one embodiment of the invention, one or more known colorectal cancer biomarker are additionally analysed. Such colorectal cancer biomarkers can be a gene, e.g. encoding for SEPT9, ALX4, BRAF, MLH1, TMEFF2, BMP3, EYA2,or APC. Such biomarkers can also be based on gene expression, e.g. of said encoding genes. The analysis of the biomarkers within this context can be the analysis of the methylation status, the analysis of the gene expression (mRNA), or the analysis of the amount or concentration or activity of protein.

In another embodiment one or more further genomic region according to the invention is analysed. For example, a total of 2, 3, 4, 5, 6, 7, 8, 9 or 10 genomic regions selected from the group of Table 1 is analysed. In a specific embodiment, at least two genomic regions are analysed: The first genomic region has the sequence according to GR NO. 4 and the second genomic region is selected from the group of Table 1. However, it is to be understood that the invention is not restricted to a specific combination. As will be understood by the skilled person the presence of differential methylation of the biomarkers in the biological sample is determined; and the presence of differential methylation of said biomarkers is correlated with a positive indication of colorectal cancer in said subject.

The method is particularly useful for early diagnosis of colorectal cancer. The method is useful for further diagnosing patients having symptoms associated with colorectal cancer. The method of the present invention can further be of particular use with patients having an enhanced risk of developing colorectal cancer (e.g., patients having a familial history of colorectal cancer and patients identified as having a mutant oncogene). The method of the present invention may further be of particular use in monitoring the efficacy of treatment of a colorectal cancer patient (e.g. the efficacy of chemotherapy).

In one embodiment of the method, the sample comprises cells obtained from a patient. The cells may be found in a colorectal tissue sample collected, for example, by a colorectal tissue biopsy or histology section, or a bone marrow biopsy if metastatic spreading has occurred. In another embodiment, the patient sample is a colorectal-associated body fluid. Such fluids include, for example, blood fluids, lymph, and feces. From the samples cellular or cell free DNA is isolated using standard molecular biological technologies and then forwarded to the analysis method.

In order to analyse the methylation status of a genomic region, conventional technologies can be used.

Either the DNA of interest may be enriched, for example by methylated DNA immunoprecipitation (MeDIP) followed by real time PCR analyses, array technology, or next generation sequencing. Alternatively, the methylation status of the DNA can be analysed directly or after bisulphite treatment.
In one embodiment, bisulphite-based approaches are used to preserve the methylation information. Therefore, the DNA is treated with bisulphite, thereby converting non-methylated cytosine residues into uracil while methylated cytosines are left unaffected. This selective conversion makes the methylation easily detectable and classical methods reveal the existence or absence of DNA (cytosine) methylation of the DNA of interest. The DNA of interest may be amplified before the detection if necessary. Such detection can be done by mass spectrometry or, the DNA of interest is sequenced. Suitable sequencing methods are direct sequencing and pyrosequencing. In another embodiment of the invention the DNA of interest is detected by a genomic region specific probe that is selective for that sequence in which a cytosine was either converted or not converted. Other techniques that can be applied after bisulphite treatment are for example methylation-sensitive single-strand conformation analysis (MS-SSCA), high resolution melting analysis (HRM), methylation-sensitive single-nucleotide primer extension (MS-SnuPE), methylation specific PCR (MSP) and base-specific cleavage.
In an alternative embodiment the methylation status of the DNA is analysed without bisulphite treatment, such as by methylation specific enzymes or by the use of a genomic region specific probe or by an antibody, that is selective for that sequence in which a cytosine is either methylated or non-methylated.
In a further alternative, the DNA methylation status can be analysed via single-molecule real-time sequencing, single-molecule bypass kinetics and single-molecule nanopore sequencing. These techniques, which are within the skill of the art, are fully explained in: Flusberg et al. Direct detection of DNA methylation during single-molecule, real-time sequencing. Nature methods 7(6): 461-467. 2010; Summerer. High-Througput DNA Sequencing Beyond the Four-Letter Code: Epigenetic Modifications Revealed by Single-Molecule Bypass Kinetics. ChemBioChem 11: 2499-2501. 2010; Clarke et al. Continuous base identification for single-molecule nanopore DNA sequencing. Nature Nanotechnology 4: 265-270. 2009; Wallace et al. Identification of epigenetic DNA modifications with a protein nanopore. Chemical Communication 46:8195-8197.

To translate the raw data generated by the detection assay (e.g. a nucleotide sequence) into data of predictive value for a clinician, a computer-based analysis program can be used.

The profile data may be prepared in a format suitable for interpretation by a treating clinician. For example, rather than providing raw nucleotide sequence data or methylation status, the prepared format may represent a diagnosis or risk assessment (e.g. likelihood of cancer being present or the subtype of cancer) for the subject, along with recommendations for particular treatment options.

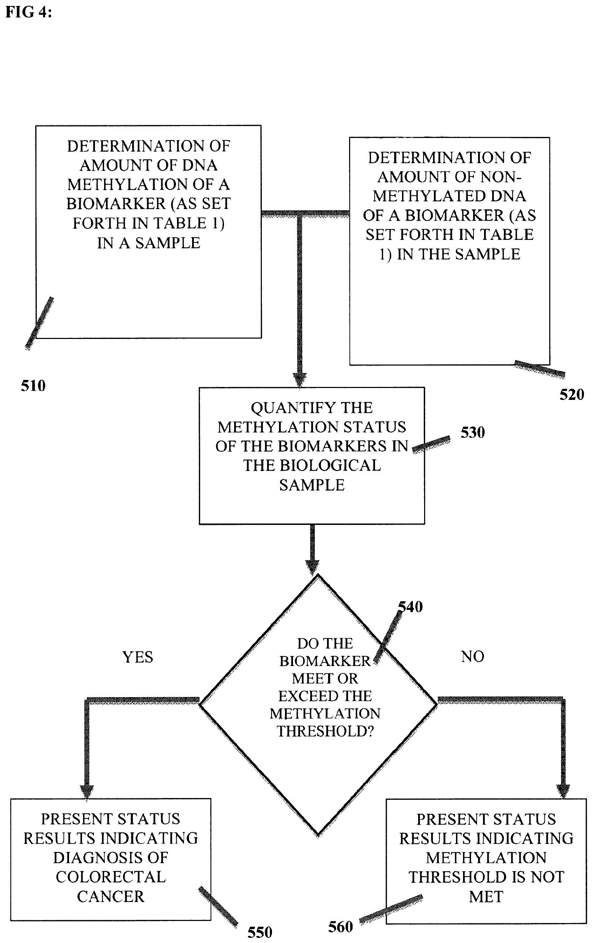

In one embodiment of the present invention, a computing device comprising a client or server component may be utilized. Figure 4 is an exemplary diagram of a client/server component, which may include a bus **210,** a processor **220,** a main memory **230,** a read only memory (ROM) **240,** a storage device **250,** an input device **260,** an output device **270,** and a communication interface **280.** Bus 210 may include a path that permits communication among the elements of the client/server component.

Processor **220** may include a conventional processor or microprocessor, or another type of processing logic that interprets and executes instructions. Main memory **230** may include a random access memory (RAM) or another type of dynamic storage device that stores information and instructions for execution by processor 220. ROM **240** may include a conventional ROM device or another type of static storage device that stores static information and instructions for use by processor **220.** Storage device **250** may include a magnetic and/or optical recording medium and its corresponding drive.

Input device 260 may include a conventional mechanism that permits an operator to input information to the client/server component, such as a keyboard, a mouse, a pen, voice recognition and/or biometric mechanisms, etc. Output device **270** may include a conventional mechanism that outputs information to the operator, including a display, a printer, a speaker, etc. Communication interface **280** may include any transceiver-like mechanism that enables the client/server component to communicate with other devices and/or systems. For example, communication interface **280** may include mechanisms for communicating with another device or system via a network.

As will be described in detail below, the client/server component, consistent with the principles of the invention, may perform certain measurement determinations of methylation, calculations of methylation status, and/or correlation operations relating to the diagnosis of colorectal cancer. It may further optionally output the presentation of status results as a result of the processing operations conducted. The client/server component may perform these operations in response to processor **220** executing software instructions contained in a computer-readable medium, such as memory **230.** A computer-readable medium may be defined as a physical or logical memory device and/or carrier wave.

The software instructions may be read into memory **230** from another computer-readable medium, such as data storage device **250,** or from another device via communication interface **280.** The software instructions contained in memory **230** may cause processor **220** to perform processes that will be described later. Alternatively, hardwired circuitry may be used in place of or in combination with software instructions to implement processes consistent with the principles of the invention. Thus, implementations consistent with the principles of the invention are not limited to any specific combination of hardware circuitry and software.

FIG. 4 is a flowchart of exemplary processing of methylation status for biomarkers present in biological samples according to an implementation consistent with the principles of the present invention. Processing may begin with quantifying the methylation **510** and non-methylation **520** of the DNA of a biological sample for a biomarker of Table 1 or, in an alternative embodiment, for more than a single biomarker if desired (see above). The processor may then quantify the methylation status **530,** as described above, as the ratio of methylated DNA to non-methylated of the biological sample for the biomarker(s). The methylation status may then be evaluated either via a computing device **540** or by human analysis to determine if the biomarker(s) meet or exceed a predetermined methylation threshold. If the threshold is met or exceeded, the computing device may then, optionally, present a status result indicating a positive diagnosis of colorectal cancer **550.** Alternatively, if the threshold is not met, then the computing device may, optionally, present a status result indicating that the threshold is not satisfied **560.** It is noted that the output displaying results may differ depending on the desired presentation of results. For example, the output may be quantitative in nature, e.g., displaying the measurement values of each of the biomarkers in relation to the predetermined methylation threshold value. The output may be qualitative, *e.g*., the display of a color or notation indicating a positive result for colorectal cancer, or a negative results for colorectal cancer, as the case may be. Notably, this process may be repeated multiple times using different genomic regions, as set forth in Table 1. The computing device may alternatively be programmed to permit the analysis of more than one genomic region at one time.

In some embodiments, the results are used in a clinical setting to determine a further diagnostic (e.g., additional further screening (e.g., other markers or diagnostic biopsy) course of action. In other embodiments, the results are used to determine a treatment course of action (e.g., choice of therapies or watchful waiting).

The inventors surprisingly found that the methylation status within a genomic region according to the invention is almost constant, leading to a uniform distribution of either hyper- or hypomethylated CpG positions within said genomic region. In one embodiment of the invention, all CpG positions of a genomic region are analysed. In a specific embodiment, CpG positions in the vicinity of the genomic region may be analysed. In an alternative embodiment, a subset of CpG positions of a genomic region is analysed. Ideally, 1, 2, 3, 4, 5, 6, 7, 8, 9, 10 GpG positions of a genomic region are analysed. Therefore, a preferred embodiment of the invention relates to a method, wherein analysing the methylation status of a genomic region means analysing the methylation status of at least one CpG position per genomic region.

In a preferred embodiment the invention relates to a method, wherein the methylation status is analysed by non-methylation-specific PCR based methods followed by sequencing, methylation-based methods such as methylation sensitive PCR, EpiTyper and Methylight assays or enrichment-based methods such as MeDIP-Seq. In an alternative embodiment of the present invention, the DNA methylation is assessed by methylation-specific restriction analysis.

In a preferred embodiment of the invention Epityper® and Methylight® assays may be used for the analysis of the methylation status.

The invention also relates to a preferably synthetic nucleic acid molecule that hybridizes under stringent conditions in the vicinity of one of the genomic region 4, wherein said vicinity relates to a position as defined above. In one embodiment said nucleic acid is 15 to 100 nt in length. In a preferred embodiment said nucleic acid is 15 to 50 nt, in a more preferred embodiment 15 to 40 nt in length.
In another embodiment said nucleic acid is a primer. The inventive primers being specific for a genomic region can be used for the analysis methods of the DNA methylation status. Accordingly, they are used for amplification of a sequence comprising the genomic region or parts thereof in the inventive method for the diagnosis of PC. Within the context of the invention, the primers selectively hybridizes in the vicinity of the genomic region as defined above.
Primers or synthetic nucleic acid molecules may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981). One method for synthesizing oligonucleotides on a modified solid support is described in U.S. Pat. No. 4,458,006. It is also possible to use a primer which has been isolated from a biological source (such as a restriction endonuclease digest).
The methylation status of a genomic region may be detected indirectly (e.g. by bisulphite sequencing) or directly by using a genomic region specific probe, e.g. in a methylight assay. Thus, the present invention also relates to said nucleic acid being a probe. In a preferred embodiment of the present invention the probe is labelled.
Said probes can also be used in techniques such as quantitative real-time PCR (qRT-PCR), using for example SYBR®Green, or using TaqMan® or Molecular Beacon techniques, where the nucleic acids are used in the form of genomic region specific probes, such as a TaqMan labelled probe or a Molecular Beacon labelled probe. Within the context of the invention, the probe selectively hybridizes to the genomic region as defined above. Additionally, in qRT-PCR methods a probe can also hybridize to a position in the vicinity of a genomic region.

Current methods for the analysis of the methylation status require a bisulphite treatment a priori, thereby converting non-methylated cytosines to uracils. To ensure the hybridization of the genomic region specific nucleic acid of the invention to the bisulphite treated DNA, the nucleotide sequence of the nucleic acid may be adapted. For example, if it is desired to design nucleic acids being specific for a sequence, wherein a cytosine is found to be differentially methylated, that genomic region specific nucleic acid may have two sequences: the first bearing an adenine, the second bearing an guanine at that position which is complementary to the cytosine nucleotide in the sequence of the genomic region. The two forms can be used in an assay to analyse the methylation status of a genomic region such that they are capable of discriminating between methylated and non-methylated cytosines. Depending on the analysis method and the sort of nucleic acid (primer/probe), only one form or both forms of the genomic region specific nucleic acid can be used within the assay. Thus, in an alternative embodiment of the present invention the nucleic acid hybridizes under stringent conditions in said vicinity of genomic region 4 after a bisulphite treatment.

The present invention also relates to the use of genomic region specific nucleic acids for the diagnosis of colorectal cancer.

The preferably synthetic nucleic acid or antibody for performing the method according to the invention is advantageously formulated in a stable composition. Accordingly, the present invention relates to a composition for the diagnosis of colorectal cancer comprising said preferably synthetic nucleic acid or antibody.

The composition may also include other substances, such as stabilizers.

### FIGURE CAPTIONS

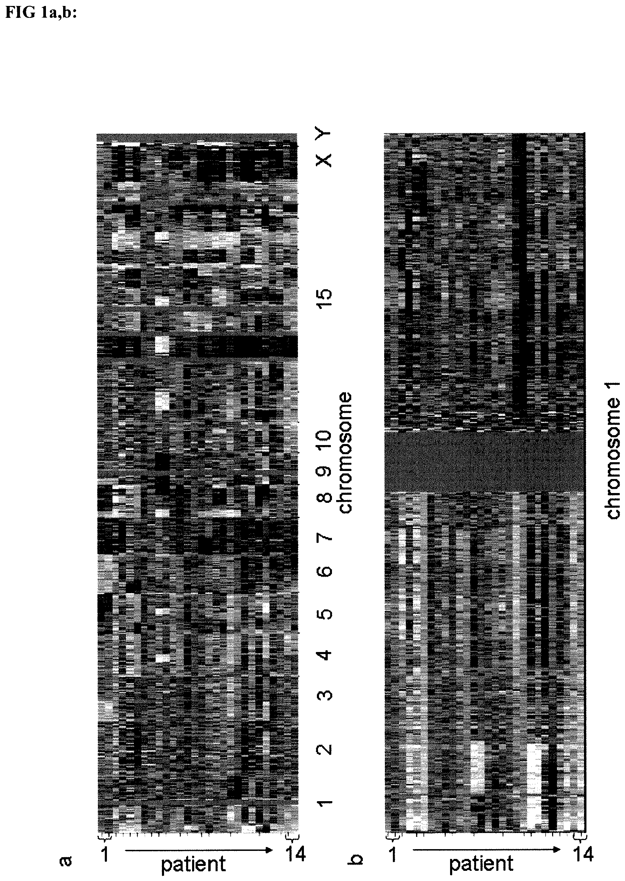

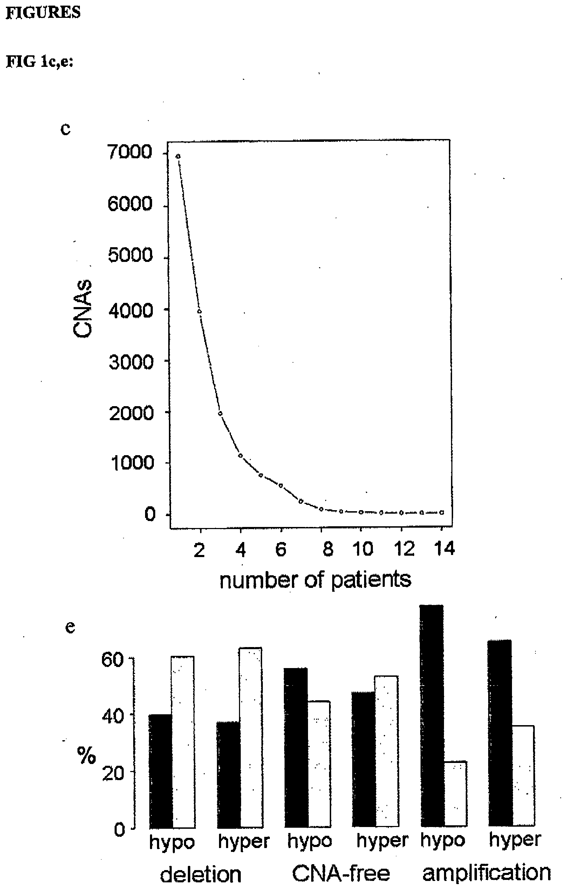

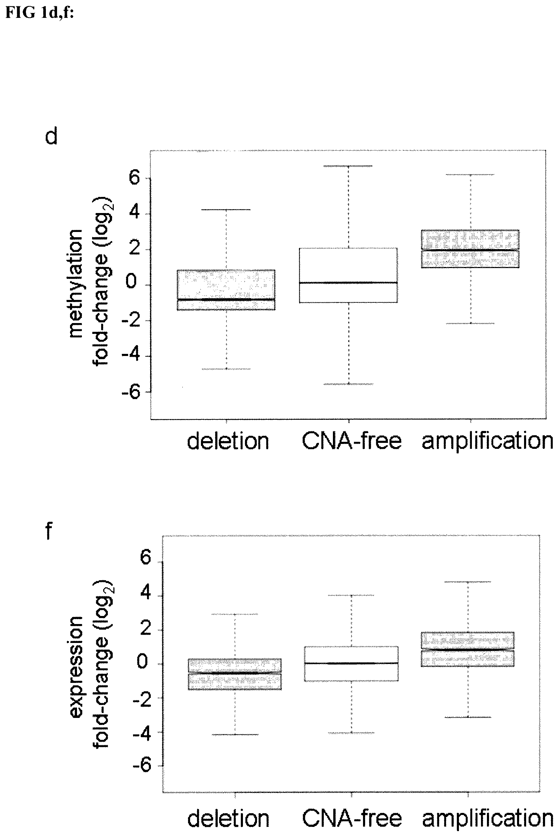

**Figure 1****: Impact of CNA status on methylation and gene expression.** (a) Global patterns of DNA methylation and CNAs. For each patient (PI-PI4) a color-coded representation of methylation (orange labelled rows) and CNA fold-changes (green labelled rows) is shown for 5 million bp adjacent windows across all chromosomes (log2-scale). Yellow colors refer to deletions and hypomethylations and blue colors refer to amplifications and hypermethylations respectively when comparing tumor versus normal tissue. (b) Magnification of chromosome 1 with windows of 0.5 million bp length using the same color-coding. (c) Distribution of somatic CNAs (Y-axis) across all patients (X-axis). (d) Correlation of methylation fold-changes (Y-axis, log2-scale) and CNA status (X-axis). DMRs (tumor versus normal) from all patients were sampled and divided in three groups: DMRs that fall into deletions, amplifications and CNA-free regions. Box plots show the median methylation fold-changes for the three groups and the interquartile range. (e) Correlation of gene expression, DNA methylation and CNAs. Differentially expressed genes were divided into three groups (deletions, CNA-free and amplifications). Bars show the proportion of hyper- and hypomethylated proximal promoter regions (-1 kb to + 0.5kb) within these groups. For each combination of copy number and promoter methylation status the number of up-regulated (dark grey) - and down-regulated (light grey) genes were calculated. For promoters localized in CNA free regions significant correlations between hypermethylation and decreased gene expression as well as between hypomethylation and increased gene expression was observed (Fisher's exact test p-value<0.006). (f) Correlation of expression fold-changes (Y-axis, log2-scale) and CNA status (X-axis). Gene expression values (tumor versus normal) for P12 were divided in three groups: genes that fall into deletions, amplifications and CNA-free regions. Box plots show the median values for the three groups and the interquartile range.

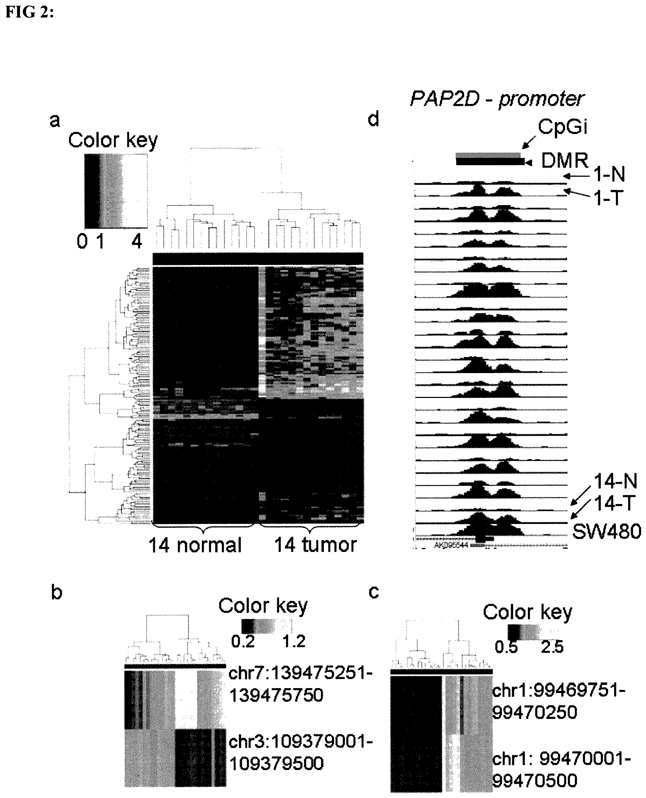

**Figure 2****: Biomarker analysis.** (a) Dendrogram of 158 cDMRs differentially methylated regions comparing tumor (red column labels) and normal tissue (blue column labels). DMRs were selected based on Wilcoxon's test between all samples. Only regions outside of CNAs and with a coefficient of variance below 0.5 were selected. Hierarchical clustering was performed with Canberra distance as pairwise distance measure and complete linkage as update rule using the R software (www.R-project.org). (b) An example of two DMRs sufficient for a correct discrimination of tumor and normal tissues. (c) An example of a single genomic region on chromosome 1 containing two overlapping DMRs that is related to clinical parameters. (d) Visualization of the region on chromosome 1 using the UCSC browser. RPM values are shown in wiggle format and show a consistent hypermethylation in the PAP2D promoter region. The maximal height for visualization was set to rpm=2 for all tracks. Panels show normal and tumor tissue for each patient as well as the SW480 cell line (bottom).

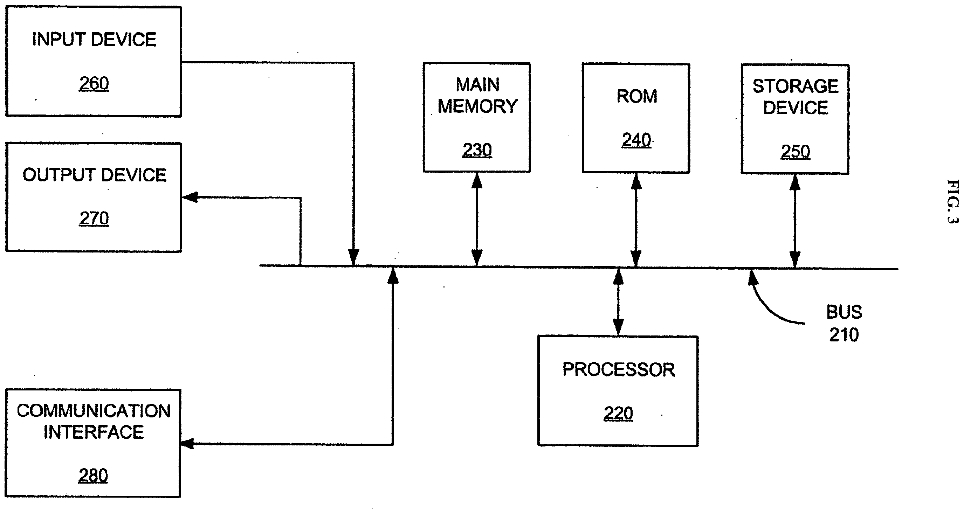

**FIG. 3** is an exemplary diagram of a computing device comprising a client and/or server according to an implementation consistent with the principles of the invention.
**FIG. 4** is a flowchart of exemplary processing of methylation status for biomarker(s) present in biological samples according to an implementation consistent with the principles of the present invention.

### EXAMPLES

### Experimental Procedure

**Tissue samples, DNA and RNA isolation.** The study has been approved by the Ethical Committee of the Medical University of Graz. For recent samples patients have given their written informed consent. For samples older than 15 years no informed consent was available, therefore all samples and medical data used in this study have been irreversibly anonymized.
Human tissue obtained during surgery was snap-frozen in liquid nitrogen. Cryosections (3 µm thick) were prepared and stained with haematoxylin and eosin to evaluate tumor cell content. Dissections were performed under the microscope to achieve a tumor cell content of >80%. DNA isolation was performed using the QIAamp DNA Mini Kit (Qiagen, Hilden, Germany), according to the manufacturer's instructions. DNA from the SW480 cell line was isolated using phenol/chloroform extraction followed by ethanol precipitation. Concentrations were measured on a Nanodrop and quality was assessed on an agarose gel. 10 µg of DNA was treated with 1 µl RNAse A (10µg/µl) for 1h at 37°C prior to fragmentation. Microsatellite stabilities were determined following Promega's MSI Analysis System Protocol.
CpG island methylator phenotype (CIMP) was determined by assessing the MeDIP methylation values of the marker regions described in Issa and Weisenberger et al. (Issa, J.P. CpG island methylator phenotype in cancer. Nat Rev Cancer 4, 988-993 (2004); Weisenberger, D.J. et al. CpG island methylator phenotype underlies sporadic microsatellite instability and is tightly associated with BRAF mutation in colorectal cancer. Nat Genet 38, 787-793 (2006)). A tumor was classified as CIMP positive if at least 3 marker-regions of the classical marker set1 displayed a MeDIP-rpm value >0.26 which corresponds to the 0.99 quantile of the non-enriched input sequence.

**Library preparation and methylated DNA immunoprecipitation (MeDIP).** Genomic DNA of the colon cancer patients was sonicated as described in Parkhomchouk et al. (Parkhomchuk, D. et al. Transcriptome analysis by strand-specific sequencing of complementary DNA. Nucleic Acids Res 37, e123 (2009)) to a size range of 100-400 bp and purified using Qiagen's AllPrep protocol (Qiagen). Then, 5 µg of fragmented DNA was subjected to single end library preparations using the genomic DNA sample prep kit (#FC-102-1002, Illumina, San Diego, USA) according to the manufacturer's instructions with modifications: End repair was performed in 317 µl total volume with 0.25 mM dNTPs Mix, 0.1 U T4 DNA Polymerase, 0.03 u Polymerase I, Klenow DNA Polymerase I (large fragment) and 0.3 U T4 DNA Polynukleotide Kinase. For A-tailing a total volume of 88 µl in the presence of 0.2 mM dATP and 0.5 u Klenow Fragment (3'->5'exo-) was used. Adapters were ligated in a total volume of 98 µl using 29 µl of 'Adapter oligo mix' and two times increased amounts of ligase. Subsequently, the libraries were used for methylated DNA immunoprecipitation (see below). Libraries were amplified after MeDIP and prior to size selection in a total volume of 30 µl using 20 % of the immunoprecipitated DNA or 40 ng of non-immunoprecipitated library (input) for 6 PCR-cycles. Amplified libraries were run on a 2 % agarose gel and fragments of 150-400 bp were excised (corresponding to insert sizes of 80-330 bp) and purified using the Quiaquick Gel Extraktion Kit (Qiagen). Size-selected libraries were quantified using the QuantIt dsHS Assay Kit on a Qubit fluorometer (Invitrogen, Darmstadt, Germany).
MeDIP was adapted from a previously published protocol (Weber et al., 2005). In brief, 10 µl of monoclonal antibody against 5-methylcytidine (#BI-MECY, Eurogentec, Cologne, Germany) were incubated over night with 40 µl Dynabeads M-280 sheep anti-mouse IgG (Invitrogen) in 500 µl 0.5% BSA/PBS, washed two times with 0.5% BSA/PBS and one time with IP-buffer (10 mM sodium phosphate (pH7.0), 140 mM NaCl, 0.25 % Triton X100). Prior to immunoprecipitation, the sequencing libraries were denatured for 1 min at 95°C. Subsequently, 4 µg library was immunoprecipitated for 4 h at 4 °C using a 5-methylcytidine antibody coupled to Dynabeads in a total volume of 230 µl IP-buffer. After immunoprecipitation, the beads were washed three times with 700 µl IP-buffer and then treated with 50 mM Tris-HCl, pH 8.0; 10 mM EDTA, 1% SDS for 15 min at 65 °C. The supernatant containing the methylated DNA (200 µl) was diluted with 200 µl 10 mM Tris pH 8,0, 1 mM EDTA, treated with proteinase K (0.2 µg/µl) for 2 h at 55°C, followed by phenol-chloroform-extraction and ethanol precipitation. The DNA was resuspended in 20 µl 10 mM Tris pH 8.5.

**Validation of the MeDIP-enrichment by quantitative PCR.** The successful enrichment of methylated DNA was controlled by quantitative PCR. The PCR reactions were carried out in 10µl volume in 384 well plates on a 7900 Fast Real-Time PCR system using SYBR Green PCR master mix (Applied Biosystems, Darmstadt, Germany). Relative enrichment was calculated by the ratios of the signals in the immunoprecipitated DNA versus input DNA for a methylated positive and an unmethylated negative control region. Enrichment factors of approximately 50fold were used as parameter for successful enrichment. Primer sequences for methylated and unmethylated control regions were kindly provided by Dr. Vardham Rakyan (Barts and The London School of Medicine and Dentistry) and Prof. Dr. S. Beck, (UCL, Cancer Institute, London) (methylated: #4994; unmethylated: #8804)

**Preparation of RNA-seq libraries.** 2 µg of total RNA were depleted for ribosomal RNA using the RiboMinus Eukaryote Kit for RNA-seq (Invitrogen) following the manufacturer's instructions. The RiboMinus depleted RNA was then used for the generation of RNA-seq libraries using a strand-specific protocol as described previously (Parkhomchouk et al., 2009).

**Next generation sequencing.** After library quantification at a Qubit (Invitrogen) a 10 nM stock solution of the amplified library was created. Then, 12pmol of the stock solution were loaded onto the channels of a 1.4 mm flow cell and cluster amplification was performed. Sequencing-by-synthesis was performed on an Illumina Genome Analyser (GAIIx). All MeDIP and input samples were subjected to 36 nt single read sequencing. The raw data processing was done with the Illumina 1.5 and 1.6 pipeline.
For each of the 29 MeDIP-samples approximately 16 to 32 million uniquely aligned single end reads were generated with a total of over 22 Gb of MeDIP- and 11 Gb of input sequences. On average 69% of the generated reads for the input and 45% of the generated MeDIP-seq reads were uniquely aligned suggesting that approximately 24% of the generated reads (methylated DNA fragments) were located within repetitive sequences.

**Bisulfite treatment and PCR.** Bisulfite treatment was performed using standard protocols. Briefly, 500 ng genomic DNA was treated with 2 M sodium bisulfite and 0.6 M NaOH. Two thermo spikes of 99 °C for 5 min were introduced followed by two incubation steps of 1.5 h at 50 °C. Purification was achieved by loading, desulfonation and washing on a microcon YM-50 column (Millipore, Schwalbach, Germany). Bisulfite DNA was eluted in 50 µl 1xTE. PCRs for validation of MeDIP-seq data were performed in 30 µl reaction volume in presence of 1 x reaction buffer (10 mM Tris-HCL (pH 8.6), 50 mM KCl, 1.5 mM MgCl2), 0.06 mM of each dNTP, 200 nM each, forward and reverse primer, 1.25 U HotStart-IT DNA polymerase (USB, Staufen, Germany) and 2 µl template. Finally, 5 µl of the PCR reaction products were differentiated on a 1.5% agarose gel.

**SIRPH analyses.** The methylation indices at particular CpGs in MeDIP enriched regions were determined using single-nucleotide primer extension (SNuPE) assays in combination with ion pair reverse phase high performance liquid chromatography (IP RP HPLC) separation techniques (SIRPH) (see El-Maarri, O. SIRPH analysis: SNuPE with IP-RP-HPLC for quantitative measurements of DNA methylation at specific CpG sites. Methods Mol Biol 287, 195-205 (2004)). In brief, 5 µl of each PCR product was purified using an ExonucleaseI/SAP mix (1U each, USB, Cleveland, USA) for 30 min at 37° C followed by a 15 min inactivation step at 80° C. Then, 14 µl primer extension mastermix (50 mM Tris-HCL, pH9.5, 2.5 mM MgCl2, 0.05 mM ddCTP, 0.05 mM ddTTP, 3.6 µM of each SNuPE primer) was added and SNuPE reactions were performed. Obtained unpurified products were loaded on a DNASepTM (Transgenomic, Omaha, USA) column and separated in a primer-specific acetonitril gradient on the WAVETM system (Transgenomic). Methylation indices (MI) were obtained by measuring the peak heights (h) and calculating the ratio h(C)/[h(C)+h(T)]. To confirm the methylation assignment across the DMRs the second CpG position in most amplicons was analyzed in addition. For the SIRPH analyses 17 regions were selected and the analyses were performed for three patients and the colon cancer cell line SW480. Median Pearson's correlation values of 0.941 between the rms values (see below) of the MeDIP-seq and the methylation indices of the SIRPH results were achieved.

**Bisulfite pyrosequencing.** 454 GS-FLX: Amplicons were generated using region-specific primers with the recommended adaptors at their 5'-end. PCRs were performed in 30 µl reaction volumes in presence of 10 mM Tris-HCL (pH 8.6), 50 mM KCl, 1.5 mM MgCl2, 0.06 mM of each dNTP, 200 nM each, forward and reverse primer, 1.25 U HotStart-IT DNA polymerase (USB, Staufen, Germany) and 2 µl template. For the amplicons BMP1 and 'T' the usage of 1.5 U HotStarTaq and Q-Solution (Qiagen, Hilden, Germany) was necessary instead of HotStart-IT to obtain specific PCR products. Specific primer sequences and PCR protocols are provided in Supplementary Table 9. Amplicons were purified, measured using the Qubit Fluorometer (Invitrogen) and pooled. After emPCR, DNA containing beads were recovered, enriched and loaded onto a XLR70 Titanium PicoTiterPlate according to the manufacturer's protocols. Methylation level and pattern was assessed using multiple sequence alignment with an extended and improved version of BiQ Analyzer6. For the bisulfite pyrosequencing 25 regions in two patients were investigated and Pearson's correlations for the log2 ratios of tumor vs. normal of 0.842 (0.840) and 0.849 (0.859) for the rpm (rms) and bisulfite values were obtained.

**Alignment and pre-processing of sequencing reads.** Single end sequencing reads (36 bp) generated from MeDIP-seq experiments and input samples were aligned to the human genome (UCSC hgl9) using Bowtie (version 0.12.5 parameter set -q -n 2 -k 5 --best-maxbts 10000 -m 1) allowing up to 2 nucleotide mismatches to the reference genome per seed and returning only uniquely mapped reads. Replicate sequencing reads (i.e. reads with exactly the same starting position) were counted only once.
The analysis of the MeDIP-seq data was performed with the MEDIPS package described in Chavez, L. et al. Computational analysis of genome-wide DNA methylation during the differentiation of human embryonic stem cells along the endodermal lineage. Genome Res 20, 1441-1450 (2010). For each MeDIP-seq and its corresponding input sample, the aligned reads were extended to 300 nt in the sequencing direction. The short read coverage of the extended reads was calculated at genome wide 50 bp bins. Subsequently, the final short read count at each genomic bin is transformed into reads per million format (rpm = number of reads in the bin / number of uniquely aligned reads x 1000000) (see Mortazavi, A., Williams, B.A., McCue, K., Schaeffer, L. & Wold, B. Mapping and quantifying mammalian transcriptomes by RNA-Seq. Nat Methods 5, 621-628 (2008)). Saturation analyses were performed to estimate the required read depth.

**Identification of cancer differentially methylated regions (cDMRs) between tumor and normal samples.** Mean rpm values were calculated for genome-wide 500 bp windows overlapping by 250 bp using MEDIPS. Subsequently, for each 500 bp window, we applied a Wilcoxon's test in order to assess significance of methylation differences between the 14 controls (normal mucosa samples) and the 14 tumor samples. P-values were adjusted using the method of Benjamini and Yekutieli (2001) after exclusion of the mitochondrial and the sex chromosomes. Differentially methylated regions (cDMRs) were identified by filtering for 500 bp windows associated with adjusted p-values < 0.05. Overlapping significant 500 bp windows were merged if their ratios indicated the same hyper- or hypomethylated status. In order to assure that signals within DMRs are above background noise, a ratio of MeDIP versus input rpm-values > 1.5 was required. Here, the MeDIP/input ratio is calculated either for the tumor sample (hypermethylation) or for the normal sample (hypomethylation). In addition, only cDMRs outside of copy number alterations (CNAs) were considered (i.e. none of the patients in our sample set displayed a copy number alteration). Finally, the resulting significant CNA-free DMRs were selected with respect to a minimal p-value and coefficient of variance.
In order to visualize the performance of epigenetic biomarkers for discriminating between tumor and normal samples we performed hierarchical cluster analysis using Canberra distance as pairwise distance measure and complete linkage as update rule using the R software package.
Furthermore, plausible associations between the selected group of 158 cDMRs and clinicopathological characteristics were evaluated using one independent generalized linear model with a quassi-poisson link for each clinical characteristic under consideration (CIMP status, grade, localization, histology, lymphatic node as absent or present, pT, sex, age as younger than or equal to 55 or older or equal than 70). In all the models the response was the rpm values for each tumor. Only conditions with more than one patient were assessed.; p-values below 0.05 were considered as significant and in Table 2 the clinical characteristics significant for more than 5% of the tested cDMRs (>8 single significant cDMRs) were reported.

**Table 2: Most significant cDMRs in CNA-free regions with impact on clinical features (lymph node status, CIMP status and histology).**

| **Chr** | **Start** | **End** | **HUGO gene name** | **Repeat class** | **Ratio T vs N** | **Lymph node pvalue** | **CIMP pvalue** | **Histology pvalue** |
|---|---|---|---|---|---|---|---|---|
| chr1 | 77334501 | 77335000 | ST6GALNAC5 | | 3,8 | 0,041 | 0,094 | 0,109 |
| chr1 | 99469501 | 99470250 | RP11-254O21.1; RP5-896L10.1 | Simple repeat | 3,7 | 0,379 | 0,025 | 0,061 |
| chr1 | 99470501 | 99471000 | RP11-254O21.1; RP5-896L10.1 | Low complexity | 4,8 | 0,193 | 0,047 | 0,123 |
| chr1 | 158151251 | 158151750 | CD1D | | 4,0 | 0,279 | 0,011 | 0,255 |
| chr1 | 170630001 | 170630500 | | | 3,9 | 0,104 | 0,033 | 0,107 |
| chr1 | 177133501 | 177134000 | ASTN1 | Low complexity | 7,6 | 0,139 | 0,043 | 0,086 |
| chr1 | 181452501 | 181453000 | CACNA1 E | Simple repeat | 3,1 | 0,265 | 0,037 | 0,076 |
| chr1 | 181638501 | 181639000 | CACNA1 E | LINE | 0,4 | 0,047 | 0,767 | 0,304 |
| chr1 | 217313001 | 217313750 | | Low complexity | 3,9 | 0,012 | 0,695 | 0,364 |
| chr2 | 7101001 | 7101500 | AC017076.1; AC013460.1; RNF144A | Simple repeat | 3,0 | 0,302 | 0,016 | 0,676 |
| chr2 | 40679501 | 40680000 | SLC8A1 | | 2,7 | 0,721 | 0,042 | 0,588 |
| chr2 | 55062251 | 55062750 | EML6 | LINE | 0,6 | 0,034 | 0,696 | 0,236 |
| chr2 | 66653751 | 66654250 | AC092669.5 | | 3,1 | 0,374 | 0,040 | 0,255 |
| chr2 | 115919751 | 115920750 | DPP10 | Simple repeat | 7,6 | 0,232 | 0,007 | 0,075 |
| chr3 | 149374751 | 149375250 | WWTR1; RP11-255N4.2 | | 3,2 | 0,591 | 0,047 | 0,089 |
| chr3 | 192128001 | 192128500 | FGF12 | Low complexity | 4,6 | 0,033 | 0,411 | 0,768 |
| chr4 | 20254751 | 20255500 | SLIT2 | | 5,7 | 0,032 | 0,362 | 0,361 |
| chr4 | 188666001 | 188666500 | | LINE | 0,4 | 0,009 | 0,418 | 0,821 |
| chr5 | 61041001 | 61041500 | CTD-2170G1.1 | LTR | 0,5 | 0,021 | 0,568 | 0,853 |
| chr5 | 173602501 | 173603000 | | LTR | 0,5 | 0,434 | 0,078 | 0,031 |
| chr6 | 36808251 | 36809000 | | | 3,4 | 0,000 | 0,494 | 0,675 |
| chr6 | 137322751 | 137323250 | IL20RA | | 0,4 | 0,008 | 0,737 | 0,796 |
| chr6 | 151561001 | 151561500 | AKAP12 | | 3,5 | 0,017 | 0,125 | 0,407 |
| chr7 | 79083751 | 79084250 | AC004945.2 | | 3,5 | 0,008 | 0,365 | 0,497 |
| chr7 | 98466751 | 98467500 | TMEM130 | | 7,4 | 0,539 | 0,024 | 0,312 |
| chr10 | 3805001 | 3805500 | RP11-184A2.3 | | 0,5 | 0,046 | 0,537 | 0,557 |
| chr10 | 7454751 | 7455500 | | | 6,0 | 0,369 | 0,029 | 0,059 |
| chr10 | 57389751 | 57390500 | | | 4,8 | 0,008 | 0,189 | 0,047 |
| chr12 | 3602251 | 3603000 | PRMT8 | | 9,2 | 0,476 | 0,014 | 0,006 |
| chr12 | 5019001 | 5019500 | KCNA1 | | 13,5 | 0,043 | 0,248 | 0,184 |
| chr12 | 5019751 | 5020750 | KCNA1 | | 6,9 | 0,044 | 0,014 | 0,012 |
| chr12 | 72667251 | 72667750 | AC087886.1 ; TRHDE | | 6,8 | 0,021 | 0,254 | 0,159 |
| chr12 | 95942751 | 95943250 | USP44 | | 6,1 | 0,361 | 0,002 | 0,016 |
| chr12 | 101916501 | 101917250 | | DNA;SINE | 0,4 | 0,211 | 0,530 | 0,150 |
| chr16 | 55364501 | 55365000 | IRX6 | | 3,7 | 0,003 | 0,241 | 0,258 |
| chr17 | 32908001 | 32908500 | TMEM132E | Low complexity | 7,4 | 0,067 | 0,047 | 0,515 |
| chr19 | 15090751 | 15091250 | | SINE | 3,7 | 0,244 | 0,028 | 0,008 |
| chr19 | 56904751 | 56905250 | ZNF582;AC00 6116.1 | | 7,6 | 0,570 | 0,153 | 0,049 |
| chr19 | 58125751 | 58126250 | | LINE | 3,9 | 0,112 | 0,021 | 0,004 |

**Annotation of the cDMRs.** Each DMR was annotated using ENSEMBL v589. Annotation included gene structures, transcripts, promoter regions (defined as -2 kb downstream and +500 bp upstream of the transcription start site), exons and introns. Furthermore, CpG islands were identified according to the criteria of Takai and Jones (Takai, D. & Jones, P.A. Comprehensive analysis of CpG islands in human chromosomes 21 and 22. Proc Natl Acad Sci U S A 99, 3740-3745 (2002)) and the UCSC annotation. CpG island shores were defined as 1 kb regions upstream or downstream of a CpG island. DMRs were annotated with repetitive regions using the repeat masker table provided by UCSC. CDMRs overlapping conserved elements were identified using the table browser function of the UCSC genome browser (hgl9) and the phastConsElements46wayPrimates track (The Genome Sequencing Consortium, 2001; Fujita, P.A. et al. The UCSC Genome Browser database: update 2011. Nucleic Acids Res 39, D876-882 (2011); Karolchik, D. et al. The UCSC Table Browser data retrieval tool. Nucleic Acids Res 32, D493-496 (2004); Kent, W.J. et al. The human genome browser at UCSC. Genome Res 12, 996-1006 (2002)). For a comparison with colorectal cancer specific cDMRs identified previously by a restriction enzyme based approach and array hybridization, the cDMRs presented by Irizarry et al. (Irizarry, R.A. et al. The human colon cancer methylome shows similar hypo- and hypermethylation at conserved tissue-specific CpG island shores. Nat Genet 41, 178-186 (2009)) were converted from the hgl8 to the hgl9 version using the Batch Coordinate Conversion (liftOver) tool provided by UCSC. The resulting genomic positions were prolonged by 500 bp in each direction and an intersection with the cDMRs identified in this study was determined.

**CNA analysis.** Copy number alterations were detected using CNV-seq by calculating log2-ratios of read counts of the input sequences in tumor and normal tissue per patient in overlapping 25 kb windows along the genome15. The windows overlap by half of their total size (i.e. 12.5 kb). We run CNV-seq with the parameter set: --window-size 25000-log2-threshold 0.6 --p-value 0.005 --minimum-windows-required 1 --genome-size 3095693983 --global-normalization --annotate. P-values were computed based on a Gaussian distribution of the log2-ratios. Subsequently, CNV-seq combined overlapping windows that exceeded both the log2-ratio and p-value thresholds (0.6 and 0.005) and recalculated p-values and log2-ratios for these CNA regions. The detected CNA regions were annotated with exons using BioMart/ ENSEMBL v58.

**RNA-seq analysis.** 36mer RNA-seq reads were aligned to the human genome using Bowtie (version 0.12.5 - parameter set: -n 2 -1 36 -y --chunkmbs 256 --best --strata -k 1 -m 1) against the genomic reference UCSC hgl9. Subsequently, reads that did not map to the genome were aligned to the cDNA reference ENSEMBL v58 in order to map reads spanning exon junctions. Then, uniquely mapped reads aligning to the sense strand of a gene were counted. Differential expression was calculated using the R/BioConductor edgeR package16. Genes were assigned as differentially expressed if the absolute log2 fold-change values were greater than 0.5.

**Correlation of gene expression, copy number and methylation.** A total set of 49,646 genes from ENSEMBL v58 was evaluated in order to determine the interdependence of expression levels, copy number and methylation status.
The methylation status was determined in the promoter region of the genes (defined as 1 kb upstream and 500 bp downstream of the TSS). Here, Wilcoxon's test was performed with the MeDIP-seq data of the individual patient comparing tumor versus normal tissue using 10 adjacent 50bp bins for each 500 bp window in the promoter region. Promoter regions with at least two consistent DMRs with significant corrected p-values <0.1 were considered as hypo- or hypermethylated respectively.
An association analysis was conducted using a qualitative measure for the copy number status (deletion, CNA-free and amplification) and for the methylated status (hypo-, hypermethylated, non-consistent). Expression was considered either quantitatively using the whole set of log2 expression fold-changes (Fig. If), or qualitatively counting only differentially expressed genes (Fig. 1e). For two-sided comparisons (expression versus CNA and CNA versus methylation), quantitative values for the fold-changes were used (Fig. 1d,f). In order to assess associations between copy number or methylation status and gene expression a Kruskal Wallis test was applied to compare the conditions simultaneously and a Wilcoxon test was applied to perform pairwise comparisons. In order to assess associations between methylation status and gene expression given a certain CNA status we evaluated 2x2 contingency tables with an exact Fisher test (Fig. 1e).

### Results

In order to gain a clearer view of the relationships between cytosine methylation, CNAs and the transcriptome we generated genome-wide maps with high-throughput sequencing (HTS) technologies in combination with methylated cytosine specific immunocapturing (MeDIP-seq) for the analyses of 14 heterogeneous colorectal cancers with matched-pair tumor and normal tissues, as well as for the colorectal cancer cell line SW480 as a reference (Table 3). Pairwise Pearson's correlation coefficients indicate on average a greater homogeneity of normal mucosa (0.84 to 0.94), compared to tumor tissue (0.76 to 0.90).

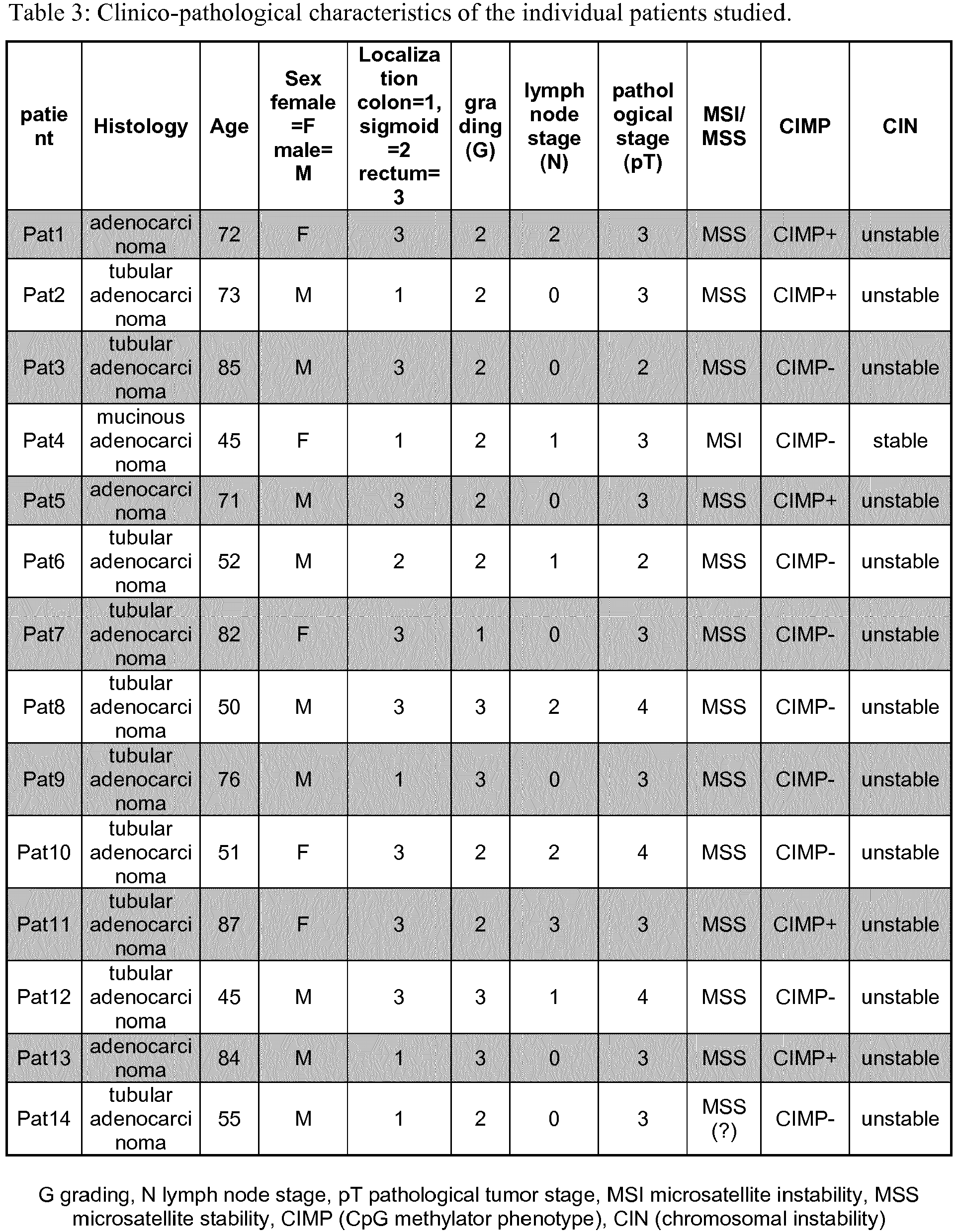

Using a robust non-parametric statistical test in a sliding window approach we identified a total of 7,912 cancer differentially methylated regions (cDMRs), corresponding to 4,381 merged cDMRs (1,673 tumor hyper-, and 2,708 tumor hypo- methylations). The majority (81%) of the tumor hypermethylation marks were located within CpG islands (1,358 cDMRs) and approximately 50% resided in promoters (839 cDMRs). In contrast, most tumor-specific hypomethylations were found in repetitive regions. Within our data set, we observed hypermethylations in low complexity regions and simple repeats, whereas most transposable elements, such as LINE, SINE and LTRs, were demethylated in tumor.

We were able to confirm several cDMRs known to be differentially methylated in cancer and which are described as potential biomarkers like EYA2, UCHL1, LRRC3B, HACE1, BAGE, MLH1, TMEFF2, NGFR, BMP3, ALX4, APC, DAPK, MGMT or SEPT9. However, based on the methylation values a complete discrimination between normal and tumor tissue was not possible or the markers are located within CNA containing regions (UCHL1 and LRRC3B).

To assess the validity of the large number of previously unknown cDMRs found in our study, MeDIP-seq data were validated using two different bisulfite-based validation techniques: methylation-specific single-nucleotide primer extension (SNuPE) followed by HPLC separation (SIRPH), as well as bisulfite pyrosequencing. Both, SIRPH analyses and bisulfite pyrosequencing, strongly correlated with the MeDIP-seq findings (0.94 and 0.85, respectively) indicating a high level of agreement between these techniques.
Our data gives evidence for genome-wide correlations of somatic CNA and methylation patterns (Fig. la,b). Most CNAs were detected in a single, or a low number, of patients (Fig. 1c) and, thus, might bias the discovery of epigenetic biomarkers (Fig. 1d). In addition, CNAs are thought to be partly responsible for transcriptome dosage effects. Therefore, we quantified the expression levels of 49,646 genes with RNA-seq and correlated them with copy number and promoter methylation changes. Indeed, we found a positive correlation between CNA and gene expression (Fig. 1e,f). As cytosine methylation is largely thought to result in transcriptional repression either by interfering with transcription factor binding or by induction of a repressive chromatin structure, we were interested to see whether these effects could be observed on a genomic scale.

Most of the large-scale associations between epigenome and the transcriptome have been studied within normal tissues and the question remains if an aberrant methylation pattern in cancer results in a concomitant misregulation of gene expression. Taking into account promoter methylation and gene expression across the genome, our data gives no evidence per se to support the hypothesis that promoter methylation leads to downregulation of gene expression. However, since we did observe an association between CNAs and gene expression (Fig. If), we correlated methylation and expression in CNA-free and affected regions separately. In contrast to the global promoter methylation analyses here we were able to detect significant correlations between hypermethylation and gene silencing and of hypomethylation with an increase in gene expression. Fig. 1e shows that in CNA free regions there are 12% more up-regulated compared to down-regulated genes, associated with hypomethylated promoters, whereas this trend is reversed for genes with hypermethylated promoters, where we observed 6% more down-regulated genes compared to up-regulated genes. This significantly connects promoter hypermethylation with down-and promoter hypomethylation with up-regulation of gene expression (Fisher test P=0.006); an effect that cannot be observed without corrections for CNAs. It is not clear from these data if the alteration in the methylation pattern within CNA regions observed is due to differing immunoprecipitation yields arising from variation in DNA levels, or if it is a physiological response to compensate differential gene expression arising from copy number alterations. This mechanism might not occur in a linear manner and simple proportional normalizations might be problematic. Taken together, we conclude that copy number aberrations impair the correlation between transcript and DNA methylation levels in the respective regions.

In particular for the identification of biomarkers this conclusion plays an important role: Within our patient's cohort we find CNA-free regions to be consistently represented across many patients (Fig. 1c). Here we detected 1,483 cDMRs (out of the 7,912 significant cDMRs described earlier) free of CNAs for all of the patients including 158 highly statistically robust regions, highlighting them as extremely attractive options for biomarker development (significant p-value <0.00684 after correction for multiple testing and lowest coefficients of variance <0.5) (Fig. 2a). Of these regions, already two were able to accurately classify the patients' tissues (Fig. 2b). Finally, we correlated these DMRs with the clinical parameters of the patients and derived a potential biomarker subset associated with CIMP status, histological observation and lymph node status (Table 2). Strikingly, we find among this subset that even one single region on chromosome 1 (composed of two overlapping significant cDMRs), can successfully separate tumor from normal tissue (Fig 2c, d). This means, for classification two regions are required, while for diagnosis a single genomic region that is selected from the group of Table 1 is sufficient.

The performance of this biomarker, and others found in CNA-free regions of the tumor genome, outperforms that of recently suggested biomarkers, SEPT9 or ALX425. The variable performance of these biomarkers may be linked to their location within CNAs in two (four for ALX4) patients studied here. For other regions described in the literature such as BRAF, MLH1 or APC we do not find significant differential methylation over the patients (see above). Our findings challenge the efficacy of using these biomarkers as general diagnostics.

Taken together, our results of the genome-wide interplay between CNAs, methylome and transcriptome, have important implications on the use of cancer diagnostic assays. We propose here that clinical analysis of cDMRs in regions devoid of CNAs could eliminate variation, decrease failure rate, and thus improve the predictive power of such assays. These quality control steps will make it possible in the future to identify methylation marks as robust biomarkers for the diagnosis and the prediction of tumor progression and response.

## Claims

1. A method for diagnosis of colorectal cancer, comprising the steps of
a. analysing in a sample of a subject the DNA methylation status of at least genomic region 4 (SEQ ID NO:4),
b. wherein, if the at least one genomic region is hypermethylated, the sample is designated as colorectal cancer positive.

2. The method according to any of the preceding claims, wherein the genomic region is located in a region that is free of copy number alterations (CNAs).

3. The method according to any of the preceding claims, wherein the methylation status of a further genomic region and/or a further biomarker is analysed.

4. The method according to any of the preceding claims, wherein analysing the methylation status of a genomic region means analysing the methylation status of at least one CpG position per genomic region.

5. The method according to any of the preceding claims, wherein the methylation status is analysed by non-methylation-specific PCR based methods, methylation-based methods or microarray-based methods.

6. The method according to claim 5, wherein the methylation status is analysed by Epityper and Methylight (qPCR) assays.

7. The method according to any of the preceding claims, wherein the methylation status is calculated as a ratio of the percentage of methylated DNA of the biomarker in the sample to the percentage of non-methylated DNA of the biomarker in the sample.

8. The method according to any of the preceding claims, wherein the measuring step is conducted by a computing device.

9. The method according to any of the preceding claims, wherein the correlating step is conducted by a computing device.

10. The method according to any of the preceding claims, further comprising outputting for presentation on a display associated with the computing device.

11. Use of a chemically synthesized nucleic acid molecule that hybridizes under stringent conditions in the vicinity of genomic region 4 (SEQ ID NO:4), wherein said vicinity is any position having a distance of up to 500 nt from the 3' or 5' end of said genomic region, wherein said vicinity includes the genomic region itself, for the diagnosis of colorectal cancer.

12. Use according to claim 11, wherein the nucleic acid is 15 to 100 nt in length.

## Patentansprüche

1. Eine Methode zur Diagnose von Darmkrebs, umfassend der Schritte
a. analysieren des DNA Methylierungs-Zustands von mindestens einer genomischen Region 4 (SEQ ID NO: 4),
b. wobei, wenn die mindestens eine genomische Region hypermethyliert ist, die Probe als Darmkrebs positiv ausgewiesen wird.

2. Die Methode nach einem der vorhergehenden Ansprüche, wobei die genomische Region in einer Region gelegen ist, die frei von Änderungen der Anzahl an Kopien ist (CNAs).

3. Die Methode nach einem der vorhergehenden Ansprüche, wobei der Methylierungs-Zustand einer weiteren genomischen Region und/oder eines weiteren Biomarkers analysiert wird.

4. Die Methode nach einem der vorhergehenden Ansprüche, wobei analysieren des Methylierungs-Zustands einer genomischen Region, analysieren des Methylierungs-Zustands von mindestens einer CpG Position pro genomischer Region bedeutet.

5. Die Methode nach einem der vorhergehenden Ansprüche, wobei der Methylierungs-Zustand durch nicht-Methylierungs-spezifische PCR basierte Methoden, Methylierungsbasierte Methoden oder Mikroarray-basierte Methoden analysiert wird.

6. Die Methode nach Anspruch 5, wobei der Methylierungs-Zustand durch Epityper und Methylight (qPCR) Assays analysiert wird.

7. Die Methode nach einem der vorhergehenden Ansprüche, wobei der Methylierungs-Zustand als Verhältnis des Anteils der methylierten DNA des Biomarkers in der Probe, zum Anteil an nicht-Methylierter DNA des Biomarkers in der Probe berechnet wird.

8. Die Methode nach einem der vorhergehenden Ansprüche, wobei der Messungsschritt mit einem Computergerät durchgeführt wird.

9. Die Methode nach einem der vorhergehenden Ansprüche, wobei der Korrelationsschritt mit einem Computergerät durchgeführt wird.

10. Die Methode nach einem der vorhergehenden Ansprüche, des Weiteren umfassend ausgeben zur Präsentation auf einem Display, der mit dem Computergerät assoziiert ist.

11. Verwendung eines chemisch synthetisierten Nukleinsäure-Moleküls zur Diagnose von Darmkrebs, welches unter strengen Bedingungen in der Nähe der genomischen Region 4 (SEQ ID NO:4) hybridisiert, wobei besagte Nähe jede Position, die einen Abstand von bis zu 500nt von dem 3' oder 5' Ende von besagter genomischer Region hat, wobei besagte Nähe die genomische Region selber beinhaltet.

12. Verwendung nach Anspruch 11, wobei die Nukleinsäure 15 bis 100 nt lang ist.

## Revendications

1. Un procédé pour le diagnostic du cancer colorectal, comprenant les étapes de
a. analyser dans un échantillon d'un sujet l'état de méthylation de l'ADN au moins de la région génomique 4 (SEQ ID NO :4),
b. où, si la au moins une région génomique est hyperméthylée, l'échantillon est désigné comme positif pour le cancer colorectal.

2. Le procédé selon l'une quelconque des revendications précédentes, dans lequel la région génomique est située dans une région qui est libre de modifications de nombre de copies (CNAs).

3. Le procédé selon l'une quelconque des revendications précédentes, dans lequel l'état de méthylation d'une région génomique supplémentaire et/ou d'un biomarqueur supplémentaire est analysé.

4. Le procédé selon l'une quelconque des revendications précédentes, dans lequel analyser l'état de méthylation d'une région génomique signifie analyser l'état de méthylation d'au moins une position CpG par région génomique.

5. Le procédé selon l'une quelconque des revendications précédentes, dans lequel l'état de méthylation est analysé par un procédé basé sur la PCR spécifique à la non-méthylation, des procédés basés sur la méthylation ou des procédés basés sur le microarray.

6. Le procédé selon la revendication 5, dans lequel l'état de méthylation est analysé par des essais Epityper et Methylight (qPCR).

7. Le procédé selon l'une quelconque des revendications précédentes, dans lequel l'état de méthylation est calculé en tant que rapport entre le pourcentage d'ADN méthylé du biomarqueur dans l'échantillon et le pourcentage d'ADN non-méthylé du biomarqueur dans l'échantillon.

8. Le procédé selon l'une quelconque des revendications précédentes, dans lequel l'étape de mesure est effectuée par un dispositif informatique.

9. Le procédé selon l'une quelconque des revendications précédentes, dans lequel l'étape de corrélation est effectuée par un dispositif informatique.

10. Le procédé selon l'une quelconque des revendications précédentes, comprenant en plus la sortir les données pour la présentation sur un écran associé avec le dispositif informatique.

11. Utilisation d'une molécule d'acide nucléique synthétisée chimiquement qui s'hybride sous des conditions strictes au voisinage de la région génomique 4 (SEQ ID NO : 4), où ledit voisinage est n'importe quelle position qui est à une distance de jusqu'à 500 nt de la fin 3' ou 5' de ladite région génomique, où ledit voisinage inclut la région génomique elle-même, pour le diagnostic du cancer colorectal.

12. Utilisation selon la revendication 11, dans laquelle l'acide nucléique a une longueur de 15 à 100 nt.
